# EUROPÄISCHE PATENTSCHRIFT

(11) **EP 2 089 380 B1**
(45) Veröffentlichungstag und Bekanntmachung des Hinweises auf die Patenterteilung: **29.06.2016**
(21) Anmeldenummer: 07819045.1
(22) Anmeldetag: 17.10.2007
(51) Int. Cl.: C07D 401/14, C07D 417/14, A61K 31/4155

(54) **SUBSTITUIERTE DIHYDROPYRAZOLONE UND IHRE VERWENDUNG ALS HIF-PROLYL-4-HYDROXYLASE INHIBITOREN**
SUBSTITUTED DIHYDROPYRAZOLONES AND USE THEREOF AS HIF-PROLYL-4 -HYDROXYLASE INHIBITORS
DIHYDROPYRAZOLONES SUBSTITUÉES ET LEUR UTILISATION COMME INHIBITEURS DE L'HIF-PROLYL-4-HYDROXYLASE

(30) Priorität: 26.10.2006 DE 102006050513
(43) Veröffentlichungstag der Anmeldung: 19.08.2009
(73) Patentinhaber: Bayer Intellectual Property GmbH, 40789 Monheim (DE)
(72) Erfinder: THEDE, Kai, 10405 Berlin (DE); FLAMME, Ingo, 51580 Reichshof (DE); OEHME, Felix, 42113 Wuppertal (DE); ERGÜDEN, Jens-Kerim, 42489 Wülfrath (DE); STOLL, Friederike, 40215 Düsseldorf (DE); SCHUHMACHER, Joachim, 42113 Wuppertal (DE); WILD, Hanno, 42113 Wuppertal (DE); KOLKHOF, Peter, 42113 Wuppertal (DE); BECK, Hartmut, 50733 Köln (DE); AKBABA, Metin, 40880 Ratingen (DE); JESKE, Mario, 42699 Solingen (DE)
(74) Vertreter: BIP Patents
(86) Internationale Anmeldenummer: PCT/EP2007/008977
(87) Internationale Veröffentlichungsnummer: WO 2008/049538

(56) Entgegenhaltungen:
- EP-A- 0 183 159
- WO-A-2006/114213
- DOWELL R I ET AL: "Novel inhibitors of prolyl 4-hydroxylase. Part 4. Pyridine-2-carboxylic acid analogues with alternative 2-substituents" EUROPEAN JOURNAL OF MEDICINAL CHEMISTRY, EDITIONS SCIENTIFIQUE ELSEVIER, PARIS, FR, Bd. 28, 1993, Seiten 513-516, XP002087215 ISSN: 0223-5234

## Beschreibung

Die vorliegende Anmeldung betrifft neue, substituierte Dihydropyrazolon-Derivate, Verfahren zu ihrer Herstellung, sowie ihre Verwendung zur Herstellung von Arzneimitteln zur Behandlung und/oder Prophylaxe von Krankheiten, insbesondere von kardiovaskulären und hämatologischen Erkrankungen, von Nierenerkrankungen sowie zur Förderung der Wundheilung.

Eine mangelhafte Versorgung des menschlichen Organismus oder seiner Teile mit Sauerstoff, die entweder durch ihre Dauer und/oder ihr Ausmaß eine regelrechte Funktion des Organismus oder seiner Teile beeinträchtigt oder ihre Funktion völlig zum Erliegen bringt, wird als Hypoxie bezeichnet. Eine Hypoxie kann verursacht werden durch eine Verminderung des verfügbaren Sauerstoffs in der Atemluft (z.B. bei Aufenthalten in großer Höhe), durch Störungen der äußeren Atmung (z.B. infolge gestörter Funktion der Lungen oder Verlegung der Atemwege), durch eine Verminderung des Herzzeitvolumens (z.B. bei einer Herzinsuffizienz, einer akuten Rechtsherz-überlastung bei Lungenembolie), durch eine zu geringe Sauerstoff-Transportkapazität des Blutes (z.B. infolge einer Blutarmut (Anämie) oder Intoxikation z.B. mit Kohlenmonoxid), örtlich begrenzt durch eine Minderdurchblutung infolge von Gefäßverschlüssen (Ischämie-Zustände typischerweise z.B. des Herzens, der unteren Extremitäten oder des Gehirns, diabetische Makro- und Mikroangiopathie) oder auch durch erhöhten Sauerstoffbedarf des Gewebes (z.B. infolge erhöhter Muskelarbeit oder lokaler Entzündungen) [Eder, Gedigk (Hrsg.), Allgemeine Pathologie und pathologische Anatomie, 33. Aufl., Springer Verlag, Berlin, 1990].

Der menschliche Organismus ist bedingt fähig, sich an Situationen verminderter Sauerstoffversorgung akut und chronisch anzupassen. Neben einer Sofortantwort, welche u.a. durch vegetativnervöse Kontrollmechanismen eine Erhöhung des Herzzeitvolumens und des Atemzeitvolumens sowie eine lokale Erweiterung von Blutgefäßen einschließt, zieht die Hypoxie eine Veränderung der Transkription zahlreicher Gene nach sich. Die Funktion der Genprodukte dient dabei der Kompensation des Sauerstoffmangels. So werden mehrere Enzyme der Glykolyse und der Glukosetransporter 1 verstärkt exprimiert, wodurch die anaerobe ATP-Gewinnung gesteigert und ein Überleben des Sauerstoffmangels ermöglicht wird [Schmidt, Thews (Hrsg.), Physiologie des Menschen, 27. Aufl., Springer Verlag, Berlin, 1997; Löffler, Petrides (Hrsg.), Biochemie und Pathobiochemie, 7. Aufl., Springer Verlag, Berlin, 2003].

Ferner führt Hypoxie zur verstärkten Expression des vaskulären Endothelzellwachstumsfaktors, VEGF, wodurch in hypoxischen Geweben die Neubildung von Blutgefäßen (Angiogenese) angeregt wird. Hierdurch wird langfristig die Durchblutung ischämischer Gewebe verbessert. Bei verschiedenen Herzkreislauferkrankungen und Gefäßverschluß-Erkrankungen erfolgt diese Gegenregulation offenbar nur sehr unzureichend [Übersicht bei: Simons und Ware, Therapeutic angio-genesis in cardiovascular disease, Nat. Rev. Drug. Discov. 2 (11), 863-71 (2003)].

Ferner wird bei systemischer Hypoxie das vorwiegend in den interstitiellen Fibroblasten der Nieren gebildete Peptidhormon Erythropoietin verstärkt exprimiert. Hierdurch wird die Bildung roter Blutzellen im Knochenmark angeregt und damit die Sauerstoff-Transportkapazität des Blutes gesteigert. Dieser Effekt wurde und wird von Leistungssportlern beim sogenannten Höhentraining genutzt. Eine Abnahme der Sauerstoff-Transportkapazität des Blutes z.B. infolge einer Blutungsanämie verursacht normalerweise eine Zunahme der Erythropoietin-Produktion in der Niere. Bei bestimmten Formen der Anämie kann dieser Regelmechanismus gestört oder sein Sollwert niedriger eingestellt sein. So wird z.B. bei Patienten, die unter einer Niereninsuffizienz leiden, Erythropoietin im Nierenparenchym zwar produziert, jedoch bezogen auf die Sauerstoff-Transportkapazität des Blutes in deutlich verminderten Mengen, was eine sogenannte renale Anämie zur Folge hat. Insbesondere die renale Anämie, aber auch Tumor- und HIV-Infektion-bedingte Anämien werden üblicherweise durch parenterale Verabfolgung von rekombinantem humanen Erythropoietin (rhEPO) behandelt. Derzeit existiert zu dieser kostspieligen Therapie keine alternative Therapie mit einem oral verfügbaren Arzneistoff [Übersichten bei: Eckardt, The potential of erythropoietin and related strategies to stimulate erythropoiesis, Curr. Opin. Investig. Drugs 2(8), 1081-5 (2001); Berns, Should the target hemoglobin for patients with chronic kidney disease treated with erythropoietic replacement therapy be changed?, Semin. Dial. 18 (1), 22-9 (2005)]. Jüngere Untersuchungen belegen, dass Erythropoetin neben seiner die Erythropoese steigernden Wirkung auch eine hiervon unabhängige, schützende (anti-apoptotische) Wirkung auf hypoxische Gewebe, insbesondere das Herz und das Gehirn ausübt. Ferner mindert eine Therapie mit Erythropoetin neueren Studien zufolge an herzinsuffizienten Patienten den durchschnittlichen Morbiditäts-Schweregrad [Übersichten bei: Caiola und Cheng, Use of erythropoietin in heart failure management, Ann. Pharmacother. 38 (12), 2145-9 (2004); Katz, Mechanisms and treatment of anemia in chronic heart failure, Congest. Heart. Fail. 10 (5), 243-7 (2004)].

Den oben beschriebenen durch Hypoxie induzierten Genen ist gemeinsam, dass die Steigerung ihrer Expression unter Hypoxie durch den sogenannten Hypoxie-induzierbaren Transkriptionsfaktor (HIF) hervorgerufen wird. Bei HIF handelt es sich um einen heterodimeren Transkriptionsfaktor, der aus einer alpha- und einer beta-Untereinheit besteht. Es wurden drei HIF-alpha-Isoformen beschrieben, von denen HIF-1 alpha und HIF-2 alpha hoch homolog und von Bedeutung für die Hypoxie-induzierte Genexpression sind. Während die auch als ARNT (aryl hydrocarbon receptor nuclear translocator) bezeichnete beta-Untereinheit (von der 2 Isoformen beschrieben wurden) konstitutiv exprimiert ist, hängt die Expression der alpha-Untereinheit vom Sauerstoffgehalt in der Zelle ab. Unter Normoxie wird das HIF-alpha-Protein poly-ubiquitiniert und anschließend proteasomal degradiert. Unter Hypoxie ist diese Degradierung gehemmt, so dass HIF-alpha mit ARNT dimerisieren und seine Zielgene aktivieren kann. Das HIF-Dimer bindet hierbei an sogenannte Hypoxie-responsible Elemente (HRE) in den regulatorischen Sequenzen seiner Zielgene. Die HRE sind durch eine Konsensus-Sequenz definiert. Funktionelle HRE wurden in den regulatorischen Elementen zahlreicher Hypoxie-induzierter Gene nachgewiesen [Übersichten bei: Semenza, Hypoxia-inducible factor 1: oxygen homeostasis and disease pathophysiology, Trends Mol. Med. 7 (8), 345-50 (2001); Wenger und Gassmann, Oxygen(es) and the hypoxia-inducible factor-1, Biol. Chem. 378 (7), 609-16 (1997)].

Der molekulare Mechanismus, der dieser Regulation von HIF-alpha zugrunde liegt, wurde durch die Arbeiten mehrerer voneinander unabhängiger Forschergruppen aufgeklärt. Der Mechanismus ist Spezies-übergreifend konserviert: HIF-alpha wird durch eine als PHD oder EGLN bezeichnete Subklasse von Sauerstoff-abhängigen Prolyl-4-Hydroxylasen an zwei spezifischen Prolyl-Resten hydroxyliert (P402 und P564 der humanen HIF-1-alpha-Untereinheit). Bei den HIF-Prolyl-4-Hydroxylasen handelt es sich um Eisen-abhängige, 2-Oxoglutarat-umsetzende Dioxygenasen [Epstein et al., C. elegans EGL-9 and mammalian homologs define a family of dioxygenases that regulate HIF by prolyl hydroxylation, Cell 107 (1), 43-54 (2001); Bruick und McKnight, A con-served family of prolyl-4-hydroxylases that modify HIF, Science 294 (5545), 1337-40 (2001); Ivan et al., Biochemical purification and pharmacological inhibition of a mammalian prolyl hydroxy-lase acting on hypoxia-inducible factor, Proc. Natl. Acad. Sci. U.S.A. 99 (21), 13459-64 (2002)]. Die Enzyme wurden 2001 erstmals als Prolyl-Hydroxylasen annotiert [Aravind und Koonin, The DNA-repair protein AlkB, EGL-9, and leprecan define new families of 2-oxoglutarate- and irondependent dioxygenases, Genome Biol. 2 (3), research0007.1-0007.8, Epub 2001 Feb 19].

An die prolylhydroxylierte HIF-alpha-Untereinheit bindet das pVHL Tumor Suppressor Protein, welches zusammen mit Elongin B und C den sogenannten VBC-Komplex bildet, der die HIF-alpha-Untereinheit an eine E3 Ubiquitin-Ligase adaptiert. Da die Prolyl-4-Hydroxylierung der HIF-alpha-Untereinheit und ihre nachfolgende Degradierung in Abhängigkeit von der intrazellulären Konzentration des Sauerstoffs erfolgt, wurden die HIF-Prolyl-4-Hydroxylasen auch als zellulärer Sauerstoffsensor bezeichnet. Es wurden drei Isoformen dieser Enzyme identifiziert: EGLN1/PHD2, EGLN2/PHD1 und EGLN3/PHD3. Zwei dieser Enzyme (EGLN2/PHD1 und EGLN3/PHD3) werden selbst unter Hypoxie transkriptionell induziert und sind möglicherweise für die unter chronischer Hypoxie zu beobachtende Absenkung der HIF-alpha-Spiegel verantwortlich [Übersicht bei: Schofield und Ratcliffe, Oxygen sensing by HIF hydroxylases, Nat. Rev. Mol. Cell. Biol. 5 (5), 343-54 (2004)].

Eine selektive pharmakologische Hemmung der HIF-Prolyl-4-Hydroxylasen zieht die Steigerung der Genexpression HIF-abhängiger Zielgene nach sich und ist damit für die Therapie zahlreicher Krankheitsbilder von Nutzen. Insbesondere bei Erkrankungen des Herz-Kreislaufsystems ist von der Induktion neuer Blutgefäße sowie der Änderung der Stoffwechselsituation ischämischer Organe von aerober hin zu anaerober ATP-Gewinnung eine Besserung des Krankheitsverlaufs zu erwarten. Eine Verbesserung der Vaskularisierung chronischer Wunden fördert den Heilungsprozess, insbesondere bei schwer heilenden Ulcera cruris und anderen chronischen Hautwunden. Die Induktion von körpereigenem Erythropoietin bei bestimmten Krankheitsformen, insbesondere bei Patienten mit renaler Anämie, ist ebenfalls ein anzustrebendes therapeutisches Ziel.

Die bislang in der wissenschaftlichen Literatur beschriebenen HIF-Prolyl-4-Hydroxylase-Inhibitoren erfüllen nicht die an ein Arzneimittel zu stellenden Anforderungen. Es handelt sich hierbei entweder um kompetitive Oxoglutarat-Analoga (wie z.B. N-Oxalylglycin), die durch ihre sehr geringe Wirkstärke charakterisiert sind und daher in *in* vivo-Modellen bislang keine Wirkung im Sinne einer Induktion von HIF-Zielgenen gezeigt haben. Oder es handelt sich um Eisen-Komplex-bildner (Chelatoren) wie Desferroxamin, die als unspezifische Hemmstoffe Eisen-haltiger Dioxygenasen wirken und, obwohl sie eine Induktion der Zielgene wie z.B. Erythropoetin *in vivo* herbeiführen, durch Komplexierung des verfügbaren Eisens offenbar der Erythropoese entgegenwirken.

Aufgabe der vorliegenden Erfindung ist die Bereitstellung neuer Verbindungen, die für die Behandlung von Erkrankungen, insbesondere kardiovaskulärer und hämatologischer Erkrankungen, eingesetzt werden können.

Im Rahmen der vorliegenden Erfindung werden nunmehr Verbindungen beschrieben, die als spezifische Hemmstoffe der HIF-Prolyl-4-Hydroxylasen wirken und aufgrund dieses spezifischen Wirk-mechanismus *in vivo* nach parenteraler oder oraler Verabreichung die Induktion von HIF-Ziel-genen, wie z.B. Erythropoetin, und der hierdurch verursachten biologischen Prozesse, wie z.B. Erythropoese, herbeiführen.

2-Heteroaryl-4-aryl-1,2-dihydropyrazolone mit bakterizider und/oder fungizider Wirkung werden in EP 165 448 und EP 212 281 offenbart. Die Verwendung von 2-Heteroaryl-4-aryl-1,2-dihydro-pyrazolonen als Lipoxygenase-Inhibitoren zur Behandlung von Atemwegs-, Herz-Kreislauf- und Entzündungserkrankungen wird in EP 183 159 beansprucht. 2,4-Diphenyl-1,2-dihydropyrazolone mit herbizider Aktivität werden in DE 2 651 008 beschrieben. Über die Herstellung und pharmakologischen Eigenschaften bestimmter 2-Pyridyl-1,2-dihydropyrazolone wird in Helv. Chim. Acta 49 (1), 272-280 (1966) berichtet. In WO 96/12706, WO 00/51989 und WO 03/074550 werden Verbindungen mit Dihydropyrazolon-Partialstruktur zur Behandlung unterschiedlicher Erkrankungen beansprucht, und in WO 2006/101903 werden Hydroxy- oder Alkoxy-substituierte Bipyrazole zur Behandlung neuropsychiatrischer Erkrankungen offenbart. Weiterhin werden in WO 03/051833 und WO 2004/089303 Heteroaryl-substituierte Pyrazol-Derivate zur Behandlung von Schmerz und verschiedenen ZNS-Erkrankungen beschrieben. Zwischenzeitlich sind in WO 2006/114213 2,4-Dipyridyl-1,2-dihydropyrazolone als Inhibitoren von HIF-Prolyl-4-Hydroxylasen offenbart worden. In R.I. Dowell et al, European Journal of Medicinal Chemistry 28, 513-516 (1993) wird 5-Methyl-2-(2-pyridinyl)-2,4-dihydro-3H-pyrazol-3-on als Prolyl-4-hydroxylase Inhibitor beschrieben.

Gegenstand der vorliegenden Erfindung sind Verbindungen der allgemeinen Formel (I) 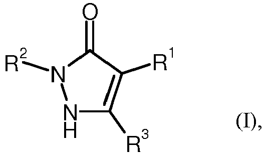 in welcher
- R¹: für eine Heteroaryl-Gruppe der Formel 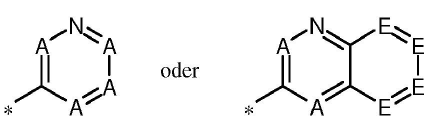 steht, worin
* die Verknüpfungsstelle mit dem Dihydropyrazolon-Ring bedeutet,
A bei jedem einzelnen Auftreten C-R⁴ oder N bedeutet, wobei maximal zwei Ringglieder A zugleich für N stehen, und
E bei jedem einzelnen Auftreten C-R⁵ oder N bedeutet, wobei maximal zwei Ringglieder E zugleich für N stehen,
- R²: für eine Heteroaryl-Gruppe der Formel 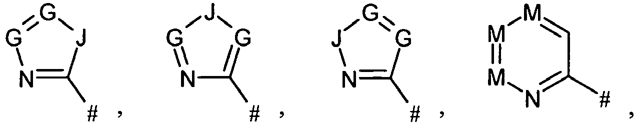 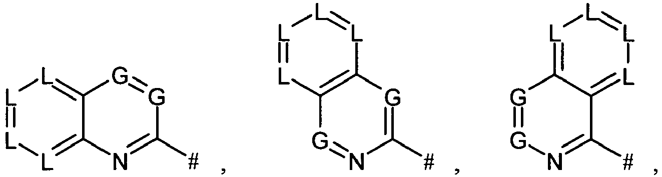 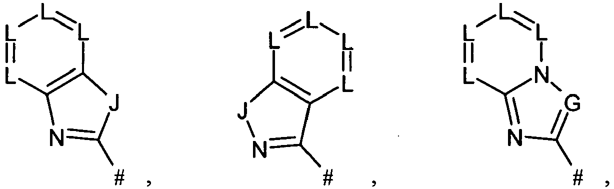 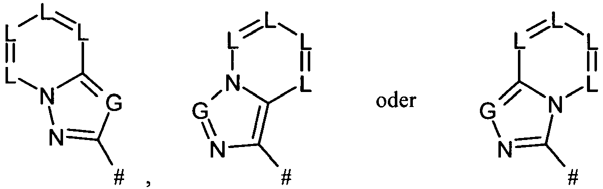 steht, worin
# die Verknüpfungsstelle mit dem Dihydropyrazolon-Ring bedeutet,
G bei jedem einzelnen Auftreten C-R⁶ oder N bedeutet,
J O, S oder N-R⁷ bedeutet,
L bei jedem einzelnen Auftreten C-R⁸ oder N bedeutet, wobei maximal zwei Ringglieder L zugleich für N stehen, und
M bei jedem einzelnen Auftreten C-R⁹ oder N bedeutet, wobei insgesamt ein oder zwei Ringglieder M für N stehen,
worin
R⁴, R⁶, R⁸ und R⁹ gleich oder verschieden sind und in jedem einzelnen Fall, unabhängig voneinander, für Wasserstoff oder einen Substituenten ausgewählt aus der Reihe Halogen, Cyano, Nitro, (C₁-C₆)-Alkyl, (C₃-C₇)-Cycloalkyl, 4- bis 10-gliedriges Heterocycloalkyl, Phenyl, 5- oder 6-gliedriges Heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² stehen, worin
(i) (C₁-C₆)-Alkyl seinerseits ein- bis dreifach, gleich oder verschieden, mit Resten ausgewählt aus der Reihe Halogen, Cyano, Oxo, (C₃-C₇)-Cyclöalkyl, 4- bis 10-gliedriges Heterocycloalkyl, Phenyl, 5- oder 6-gliedriges Heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² substituiert sein kann,
   wobei die letztgenannten Cycloalkyl-, Heterocycloalkyl-, Phenyl- und Heteroaryl-Reste ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*ii*) (C₃-C₇)-Cycloalkyl, 4- bis 10-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, (C₃-C₇)-Cycloalkyl, Halogen, Cyano, Oxo, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² substituiert sein können,
   wobei der letztgenannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cycloalkyl, 4- bis 7-gliedrigem Heterocycloalkyl, Phenyl und/oder 5- oder 6-gliedrigem Heteroaryl substituiert sein kann,
(*iii*) R¹⁰, R¹¹, R¹², R¹⁴, R¹⁵, R¹⁸, R²⁰, R²², R²⁵, R²⁶, R²⁷, R²⁹, R³⁰ und R³¹ unabhängig voneinander bei jedem einzelnen Auftreten für einen Rest ausgewählt aus der Reihe Wasserstoff, (C₁-C₆)-Alkyl, (C₃-C₇)-Cycloalkyl, 4- bis 7-gliedriges Heterocycloalkyl, Phenyl und 5- oder 6-gliedriges Heteroaryl stehen, wobei
   (C₃-C₇)-Cycloalkyl, 4- bis 7-gliedriges Heterocycloalkyl, Phenyl und 5- oder 6-gliedriges Heteroaryl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
   und
   (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cycloalkyl, 4- bis 7-gliedrigem Heterocycloalkyl, Phenyl und/oder 5- oder 6-gliedrigem Heteroaryl substituiert sein kann,
   worin der letztgenannte Heterocycloalkyl-Rest seinerseits bis zu zweifach, gleich oder verschieden, mit (C₁-C₄)-Alkyl substituiert sein kann,
(*iv*) R¹³, R¹⁶, R¹⁷, R¹⁹, R²¹, R²³, R²⁴, R²⁸ und R³² unabhängig voneinander bei jedem einzelnen Auftreten für einen Rest ausgewählt aus der Reihe Wasserstoff und (C₁-C₆)-Alkyl stehen,
   wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
   und/oder worin
(v) R¹² und R¹³, R¹⁵ und R¹⁶, R¹⁷ und R¹⁸, R¹⁹ und R²⁰, R²¹ und R²², R²² und R²³, R²⁴ und R²⁵, R²⁷ und R²⁸ sowie R³¹ und R³² jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,

R⁵ in jedem einzelnen Fall, unabhängig voneinander, für Wasserstoff oder einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino, Mono-(C₁-C₆)-alkylamino, Di-(C₁-C₆)-alkylamino, Hydroxycarbo-nyl und (C₁-C₆)-Alkoxycarbonyl steht und
R⁷ für Wasserstoff oder einen Substituenten ausgewählt aus der Reihe (C₁-C₆)-Alkyl, (C₃-C₇)-Cycloalkyl, 4- bis 7-gliedriges Heterocycloalkyl, Phenyl und 5- oder 6-gliedriges Heteroaryl steht, worin
(i) (C₁-C₆)-Alkyl seinerseits ein- bis dreifach, gleich oder verschieden, mit Resten ausgewählt aus der Reihe Halogen, Cyano, Oxo, (C₃-C₇)-Cycloalkyl, 4- bis 7-gliedriges Heterocycloalkyl, Phenyl, 5- oder 6-gliedriges Heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² substituiert sein kann,
   wobei die letztgenannten Cycloalkyl-, Heterocycloalkyl-, Phenyl- und Heteroaryl-Reste ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
   und
(*ii*) (C₃-C₇)-Cycloalkyl, 4- bis 7-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, Halogen, Cyano, Oxo, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² substituiert sein können,
   wobei der letztgenannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cycloalkyl, 4- bis 7-gliedrigem Heterocycloalkyl, Phenyl und/oder 5- oder 6-gliedrigem Heteroaryl substituiert sein kann,
worin
(*a*) R¹⁰, R¹¹, R¹², R¹⁴, R¹⁵, R¹⁸, R²⁰, R²², R²⁵, R²⁶, R²⁷, R²⁹, R³⁰ und R³¹ unabhängig voneinander bei jedem einzelnen Auftreten für einen Rest ausgewählt aus der Reihe Wasserstoff, (C₁-C₆)-Alkyl, (C₃-C₇)-Cycloalkyl, 4- bis 7-gliedriges Heterocycloalkyl, Phenyl und 5- oder 6-gliedriges Heteroaryl stehen, wobei
   (C₃-C₇)-Cycloalkyl, 4- bis 7-gliedriges Heterocycloalkyl, Phenyl und 5- oder 6-gliedriges Heteroaryl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können und
   (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cycloalkyl, 4- bis 7-gliedrigem Heterocycloalkyl, Phenyl und/oder 5- oder 6-gliedrigem Heteroaryl substituiert sein kann,
(*b*) R¹³, R¹⁶, R¹⁷, R¹⁹, R²¹, R²³, R²⁴, R²⁸ und R³² unabhängig voneinander bei jedem einzelnen Auftreten für einen Rest ausgewählt aus der Reihe Wasserstoff und (C₁-C₆)-Alkyl stehen,
   wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
   und/oder
(c) R¹² und R¹³, R¹⁵ und R¹⁶, R¹⁷ und R¹⁸, R¹⁹ und R²⁰, R²¹ und R²², R²² und R²³, R²⁴ und R²⁵, R²⁷ und R²⁸ sowie R³¹ und R³² jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und
R³ für Wasserstoff, (C₁-C₆)-Alkyl oder (C₃-C₇)-Cycloalkyl steht, sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Trimethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert.*-Butyl, 1-Ethylpropyl, *n*-Pentyl und *n*-Hexyl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy, *tert*.-Butoxy, *n*-Pentoxy und *n-*Hexoxy.

Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, *n*-Propylamino, Isopropylamino, *n*-Butylamino, *tert.*-Butylamino, *n*-Pentylamino und *n*-Hexylamino.

Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N*,*N-*Dimethylamino, *N*,*N-*Diethylamino, *N-*Ethyl-*N-*methylamino, *N-*Methyl-*N-n*-propylamino, *N-*Isopropyl-*N*-*n*-propylamino, *N*,*N-*Diisopropylamino, *N-n*-Butyl*-N-*methylamino, *N-tert.*-Butyl-*N-*methylamino, *N-*Methyl-*N-n*-pentylamino und *N-n*-Hexyl-*N-*methylamino.

(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonyl-Rest mit 1 bis 4 Kohlenstoffatomen in der Alkoxygruppe. Beispielhaft und vorzugsweise seien genannt: Methoxycarbonyl, Ethoxycarbonyl, *n*-Propoxycarbonyl, Isopropoxycarbonyl, *n*-Butoxycarbonyl und *tert.*-Butoxycarbonyl.

(C₃-C₇)-Cycloalkyl und (C₃-C₆)-Cycloalkyl stehen im Rahmen der Erfindung für einen monocyclischen, gesättigten Carbocyclus mit 3 bis 7 bzw. 3 bis 6 Ring-Kohlenstoffatomen. Beispielhaft und vorzugsweise seien genannt: Cyclopropyl, Cyclobutyl, Cyclopentyl, Cyclohexyl und Cycloheptyl.

4- bis 10-gliedriges Heterocycloalkyl steht im Rahmen der Erfindung für einen mono- oder gegebenenfalls bicyclischen, gesättigten oder eine Doppelbindung enthaltenden Heterocyclus mit insgesamt 4 bis 10 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Oxetanyl, Thietanyl, Pyrrolidinyl, Pyrrolinyl, Pyrazolidinyl, Dihydropyrazolyl, Tetrahydrofuranyl, Thiolanyl, 1,3-Oxazolidinyl, 1,3-Thiazolidinyl, Piperidinyl, Tetrahydropyridyl, Piperazinyl, Tetrahydropyranyl, Dihydropyranyl, Tetrahydrothiopyranyl, 1,3-Dioxanyl, 1,4-Dioxanyl, Morpholinyl, Thiomorpholinyl, Hexahydroazepinyl, Hexahydro-1,4-diazepinyl, Octahydroazocinyl, Octahydropyrrolo[3,4-b]pyrrolyl, Octahydroisoindolyl, Octahydropyrrolo[3,4-b]pyridyl, Hexahydropyrrolo[3,4-c]pyridyl, Octahydropyrrolo[1,2-a]pyrazinyl, Decahydroisochinolinyl, Octahydropyrido[1,2-a]pyrazinyl, 7-Azabicyclo[2.2.1]heptanyl, 3-Azabicyclo[3.2.0]heptanyl, 3-Azabicyclo[3.2.1]octanyl, 8-Azabicyclo[3.2.1]octanyl, 8-Oxa-3-azabicyclo[3.2.1]octanyl. Bevorzugt im Rahmen der Erfindung ist ein monocyclischer, gesättigter 4- bis 7-gliedriger Heterocycloalkyl-Rest mit insgesamt 4 bis 7 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Azetidinyl, Oxetanyl, Thietanyl, Pyrrolidinyl, Pyrazolidinyl, Tetrahydrofuranyl, Thiolanyl, 1,3-Oxazolidinyl, Piperidinyl, Piperazinyl, Tetrahydropyranyl, Tetrahydrothiopyranyl, 1,3-Dioxanyl, 1,4-Dioxanyl, Morpholinyl, Thiomorpholinyl, Hexahydroazepinyl, Hexahydro-1,4-diazepinyl. Besonders bevorzugt ist ein 4- bis 6-gliedriger Heterocycloalkyl-Rest mit insgesamt 4 bis 6 Ringatomen, der ein oder zwei Ring-Heteroatome aus der Reihe N und/oder O enthält, wie beispielsweise Pyrrolidinyl, Tetrahydrofuranyl, Piperidinyl, Piperazinyl, Tetrahydropyranyl und Morpholinyl.

5- oder 6-gliedriges Heteroaryl steht im Rahmen der Erfindung für einen aromatischen Heterocyclus (Heteroaromaten) mit insgesamt 5 bzw. 6 Ringatomen, der bis zu vier gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl, Triazinyl. Bevorzugt sind 5- oder 6-gliedrige Heteroaryl-Reste mit bis zu drei Ring-Heteroatomen aus der Reihe N, O und/oder S, wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl.

Halogen schließt im Rahmen der Erfindung Fluor, Chlor, Brom und Iod ein. Bevorzugt sind Fluor, Chlor und Brom, besonders bevorzugt Fluor und Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Besonders bevorzugt ist die Substitution mit einem oder zwei gleichen oder verschiedenen Substituenten.

Bevorzugt im Rahmen der vorliegenden Erfindung sind Verbindungen der Formel (I), in welcher
- R¹: für eine Heteroaryl-Gruppe der Formel 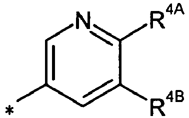 steht, worin
* die Verknüpfungsstelle mit dem Dihydropyrazolon-Ring bedeutet und
R^{4A} und R^{4B} gleich oder verschieden sind und unabhängig voneinander Wasserstoff oder einen Substituenten ausgewählt aus der Reihe Fluor, Chlor, Brom, Cyano, (C₁-C₆)-Alkyl, Hydroxy, (C₁-C₆)-Alkoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl bedeuten,
wobei der genannte (C₁-C₆)-Alkyl-Rest seinerseits bis zu dreifach, gleich oder verschieden, mit Fluor, Chlor, Brom, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
- R²: für eine Heteroaryl-Gruppe der Formel 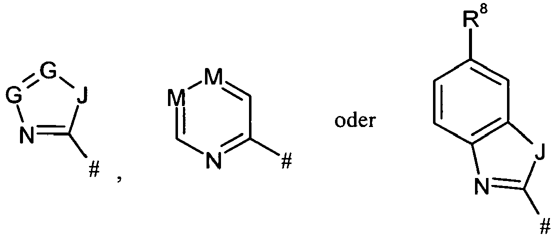 steht, worin
# die Verknüpfungsstelle mit dem Dihydropyrazolon-Ring bedeutet,
G jeweils C-R⁶ oder N bedeutet, wobei nicht mehr als eines der beiden Ringglieder G für N steht,
J O oder S bedeutet,
M jeweils C-R⁹ oder N bedeutet, wobei eines der beiden Ringglieder M für N und das andere für C-R⁹ steht,
worin
R⁶ und R⁹ in jedem einzelnen Fall, unabhängig voneinander, für Wasserstoff oder einen Substituenten ausgewählt aus der Reihe Fluor, Chlor, Brom, Cyano, (C₁-C₆)-Alkyl, (C₃-C₆)-Cycloalkyl, 4- bis 6-gliedriges Heterocycloalkyl, Phenyl, 5- oder 6-gliedriges Heteroaryl, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -OR²⁹ und -NR³¹R³² stehen, worin
(i) (C₁-C₆)-Alkyl seinerseits ein- bis dreifach, gleich oder verschieden, mit Resten ausgewählt aus der Reihe Fluor, Chlor, Brom, Cyano, (C₃-C₆)-Cycloalkyl, 4- bis 6-gliedriges Heterocycloalkyl, Phenyl, 5- oder 6-gliedriges Heteroaryl, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -OR²⁹ und -NR³¹R³² substituiert sein kann,
   wobei die letztgenannten Cycloalkyl-, Heterocycloalkyl-, Phenyl- und Heteroaryl-Reste ihrerseits jeweils bis zu zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*ii*) (C₃-C₆)-Cycloalkyl, 4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*iii*) R¹¹, R¹², R¹⁴, R¹⁵, R¹⁸, R²⁰, R²², R²⁵, R²⁹ und R³¹ unabhängig voneinander bei jedem einzelnen Auftreten für einen Rest ausgewählt aus der Reihe Wasserstoff, (C₁-C₆)-Alkyl, (C₃-C₆)-Cycloalkyl, 4- bis 6-gliedriges Heterocycloalkyl, Phenyl und 5- oder 6-gliedriges Heteroaryl stehen, wobei
   (C₃-C₆)-Cycloalkyl, 4- bis 6-gliedriges Heterocycloalkyl, Phenyl und 5- oder 6-gliedriges Heteroaryl ihrerseits jeweils bis zu dreifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
   und
   (C₁-C₆)-Alkyl ein- bis dreifach, gleich oder verschieden, mit Fluor, Chlor, Brom, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl, 4- bis 6-gliedrigem Heterocycloalkyl, Phenyl und/oder 5- oder 6-gliedrigem Heteroaryl substituiert sein kann,
(*iv*) R¹³, R¹⁶, R¹⁷, R¹⁹, R²¹, R²³, R²⁴ und R³² unabhängig voneinander bei jedem einzelnen Auftreten für einen Rest ausgewählt aus der Reihe Wasserstoff und (C₁-C₆)-Alkyl stehen,
   wobei (C₁-C₆)-Alkyl ein- oder zweifach, gleich oder verschieden, mit Fluor, Chlor, Brom, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
   und/oder worin
(*v*) R¹² und R¹³, R¹⁵ und R¹⁶, R¹⁷ und R¹⁸ R¹⁹ und R²⁰, R²¹ und R²² R²² und R²³, R²⁴ und R²⁵ sowie R³¹ und R³² jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und
R⁸ Wasserstoff oder einen Substituenten ausgewählt aus der Reihe Fluor, Chlor, Brom, Cyano, (C₁-C₆)-Alkyl, Hydroxy, (C₁-C₆)-Alkoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl bedeutet, und
R³ für Wasserstoff oder Methyl steht,
sowie ihre Salze, Solvate und Solvate der Salze.

Besonders bevorzugt im Rahmen der vorliegenden Erfindung sind Verbindungen der Formel (I), in welcher
- R¹: für eine Heteroaryl-Gruppe der Formel 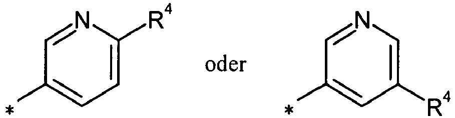 steht, worin
* die Verknüpfungsstelle mit dem Dihydropyrazolon-Ring bedeutet und
R⁴ Wasserstoff, Fluor, Chlor, Brom, Cyano, (C₁-C₄)-Alkyl, Trifluormethyl, Hydroxy-methyl, (C₁-C₄)-Alkoxy, Trifluormethoxy, Hydroxycarbonyl oder (C₁-C₄)-Alkoxy-carbonyl bedeutet,
- R²: für eine Heteroaryl-Gruppe der Formel 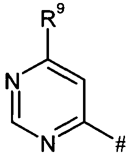 steht, worin
# die Verknüpfungsstelle mit dem Dihydropyrazolon-Ring bedeutet und
R⁹ Wasserstoff, Fluor, Chlor, Brom, Cyano, (C₁-C₄)-Alkyl, Trifluormethyl, Hydroxy, (C₁-C₄)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Hetero-cycloalkyl, Phenyl oder 5- oder 6-gliedriges Heteroaryl bedeutet, wobei
(C₁-C₄)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Tri-fluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können, und
- R³: für Wasserstoff steht,
sowie ihre Salze, Solvate und Solvate der Salze.

Besonders bevorzugt im Rahmen der vorliegenden Erfindung sind auch Verbindungen der Formel (I), in welcher
- R¹: für eine Heteroaryl-Gruppe der Formel 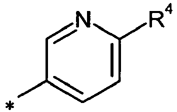 steht, worin
* die Verknüpfungsstelle mit dem Dihydropyrazolon-Ring bedeutet
und
R⁴ Wasserstoff, Fluor, Chlor, Brom, Cyano, (C₁-C₄)-Alkyl, Trifluormethyl, Hydroxy-methyl, (C₁-C₄)-Alkoxy, Trifluormethoxy, Hydroxycarbonyl oder (C₁-C₄)-Alkoxy-carbonyl bedeutet,
- R²: für eine Heteroaryl-Gruppe der Formel 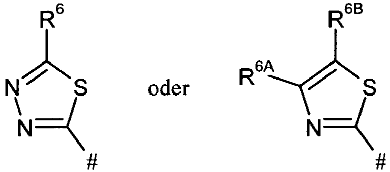 steht, worin
# die Verknüpfungsstelle mit dem Dihydropyrazolon-Ring bedeutet und
R⁶, R^{6A} und R^{6B} gleich oder verschieden sind und unabhängig voneinander Wasserstoff oder einen Substituenten ausgewählt aus der Reihe Fluor, Chlor, Brom, Cyano, (C₁-C₄)-Alkyl, Trifluormethyl, Hydroxy, (C₁-C₄)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl und 5- oder 6-gliedriges Heteroaryl bedeuten, wobei
(C₁-C₄)-Alkyl seinerseits mit Hydroxy, (C₁-C₄)-Alkoxy oder Amino und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können, und
- R³: für Wasserstoff steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen 1,2-Dihydropyrazol-3-on-Derivate der Formel (I) können auch in der tautomeren 1*H-*Pyrazol-5-ol-Form (I') vorliegen (siehe nachfolgendes Schema 1); beide tautomeren Formen werden von der vorliegenden Erfindung ausdrücklich umfasst.

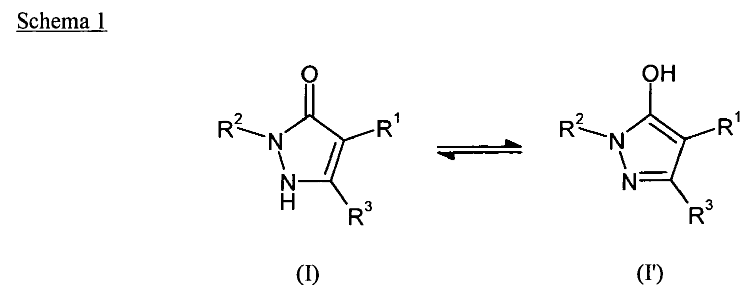

Weiterer Gegenstand der Erfindung ist ein Verfahren zur Herstellung der erfindungsgemäßen Verbindungen der Formel (I), dadurch gekennzeichnet, dass man eine Verbindung der Formel (II) 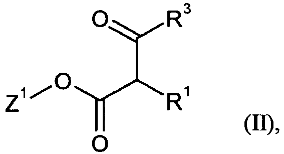 in welcher R¹ und R³ die oben angegebenen Bedeutungen aufweisen und
- Z¹: für Methyl oder Ethyl steht,
in einem inerten Lösungsmittel gegebenenfalls in Gegenwart einer Säure mit einer Verbindung der Formel (III) 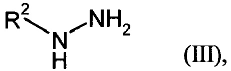 in welcher R² die oben angegebene Bedeutung aufweist,
zu Verbindungen der Formel (IV) 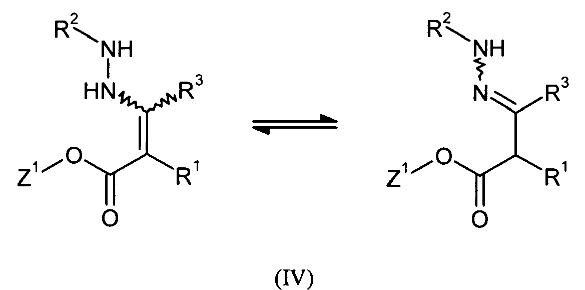 in welcher Z¹, R¹, R² und R³ die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I) cyclisieren,
und die Verbindungen der Formel (I) gegebenenfalls mit den entsprechenden (i) Lösungsmitteln und/oder (ii) Basen oder Säuren in ihre Solvate, Salze und/oder Solvate der Salze überführt.

Die erfindungsgemäßen Verbindungen der Formel (I), in welcher R³ Wasserstoff bedeutet, können auch dadurch hergestellt werden, dass man zunächst eine Verbindung der Formel (V) 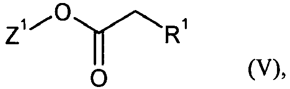 in welcher Z¹ und R¹ die oben angegebenen Bedeutungen aufweisen, mit einer Verbindung der Formel (VI) 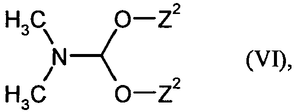 in welcher
- Z²: für Methyl oder Ethyl steht,
zu Verbindungen der Formel (VII) 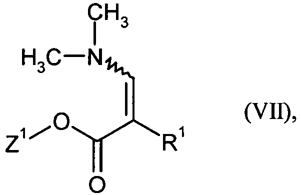 in welcher Z¹ und R¹ die oben angegebenen Bedeutungen aufweisen,
kondensiert und anschließend in Gegenwart einer Säure mit einer Verbindung der Formel (III) zu Verbindungen der Formel (IV-A) 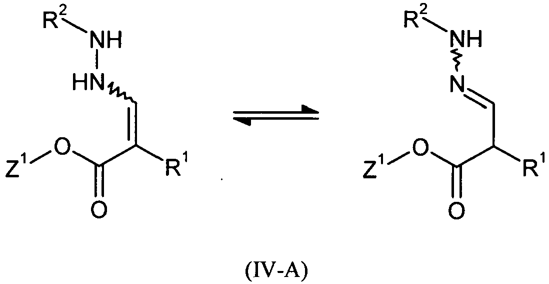 in welcher Z¹, R¹ und R² die oben angegebenen Bedeutungen aufweisen, umsetzt, welche bereits unter diesen Reaktionsbedingungen oder in einem nachfolgenden Reaktionsschritt unter dem Einfluss einer Base zu den Verbindungen der Formel (I), worin R³ für Wasserstoff steht, cyclisieren.

Weitere erfindungsgemäße Verbindungen können gegebenenfalls auch hergestellt werden durch Umwandlungen von funktionellen Gruppen einzelner Substituenten, insbesondere den unter R¹ und R² aufgeführten, ausgehend von den nach obigen Verfahren erhaltenen Verbindungen der Formel (I). Diese Umwandlungen werden nach üblichen, dem Fachmann bekannten Methoden durch-geführt und umfassen beispielsweise Reaktionen wie nukleophile oder elektrophile Substitution, Oxidation, Reduktion, Hydrierung, Übergangsmetall-katalysierte Kupplungsreaktionen, Alkylierung, Acylierung, Aminierung, Veresterung, Esterspaltung, Veretherung, Etherspaltung, Bildung von Carbonamiden, Sulfonamiden, Carbamaten und Harnstoffen, sowie die Einführung und Entfernung temporärer Schutzgruppen.

Als inerte Lösungsmittel für die Verfahrensschritte (II) + (III) → (IV), (IV) → (I), (VII) + (III) → (IV-A) und (IV-A) → (I) eignen sich insbesondere Ether wie Diethylether, Methyl-tert.-butylether, 1,2-Dimethoxyethan, Tetrahydrofuran und Dioxan, oder Alkohole wie Methanol, Ethanol, n-Propanol, iso-Propanol, n-Butanol und tert.-Butanol. Bevorzugt werden Methanol, Ethanol, Tetrahydrofuran oder Gemische dieser Lösungsmittel eingesetzt.

Der Verfahrensschritt (V) + (VI) → (VII) wird bevorzugt in Dimethylformamid als Lösungsmittel oder auch in Gegenwart eines Überschusses an (VI) ohne weiteres Lösungsmittel durchgeführt. Gegebenenfalls kann die Reaktion auch vorteilhaft unter Mikrowellen-Bestrahlung erfolgen. Die Umsetzung geschieht im Allgemeinen in einem Temperaturbereich von +20°C bis +150°C, bevorzugt bei +80°C bis +120°C [vgl. auch J.P. Bazureau et al., Synthesis 1998, 967; *ibid* 2001 (4), 581].

Die Verfahrensschritte (II) + (III) → (IV) und (VII) + (III) → (IV-A) können gegebenenfalls vorteilhaft unter Zusatz einer Säure durchgeführt werden. Hierfür eignen sich übliche anorganische oder organische Säuren wie beispielsweise Chlorwasserstoff, Essigsäure, Trifluoressigsäure, Methansulfonsäure, p-Toluolsulfonsäure oder Campher-10-sulfonsäure. Bevorzugt werden Essigsäure oder insbesondere Campher-10-sulfonsäure oder p-Toluolsulfonsäure verwendet.

Die Umsetzung (II) + (III) → (IV) erfolgt im Allgemeinen in einem Temperaturbereich von 0°C bis +100°C, bevorzugt bei +10°C bis +50°C. Die Reaktion (VII) + (III) → (IV-A) wird im Allgemeinen in einem Temperaturbereich von +20°C bis +120°C, bevorzugt bei +50°C bis +100°C durchgeführt.

Die Verfahrenssequenzen (II) + (III) → (IV) → (I) und (VII) + (III) → (IV-A) → (I) können unter zweistufiger Reaktionsführung oder auch als Eintopf-Reaktion, ohne Isolierung der Zwischenstufe (IV) bzw. (IV-A), durchgeführt werden. Für letztere Variante ist insbesondere die Umsetzung der Komponenten unter Mikrowellen-Bestrahlung geeignet; die Reaktion erfolgt hierbei im Allgemeinen in einem Temperaturbereich von +50°C bis +200°C, bevorzugt bei +100°C bis +180°C.

Teilweise tritt ein Ringschluss zu (I) auch bereits schon bei der Herstellung von (IV) bzw. (IV-A) ein; die Cyclisierung kann dann gegebenenfalls durch *in situ*-Behandlung des Reaktionsgemisches mit einer Base vervollständigt werden.

Als Base für einen solchen separaten Cyclisierungsschritt (IV) → (I) bzw. (IV-A) → (I) eignen sich übliche anorganische oder organische Basen. Hierzu gehören insbesondere Alkalihydroxide wie beispielsweise Natrium- oder Kaliumhydroxid, Alkali- oder Erdalkalicarbonate wie Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, oder Alkalihydride wie Natriumhydrid. Bevorzugt werden Natriummethanolat oder -ethanolat verwendet.

Die Basen-induzierte Umsetzung (IV) → (I) bzw. (IV-A) → (I) erfolgt im Allgemeinen in einem Temperaturbereich von 0°C bis +60°C, bevorzugt bei 0°C bis +30°C.

Alle Verfahrensschritte können bei normalem, erhöhtem oder bei erniedrigtem Druck durchgeführt werden (z.B. von 0.5 bis 5 bar). Im Allgemeinen arbeitet man bei Normaldruck.

Die Verbindungen der Formel (II) können nach literaturüblichen Methoden zur C-Acylierung von Carbonsäureestern aus Verbindungen der Formel (V) hergestellt werden. Die Verbindungen der Formeln (III), (V) und (VI) sind kommerziell erhältlich, literaturbekannt oder können in Analogie zu in der Literatur beschriebenen Verfahren hergestellt werden.

Die Herstellung der erfindungsgemäßen Verbindungen kann durch das folgende Reaktionsschema 2 veranschaulicht werden: 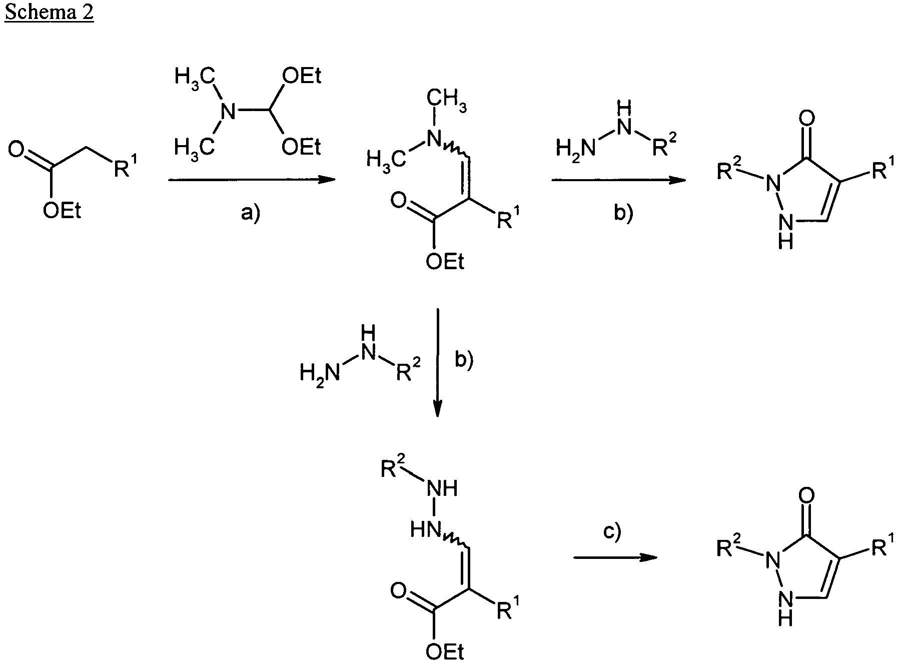
[a): DMF, 16 h, +100°C; b): Ethanol, cat. Campher-10-sulfonsäure, +78°C; c): NaOEt, Ethanol, 1 h, RT].

Die erfindungsgemäßen Verbindungen zeigen ein nicht vorhersehbares, wertvolles pharmakologisches Wirkspektrum. Sie eignen sich daher zur Verwendung als Arzneimittel zur Behandlung und/oder Prophylaxe von Krankheiten bei Menschen und Tieren.

Die erfindungsgemäßen Verbindungen zeichnen sich als spezifische Hemmstoffe der HIF-Prolyl-4-Hydroxylasen aus.

Die erfindungsgemäßen Verbindungen können aufgrund ihrer pharmakologischen Eigenschaften zur Behandlung und/oder Prophylaxe von kardiovaskulären Krankheiten, insbesondere von Herzinsuffizienz, koronarer Herzkrankheit, Angina pectoris, Myokardinfarkt, Schlaganfall, Arteriosklerose, essentieller, pulmonaler und maligner Hypertonie sowie peripherer arterieller Verschlusskrankheit eingesetzt werden.

Die erfindungsgemäßen Verbindungen eignen sich weiterhin zur Behandlung und/oder Prophylaxe von Störungen der Blutbildung, wie z.B. idiopathischen Anämien, renaler Anämie und Anämien in Begleitung einer Tumor-Erkrankung (insbesondere einer Chemotherapie-induzierten Anämie), einer Infektion (insbesondere HIV-Infektion) oder einer anderen entzündlichen Erkrankung wie z.B. rheumatoider Arthritis. Die erfindungsgemäßen Verbindungen sind darüber hinaus geeignet zur unterstützenden Behandlung von Anämien infolge Blutverlust, Eisenmangel-Anämie, Vitaminmangel-Anämie (z.B. infolge Vitamin B12-Mangel oder infolge Folsäure-Mangel), hypoplastischer und aplastischer Anämie, hämolytischer Anämie oder zur unterstützenden Behandlung von Anämien in Folge von Eisenverwertungsstörungen (sideroachrestische Anämie) oder Anämien infolge anderer endokriner Störungen (z.B. Hypothyreose).

Die Verbindungen sind ferner geeignet zur Steigerung des Hämatokrits mit dem Ziel der Gewinnung von Blut zur Eigenblutspende vor Operationen.

Die erfindungsgemäßen Verbindungen können außerdem zur Behandlung und/oder Prophylaxe von operationsbedingten Ischämiezuständen und deren Folgeerscheinungen nach chirurgischen Eingriffen, insbesondere Eingriffen am Herzen unter Verwendung einer Herz-Lungenmaschine (z.B. Bypass-Operationen, Herzklappen-Implantationen), Eingriffen an den Halsschlagadern, Eingriffen an der Körperschlagader (Aorta) und Eingriffen mit instrumenteller Öffnung oder Durchdringung der Schädelkalotte verwendet werden. Die Verbindungen eignen sich ferner zur allgemeinen Behandlung und/oder Prophylaxe bei chirurgischen Eingriffen mit dem Ziel einer Beschleunigung der Wundheilung und Verkürzung der Rekonvaleszenzzeit.

Die Verbindungen sind darüber hinaus zur Behandlung und Prophylaxe von Folgeerscheinungen akuter und protrahierter Ischämiezustände des Gehirns geeignet (z.B. Schlaganfall, Geburtsasphyxie).

Die Verbindungen können ferner zur Behandlung und/oder Prophylaxe von Krebs und zur Behandlung und/oder Prophylaxe einer im Zuge der Behandlung von Krebs auftretenden Beeinträchtigung des Gesundheitszustandes insbesondere nach Therapie mit Zytostatika, Antibiotika und Bestrahlungen eingesetzt werden.

Die Verbindungen eignen sich ferner zur Behandlung und/oder Prophylaxe von Erkrankungen des rheumatischen Formenkreises und anderen den Autoimmunerkrankungen zuzurechnenden Krankheitsformen und insbesondere zur Behandlung und/oder Prophylaxe einer im Zuge der medikamentösen Behandlung derartiger Erkrankungen auftretenden Beeinträchtigung des Gesundheitszustandes.

Weiterhin können die erfindungsgemäßen Verbindungen eingesetzt werden zur Behandlung und/ oder Prophylaxe von Erkrankungen des Auges (z.B. Glaukom), des Gehirns (z.B. Morbus Parkinson, Morbus Alzheimer, Demenz, chronisches Schmerzempfinden), von chronischen Nierenerkrankungen, Niereninsuffizienz und akutem Nierenversagen sowie zur Förderung der Wundheilung.

Weiterhin eignen sich die Verbindungen zur Behandlung und/oder Prophylaxe einer insbesondere im höheren Lebensalter gehäuft auftretenden allgemeinen körperlichen Schwäche bis hin zur Kachexie.

Ferner eignen sich die Verbindungen zur Behandlung und/oder Prophylaxe sexueller Dysfunktion. Außerdem sind die Verbindungen zur Behandlung und/oder Prophylaxe von Diabetes mellitus und seinen Folgeerkrankungen, wie z.B. diabetischer Makro- und Mikroangiopathie, diabetischer Nephropathie und Neuropathie, geeignet.

Weiterhin eignen sich die erfindungsgemäßen Verbindungen zur Behandlung und/oder Prophylaxe von fibrotischen Erkrankungen z.B. des Herzens, der Lunge und der Leber.

Insbesondere sind die erfindungsgemäßen Verbindungen auch zur Prophylaxe und Behandlung der Frühgeborenenretinopathie (Retinopathia praematurorum) geeignet.

Weiterer Gegenstand der vorliegenden Erfindung sind die erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt: ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika sowie Antibiotika.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Phosphodiesterase (PDE)-Inhibitor, wie beispielhaft und vorzugsweise Milrinone, Amrinone, Pimobendan, Cilostazol, Sildenafil, Vardenafil oder Tadalafil, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Tumor-Chemotherapeutikum verabreicht, beispielhaft und vorzugsweise aus der Gruppe der Platin-Komplexe, wie z.B. Cisplatin und Carboplatin, der Alkylantien, wie z.B. Cyclophosphamid und Chlorambucil, der Antimetabolite, wie z.B. 5-Fluoruracil und Methotrexat, der Topoisomerase-Hemmer, wie z.B. Etoposid und Camptothecin, der Antibiotika, wie z.B. Doxorubicin und Daunorubicin, oder der Kinase-Inhibitoren, wie z.B. Sorafenib und Sunitinib.

Bei einer bevorzugten Ausführungsform der Erfindung werden die erfindungsgemäßen Verbindungen in Kombination mit einem Antibiotikum verabreicht, beispielhaft und vorzugsweise aus der Gruppe der Penicilline, Cephalosporine oder Chinolone, wie z.B. Ciprofloxacin und Moxifloxacin.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- cat.: katalytisch
- d: Tag(e)
- DCI: direkte chemische Ionisation (bei MS)
- DMF: Dimethylformamid
- DMSO: Dimethylsulfoxid
- d. Th.: der Theorie (bei Ausbeute)
- EI: Elektronenstoß-Ionisation (bei MS)
- ESI: Elektrospray-Ionisation (bei MS)
- Et: Ethyl
- h: Stunde(n)
- HPLC: Hochdruck-, Hochleistungsflüssigchromatographie
- konz.: konzentriert
- LC-MS: Flüssigchromatographie-gekoppelte Massenspektroskopie
- Meth.: Methode
- min: Minute(n)
- MS: Massenspektroskopie
- NMR: Kernresonanzspektroskopie
- rac: racemisch
- Rₜ: Retentionszeit (bei HPLC)
- RT: Raumtemperatur
- TFA: Trifluoressigsäure
- THF: Tetrahydrofuran

### LC-MS- und HPLC-Methoden:

### Methode 1:

Instrument: Micromass Platform LCZ mit HPLC Agilent Serie 1100; Säule: Thermo Hypersil GOLD 3 µ, 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 2:

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3:

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 6:

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 7:

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 8:

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A; Fluss: 2.5 ml/min; Ofen: 55°C; UV-Detektion: 210 nm.

### Methode 9:

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 10:

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.1 min 100% A → 1.5 min 10% A → 2.2 min 10% A; Fluss: 0.33 ml/min; Ofen: 50°C; UV-Detektion: 210 nm.

### Methode 11:

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 12:

Instrument: Micromass Quattro LCZ mit HPLC Agilent Serie 1100; Säule: Phenomenex Synergi 2.5µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208-400 nm.

### Methode 13 (präparative LCZ-MS):

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Ausgangsverbindungen und Intermediate:

### Beispiel 1A

### (6-Chlorpyridin-3-yl)essigsäureethylester

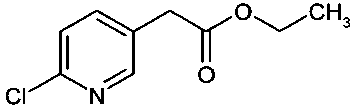

Zu einer Mischung aus 270 ml Ethanol und 101 ml konz. Schwefelsäure werden 22.0 g (144 mmol) (6-Chlorpyridin-3-yl)acetonitril gegeben und der Ansatz 24 h unter Rückfluss gerührt. Danach tropft man das Reaktionsgemisch langsam unter Rühren zu einer Mischung aus 350 g Natriumhydrogencarbonat und 1 Liter Wasser. Die wässrige Phase wird mit Dichlormethan extrahiert (fünfmal je 400 ml). Die vereinigten organischen Phasen werden über Natriumsulfat getrocknet, filtriert und am Rotationsverdampfer vom Lösungsmittel befreit. Man erhält 23.1 g (80% d. Th.) der Titelverbindung, welche ohne weitere Reinigung umgesetzt wird.
¹H-NMR (400 MHz, DMSO-d₆): δ = 8.32 (d, 1H), 7.78 (dd, 1H), 7.49 (d, 1H), 4.10 (q, 2H), 3.77 (s, 2H), 1.19 (t, 3H).
LC-MS (Methode 3): Rₜ = 1.91 min; MS (ESIpos): m/z = 200 [M+H]⁺.

### Beispiel 2A

### 2-(6-Chlorpyridin-3-yl)-3-(dimethylamino)acrylsäureethylester

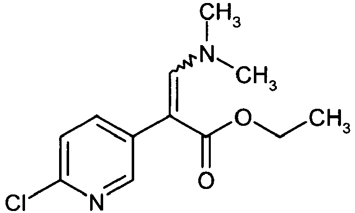

3.99 g (20.0 mmol) der Verbindung aus Beispiel 1A werden in 13.7 ml Dimethylformamid-diethyl-acetal gelöst und der Ansatz unter Mikrowellenbestrahlung 30 min bei 90°C gerührt. Danach wird am Rotationsverdampfer eingeengt und der Rückstand über Kieselgel 60 chromatographiert (Laufmittel: Dichlormethan → Dichlormethan/Methanol 20:1).
Ausbeute: 5.06 g (99% d.Th.)
¹H-NMR (300 MHz, DMSO-d₆): δ = 8.13 (d, 1H), 7.61 (s, 1 H), 7.58 (dd, 1 H), 7.41 (d, 1 H), 4.01 (q, 2H), 2.70 (s, 6H), 1.12 (t, 3H).
LC-MS (Methode 3): Rₜ = 1.98 min; MS (ESIpos): m/z = 255 [M+H]⁺.

### Beispiel 3A

### 3-(Dimethylamino)-2-pyridin-3-yl-acrylsäureethylester

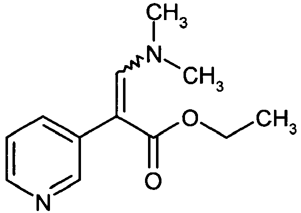

37.4 g (226 mmol) Pyridin-3-ylessigsäureethylester werden in 100 g (679 mmol) Dimethylformamid-diethylacetal über Nacht auf 100°C erhitzt. Nach dem Abkühlen engt man ein und reinigt den Rückstand zunächst mittels Flash-Chromatographie an Kieselgel vor (Laufmittel: Gradient Cyclohexan/Essigsäureethylester 1:1 → Essigsäureethylester/Ethanol 9:1). Das so erhaltene Produkt wird dann durch Vakuumdestillation (1 mbar, 200°C Badtemperatur) feingereinigt.
Ausbeute: 35.0 g (70% d. Th.)
¹H-NMR (400 MHz, DMSO-d₆): δ = 8.37 (dd, 1H), 8.31 (dd, 1H), 7.59 (s, 1H), 7.51 (dt, 1H), 7.29 (ddd, 1H), 4.00 (q, 2H), 2.67 (s, 6H), 1.11 (t, 3H).
LC-MS (Methode 1): Rₜ = 2.38 min; MS (ESIpos): m/z = 221 [M+H]⁺.

### Beispiel 4A

### Benzophenon-(6-chlorpyrimidin-4-yl)hydrazon

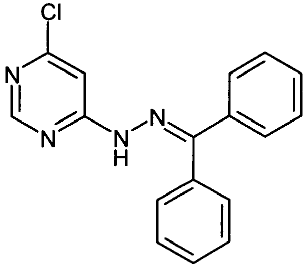

10.0 g (67.1 mmol) 4,6-Dichlorpyrimidin, 14.5 g (73.8 mmol) Benzophenonhydrazon, 9.03 g (94.0 mmol) Natrium-*tert.*-butylat, 409 mg (3.36 mmol) Phenylboronsäure, 301 mg (1.34 mmol) Palladium(II)acetat sowie 384 mg (1.34 mmol) *rac*-2,2'-Bis-(diphenylphosphino)-1,1'-binaphthalin werden zusammengegeben. Man entgast und belüftet zweimal mit Argon, setzt 400 ml trockenes, entgastes Toluol hinzu, entgast und belüftet erneut zweimal mit Argon und erwärmt über Nacht auf 90°C. Nach dem Abkühlen gießt man die Reaktionsmischung in Wasser, extrahiert die wässrige Phase mit Essigsäureethylester, engt die vereinigten organischen Phasen ein und nimmt den Rückstand in einem Gemisch aus Dichlormethan und Diethylether auf. Der verbleibende Niederschlag wird abgesaugt (und verworfen), das Filtrat eingeengt und der Rückstand durch Säulenchromatographie an Kieselgel 60 gereinigt (Laufmittel: Toluol/Essigsäureethylester 8:2).
Ausbeute: 6.00 g (29% d. Th.)
LC-MS (Methode 3): Rₜ = 2.86 min; MS (ESIpos): m/z = 309 [M+H]⁺.

### Beispiel 5A

### Benzophenon-[6-(4-methylpiperazin-1-yl)pyrimidin-4-yl]hydrazon

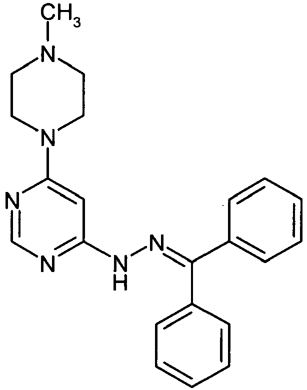

500 mg (1.62 mmol) der Verbindung aus Beispiel 4A, 178 mg (1.78 mmol) *N-*Methylpiperazin, 58 mg (0.12 mmol) Dicyclohexyl-(2',4',6'-triisopropylbiphenyl-2-yl)phosphin, 22 mg (24 µmol) Tris-(dibenzylidenaceton)-dipalladium und 1.32 g (4.05 mmol) Cäsiumcarbonat werden zusammengegeben. Man entgast und belüftet zweimal mit Argon, setzt 12.5 ml einer Mischung aus *tert.-*Butanol und Toluol (1:5) hinzu, entgast und belüftet erneut zweimal mit Argon und erwärmt für 24 h auf 120°C. Anschließend gibt man nochmals 58 mg (0.12 mmol) Dicyclohexyl-(2',4',6'-tri-isopropylbiphenyl-2-yl)phosphin sowie 22 mg (24 µmol) Tris-(dibenzylidenaceton)-dipalladium hinzu, erwärmt über Nacht auf 120°C, setzt dann nochmals 324 mg (3.24 mmol) *N-*Methyl-piperazin hinzu und erwärmt über eine weitere Nacht auf 120°C. Nach dem Abkühlen filtriert man die Reaktionsmischung über Kieselgur, engt das Filtrat ein und reinigt den Rückstand mittels präparativer HPLC (RP18-Säule; Laufmittel: Acetonitril/Wasser-Gradient unter Zusatz von 0.1% konz. Salzsäure).
Ausbeute: 312 mg (52% d. Th.)
LC-MS (Methode 5): Rₜ = 1.64 min; MS (ESIpos): m/z = 373 [M+H]⁺.

### Beispiel 6A

### 4-Hydrazino-6-(4-methylpiperazin-1-yl)pyrimidin

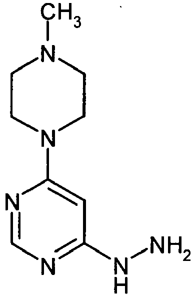

300 mg (808 µmol) der Verbindung aus Beispiel 5A werden in 15 ml konz. Salzsäure 4 h lang auf 65°C erhitzt. Nach dem Abkühlen wird die Reaktionsmischung mit Dichlormethan gewaschen und die wässrige Phase eingeengt. Man erhält 162 mg des Rohproduktes als Hydrochlorid. Dieses wird mit Polymer-gebundenem Tris-(2-aminoethyl)-amin in Dichlormethan bei RT gerührt. Nach Filtration wird das Filtrat eingeengt und der Rückstand im Hochvakuum getrocknet.

Ausbeute: 115 mg (69% d. Th.)
¹H-NMR (400 MHz, DMSO-d₆): δ = 7.93 (s, 1H), 7.64 (s, 1H), 5.91 (s, 1H), 4.13 (s, 2H), 3.48-3.44 (m, 4H), 2.36-2.31 (m, 4H), 2.20 (s, 3H).
LC-MS (Methode 1): Rₜ = 0.41 min; MS (ESIpos): m/z = 209 [M+H]⁺.

### Beispiel 7A

### 4-Chlor-6-hydrazinopyrimidin

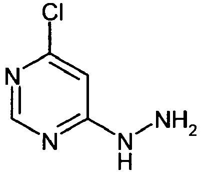

In eine Lösung von 20.0 g (134.3 mmol) 4,6-Dichlorpyrimidin in 300 ml Ethanol werden unter Rühren 11.8 ml (12.1 g, 241.6 mmol) Hydrazinhydrat bei RT getropft. Tritt eine Trübung der Lösung während der Zugabe des Hydrazinhydrats auf, gibt man weiteres Lösungsmittel (ca. 400 ml Ethanol) hinzu. Die Reaktionslösung wird 12 h bei RT nachgerührt. Zur Aufarbeitung wird der ausgefallene Feststoff abfiltriert, der Filterrückstand zweimal mit je 150 ml Wasser und zweimal mit je 100 ml Diethylether gewaschen und das Produkt im Vakuum getrocknet. Aus der eingeengten Mutterlauge wird eine weitere kristalline Produktfraktion erhalten.

Ausbeute: 16.8 g (87% d. Th.)
LC-MS (Methode 1): Rₜ = 1.17 min; MS (ESIpos): m/z = 145 [M+H]⁺;
¹H-NMR (400 MHz, DMSO-d₆): δ = 8.81 (s, 1H), 8.17 (br. s, 1H), 6.75 (s, 1H), 4.48 (br. s, 2H).

### Beispiel 8A

### 4-Hydrazino-6-piperidin-1-ylpyrimidin

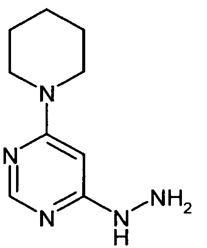

### Stufe a): 4-Chlor-6-piperidin-1-ylpyrimidin

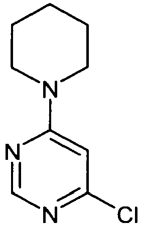

Ein Gemisch aus 10.0 g (67.1 mmol) 4,6-Dichlorpyrimidin und 5.7 g (67.1 mmol) Piperidin in 100 ml Wasser wird 16 h bei einer Badtemperatur von 115°C gerührt. Nach Abkühlen auf RT wird der Niederschlag abfiltriert, mit Wasser gewaschen und im Vakuum getrocknet.

Ausbeute: 6.4 g (47% d. Th.) LC-MS (Methode 4): Rₜ = 2.16 min; MS (ESIpos): m/z = 198 [M+H]⁺;
¹H-NMR (400 MHz, DMSO-d₆): δ = 8.29 (s, 1H), 6.92 (s, 1H), 3.65-3.58 (m, 4H), 1.66-1.62 (m, 2H), 1.60-1.48 (m, 4H).

### Stufe b): 4-Hydrazino-6-piperidin-1-ylpyrimidin

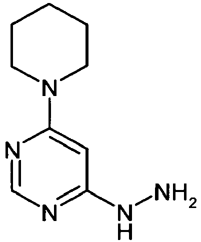

In eine Lösung von 6.0 g (30.4 mmol) 4-Chlor-6-piperidin-1-ylpyrimidin in 50 ml Ethanol werden unter Rühren 17.7 ml (18.2 g, 364.2 mmol) Hydrazinhydrat bei RT getropft. Die Reaktionslösung wird 16 h bei 80°C nachgerührt. Zur Aufarbeitung wird im Vakuum eingeengt, der Rückstand in Wasser verrührt, der ausgefallene Feststoff abfiltriert, der Filterrückstand zweimal mit je 150 ml Wasser und zweimal mit je 100 ml Diethylether gewaschen und das Produkt im Vakuum getrocknet.
Ausbeute: 4.0 g (69% d. Th.)
LC-MS (Methode 1): Rₜ = 2.06 min; MS (ESIpos): m/z = 194 [M+H]⁺;
¹H-NMR (400 MHz, DMSO-d₆): δ = 7.91 (s, 1H), 7.54 (br. s, 1H), 5.89 (s, 1H), 4.11 (br. s, 2H), 3.50-3.47 (m, 4H), 1.61-1.58 (m, 2H), 1.51-1.46 (m, 4H).

### Beispiel 9A

### 4-(6-Hydrazinopyrimidin-4-yl)morpholin

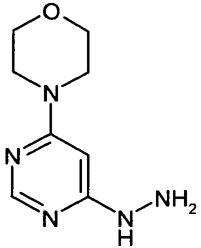

### Stufe a): 4-(6-Chlorpyrimidin-4-yl)morpholin

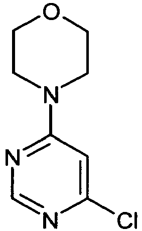

45.0 g (302.1 mmol) 4,6-Dichlorpyrimidin werden in 450 ml Wasser vorgelegt. Man gibt 26.3 g (302.1 mmol) Morpholin hinzu und rührt 16 h bei 90°C. Man kühlt dann auf 0°C ab und filtriert den entstandenen Niederschlag ab. Man wäscht den Niederschlag einmal mit 50 ml Wasser und trocknet ihn an der Luft.

Ausbeute: 51.0 g (85% d. Th.)
LC-MS (Methode 4): Rₜ = 1.09 min; MS (ESIpos): m/z = 200 [M+H]⁺;
¹H-NMR (400 MHz, DMSO-d₆): δ = 8.35 (s, 1H), 6.95 (s, 1H), 3.62 (s, 8H).

### Stufe b): 4-(6-Hydrazinopyrimidin-4-yl)morpholin

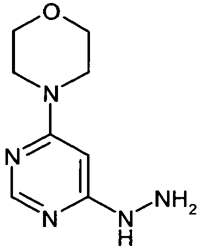

53.0 g (0.27 mol) 4-(6-Chlorpyrimidin-4-yl)morpholin werden in 260 ml Ethanol vorgelegt. Man gibt 132.9 g (2.7 mol) Hydrazinhydrat hinzu und rührt 16 h unter Rückfluss. Man kühlt auf RT ab und entfernt destillativ die Hälfte des Lösungsmittels. Man kühlt dann auf 0°C ab und filtriert den entstandenen Feststoff ab. Man wäscht mit kaltem Ethanol nach und trocknet den Feststoff zunächst an der Luft und anschließend im Vakuum.
Ausbeute: 35.0 g (68% d. Th.)
LC-MS (Methode 1): Rₜ = 0.17 min; MS (ESIpos): m/z = 196 [M+H]⁺;
¹H-NMR (400 MHz, DMSO-d₆): δ = 7.94 (s, 1H), 7.70 (s, 1H), 5.91 (s, 1H), 4.15 (s, 2H), 3.66-3.60 (m, 4H), 3.45-3.37 (m, 4H).

### Beispiel 10A

### (5-Brompyridin-3-yl)essigsäureethylester

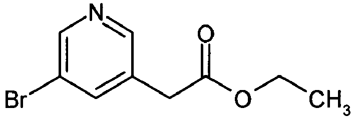

5.0 g (23.1 mmol) (5-Brompyridin-3-yl)essigsäure werden in 30 ml Ethanol mit 25 Tropfen konz. Schwefelsäure 16 h in der Siedehitze gerührt. Zur Aufarbeitung engt man die Reaktionsmischung im Vakuum ein, nimmt den Rückstand in Essigsäureethylester auf, wäscht mehrmals mit halbkonzentrierter Natriumhydrogencarbonat-Lösung, trocknet die organische Phase über Natriumsulfat, filtriert das Trockenmittel ab, entfernt das Lösungsmittel vollständig am Rotationsverdampfer und trocknet das Produkt 16 h im Vakuum.
Ausbeute: 5.2 g (91% d. Th.)
LC-MS (Methode 1): Rₜ = 1.48 min; MS (ESIpos): m/z = 246 [M+H]⁺;
¹H-NMR (400 MHz, DMSO-d₆): δ = 8.59 (d, 1H), 8.48 (d, 1H), 8.00 (dd, 1H), 4.11 (q, 2H), 3.78 (s, 2H), 1.21 (t, 3H).

### Beispiel 11A

### 3-(Dimethylamino)-2-(5-brompyridin-3-yl)-acrylsäureethylester

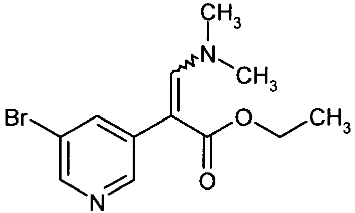

5.1 g (20.9 mmol) der Verbindung aus Beispiel 10A werden in 7.2 ml (6.2 g, 41.8 mmol) Dimethylformamid-diethylacetal 16 h bei 100°C Badtemperatur gerührt. Nach dem Abkühlen engt man im Vakuum ein, verrührt den Rückstand in Diisopropylether, filtriert den Feststoff ab und wäscht diesen abschließend mit Diisopropylether. Das Rohprodukt wird 16 h im Vakuum getrocknet.

Ausbeute: 6.1 g (73% d. Th.)
LC-MS (Methode 7): Rₜ = 1.86 min; MS (ESIpos): m/z = 299 [M+H]⁺;
¹H-NMR (400 MHz, DMSO-d₆): δ = 8.49 (d, 1H), 8.29 (d, 1H), 7.78 (dd, 1H), 7.61 (s, 1H), 4.02 (q, 2H), 2.71 (s, 6H), 1.12 (t, 3H).

### Beispiel 12A

### 2-Hydrazinopyrazin

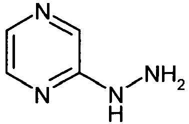

Zu 61.7 g (1.2 mol) Hydrazinhydrat tropft man 20.0 g (174.6 mmol) Chlorpyrazin und rührt 45 min bei 120°C. Anschließend wird das Gemisch 24 h bei 2°C stehen gelassen. Man filtriert den Feststoff ab und wäscht zweimal mit Petrolether. Man trocknet zuerst an der Luft und dann im Hochvakuum. Der Feststoff wird anschließend aus Toluol umkristallisiert und erneut im Hochvakuum getrocknet.
Ausbeute: 6.5 g (34% d. Th.)
LC-MS (Methode 1): Rₜ = 0.41 min; MS (ESIpos): m/z = 111 [M+H]⁺.

### Beispiel 13A

### 2-Hydrazinochinoxalin

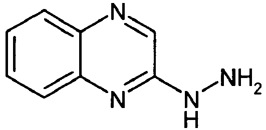

15.0 g (91.1 mmol) 2-Chlorchinoxalin werden in 150 ml Ethanol vorgelegt. Man gibt 45.6 g (911.3 mmol) Hydrazinhydrat hinzu und rührt 16 h unter Rückfluss. Man kühlt danach auf 0°C ab, filtriert den entstandenen Feststoff ab, wäscht ihn mit Ethanol und trocknet im Hochvakuum.

Ausbeute: 11.5 g (79% d. Th.)
LC-MS (Methode 1): Rₜ = 1.75 min; MS (ESIpos): m/z = 161 [M+H]⁺;
¹H-NMR (400 MHz, DMSO-d₆): δ = 8.70 (s, 1H), 8.35 (s, 1H), 7.78 (d, 1H), 7.60-7.50 (m, 2H), 7.37-7.28 (m, 1H), 4.50-4.38 (m, 2H).

### Beispiel 14A

### 2-Hydrazinochinolin

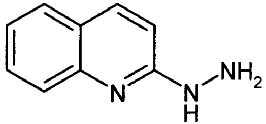

21.0 g (128.4 mmol) 2-Chlorchinolin werden in 210 ml Ethanol vorgelegt. Man gibt 64.3 g (1.3 mol) Hydrazinhydrat hinzu und rührt 16 h unter Rückfluss. Man kühlt danach auf 0°C ab, filtriert den entstandenen Feststoff ab und wäscht ihn mit wenig Ethanol. Man trocknet zunächst an der Luft und anschließend im Hochvakuum.
Ausbeute: 14.5 g (71% d. Th.)
LC-MS (Methode 1): Rₜ = 1.95 min; MS (ESIpos): m/z = 160 [M+H]⁺;
¹H-NMR (400 MHz, DMSO-d₆): δ = 8.08 (br. s, 1H), 7.87 (d, 1H), 7.63 (d, 1H), 7.57-7.43 (m, 2H), 7.16 (t, 1H), 6.85 (d, 1H), 4.35 (br. s, 2H).

### Ausführungsbeispiele:

### Beispiel 1

### 2-(6-Piperidin-1-yl-pyrimidin-4-yl)-4-pyridin-3-yl-1,2-dihydro-3H-pyrazol-3-on-Hydrochlorid

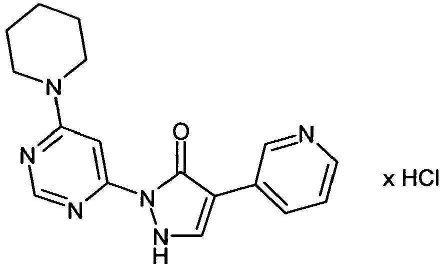

137 mg (621 µmol) der Verbindung aus Beispiel 3A, 100 mg (517 µmol) 4-Hydrazino-6-piperidin-1-yl-pyrimidin [Postovskii, I.Ya., Smirnova, N.B., DokladyAkademiiNaukSSSR 1966, 166, 1136-1139; Chem. Abstr. 64:93457 (1966)] und 12 mg (52 µmol) Campher-10-sulfonsäure werden in 3.5 ml wasserfreiem Ethanol gelöst und über Nacht unter Rückfluss erhitzt. Nach dem Abkühlen engt man ein und reinigt den Rückstand mittels präparativer HPLC (RP18-Säule; Laufmittel: Acetonitril/Wasser-Gradient unter Zusatz von 0.1% konz. Salzsäure). Man erhält 108 mg (58% d. Th.) der Titelverbindung.
¹H-NMR (400 MHz, DMSO-d₆): δ = 9.27 (s, 1H), 8.85 (d, 1H), 8.52 (s, 1H), 8.48 (s, 1H), 8.45 (d, 1H), 7.93 (dd, 1H), 7.41 (s, 1H), 3.83-3.70 (m, 4H), 1.73-1.56 (m, 6H).
LC-MS (Methode 4): Rₜ = 1.13 min; MS (ESIpos): m/z = 323 [M+H]⁺.

Die in der folgenden Tabelle 1 aufgeführten Verbindungen werden aus den entsprechenden Edukten analog zu Beispiel 1 hergestellt. Die Reinigung des jeweiligen Rohprodukts kann mittels präparativer HPLC mit oder ohne Zusatz von 0.1% konz. Salzsäure erfolgen (Methode A). In einer alternativen Aufarbeitung wird nach dem Abkühlen der entstandene Niederschlag abgesaugt, mit Ethanol und/oder Diethylether gewaschen, getrocknet und gegebenenfalls mittels präparativer HPLC (RP18-Säule; Laufmittel: Acetonitril/Wasser-Gradient mit oder ohne Zusatz von 0.1% konz. Salzsäure) feingereinigt (Methode B).

**Tabelle 1**

| **Beispiel Nr.** | **Struktur** | **Edukte; Ausbeute (% d.Th.), Methode** | **MS (ESI) [M+H]⁺; LC-MS Rₜ (Meth.)** | **¹H-NMR** (400 MHz, DMSO-d₆) |
|---|---|---|---|---|
| **2** | 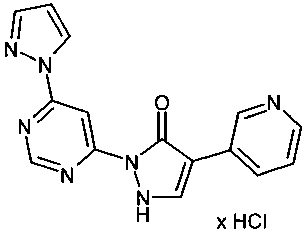 | 3A, a; 19%, A | m/z = 306; 1.08 min (4) | δ = 9.37 (s, 1H), 8.98 (d, 1H), 9.02-8.95 (m, 2H), 8.88 (d, 1H), 8.72 (d, 1H), 8.62 (d, 1H), 8.00 (s, 1H), 7.96-7.90 (m, 1H), 6.70 (s, 1H). |
| **3** | 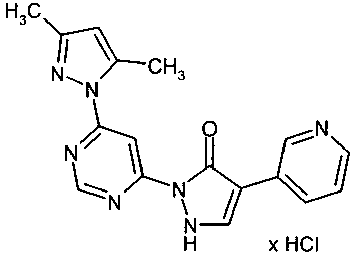 | 3A, a; 18%, A | m/z = 334; 1.38 min (4) | δ = 9.42 (s, 1H), 9.02-8.95 (m, 3H), 8.90 (s, 1H), 8.67 (d, 1H), 8.03 (dd, 1H), 6.26 (s, 1H), 2.70 (s, 3H), 2.26 (s, 3H). |
| **4** | 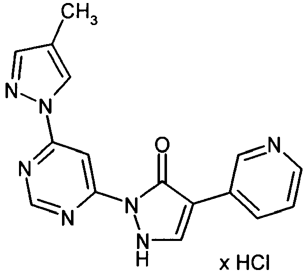 | 3A, a; 21%, A | m/z = 320; 1.43 min (3) | δ = 9.40 (s, 1H), 9.00-8.95 (m, 3H), 8.89 (s, 1H), 8.67 (d, 1H), 8.48 (s, 1H), 8.02 (dd, 1H), 7.83 (s, 1H), 2.14 (s, 3H). |
| **5** | 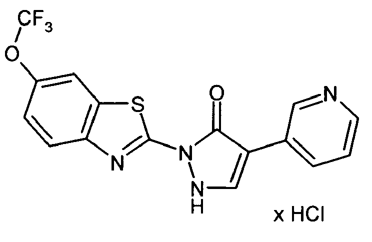 | 3A, b; 4%, A | m/z = 379; 1.63 min (4) | δ = 9.33 (s, 1H), 8.87 (d, 1H), 8.73 (s, 1H), 8.52 (d, 1H), 8.22 (s, 1H), 7.98-7.90 (m, 2H), 7.48 (d, 1H). |
| **6** | 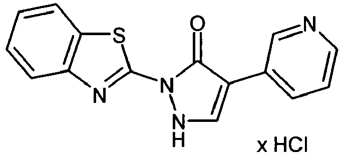 | 3A; 18%, A | m/z = 295; 1.46 min (5) | δ = 9.36 (s, 1H), 8.96-8.88 (m, 2H), 8.61 (d, 1H), 8.12 (d, 1H), 8.01 (dd, 1H), 7.89 (d, 1H), 7.53 (t, 1H), 7.41 (t, 1H). |
| **7** | 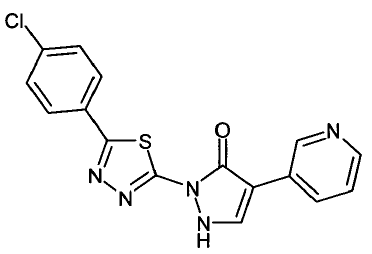 | 3A, c; 10%, B | m/z = 356; 1.72 min (5) | δ = 9.01 (d, 1H), 8.13 (d, 1H), 8.05 (d, 1H), 8.01-7.95 (m, 3H), 7.62-7.57 (m, 2H), 7.17 (dd, 1H). |
| **8** | 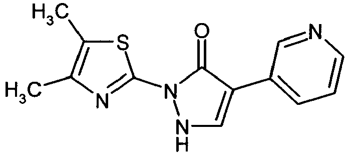 | 3A, d; 60%, B | m/z = 273; 0.83 min (4) | δ = 9.14 (s, 1H), 8.47-8.35 (m, 2H), 8.33-8.29 (m, 1H), 7.52-7.45 (m, 1H), 2.31(s, 3H), 2.24 (s, 3H). |
| **9** | 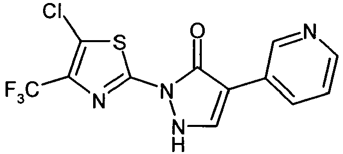 | 3A, e; 7%, B | m/z = 347; 1.53 min (4) | δ = 9.00 (s, 1H), 8.22 (d, 1H), 8.10 (d, 1H), 8.03 (s, 1H), 7.29 (dd, 1H). |
| **10** | 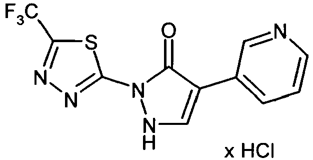 | 3A; 43%, B | m/z = 314; 1.36 min (3) | δ = 9.21 (s, 1H), 8.75 (d, 1H), 8.36-8.27 (m, 2H), 7.88-7.79 (m, 1H). |
| **11** | 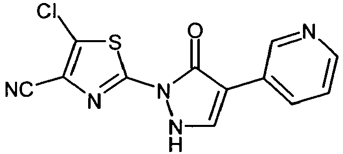 | 3A, e; 4%, B | m/z = 304; 1.22 min (4) | δ = 8.96 (d, 1H), 8.11-8.04 (m, 2H), 8.02 (s, 1H), 7.17 (dd, 1H). |
| **12** | 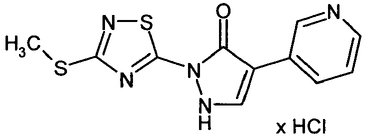 | 3A, f; 12%, B | m/z = 292; 1.21 min (3) | (DCOOD): δ = 10.1 (s, 1H), 9.62 (d, 1H), 9.49-9.35 (m, 2H), 8.87-8.77 (m, 1H), 3.30 (s, 3H). |
| **13** | 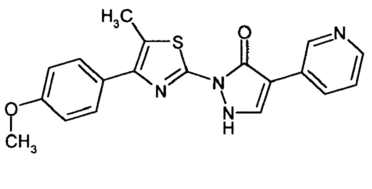 | 3A; 35%, B | m/z = 365; 1.49 min (4) | δ = 9.19 (s, 1H), 8.51-8.27 (m, 3H), 7.69-7.63 (m, 2H), 7.59-7.52 (m, 1H), 7.06-7.01 (m, 2H), 3.81 (s, 3H). |

### zur Synthese der entsprechenden Hydrazinopyrimidin-Derivate:

a): die Synthese des entsprechenden Hydrazinopyrimidins kann analog zu Beispiel 6A erfolgen;
b): 2-Hydrazino-6-trifluormethoxy-benzothiazol: S. Mignani et al., Synth. Commun. 1992, 22, 2769-2780;
c): 2-Hydrazino-5-(4-chlorphenyl)-1,3,4-thiadiazol: S. Turner et al., J. Med. Chem. 1988, 31, 902-906;
d): 2-Hydrazino-4,5-dimethyl-thiazol: Beyer et al., Chem. Ber. 1954, 87, 1385;
e): 5-Chlor-2-hydrazino-4-trifluormethyl-thiazol und 5-Chlor-4-cyano-2-hydrazino-thiazol: DE 39 40 794-A1;
f): 5-Hydrazino-3-methylthio-1,2,4-thiadiazol: K.T. Potts, R. Armbruster, J. Heterocycl. Chem. 1972, 9, 651-7.

### Beispiel 14

### 2-(6-Pyrrolidin-1-yl-pyrimidin-4-yl)-4-pyridin-3-yl-1,2-dihydro-3H-pyrazol-3-on-Hydrochlorid

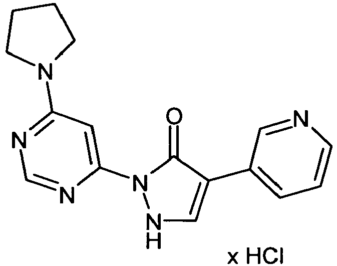

148 mg (670 µmol) der Verbindung aus Beispiel 3A, 100 mg (558 µmol) 4-Hydrazino-6-pyrrolidin-1-yl-pyrimidin [Postovskii, I.Ya., Smirnova, N.B., Doklady Akademii Nauk SSSR 1966, 166, 1136-1139; Chem. Abstr. 64:93457 (1966)] und 13 mg (56 µmol) Campher-10-sulfonsäure werden in 3.7 ml wasserfreiem Ethanol gelöst und über Nacht unter Rückfluss erhitzt. Nach dem Abkühlen engt man ein, nimmt den Rückstand in 5 ml Ethanol auf, setzt 0.25 ml (837 µmol) einer 21%-igen ethanolischen Natriumethylat-Lösung hinzu und rührt 1 h bei RT. Anschließend stellt man durch Zugabe von 1 M Salzsäure einen pH-Wert von 5-6 ein, engt ein und reinigt den Rückstand mittels präparativer HPLC (RP18-Säule; Laufmittel: Acetonitril/Wasser-Gradient unter Zusatz von 0.1% konz. Salzsäure). Man erhält 30 mg (16% d. Th.) der Titelverbindung.
¹H-NMR (400 MHz, DMSO-d₆): δ = 9.26 (s, 1H), 8.83 (d, 1H), 8.53 (s, 1H), 8.46 (s, 1H), 8.43 (d, 1H), 7.92 (dd, 1H), 7.09 (s, 1H), 3.75-3.45 (m, 4H), 2.10-1.91 (m, 4H).
LC-MS (Methode 4): Rₜ = 0.94 min; MS (ESIpos): m/z = 309 [M+H]⁺.

Die in der folgenden Tabelle 2 aufgeführten Verbindungen werden aus den entsprechenden Edukten analog zu Beispiel 14 hergestellt. Alternativ kann als Base eine entsprechende Menge methanolische Natriummethanolat-Lösung und als Lösungsmittel Methanol verwendet werden und die Reinigung mittels präparativer HPLC ohne Zusatz von 0.1% konz. Salzsäure erfolgen.

**Tabelle 2**

| **Beispiel Nr.** | **Struktur** | **Edukte; Ausbeute (% d. Th.)** | **MS (ESI) [M+H]⁺; LC-MS Rₜ (Meth.)** | **¹H-NMR** (400 MHz, DMSO-d₆) |
|---|---|---|---|---|
| **15** | 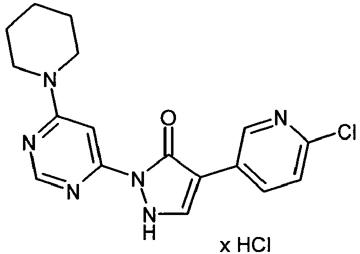 | 2A, g; 98% | m/z = 357; 2.56 min (3) | δ = 8.89 (d, 1H), 8.48 (s, 1H), 8.45 (s, 1H), 8.28 (dd, 1H), 7.46 (d, 1H), 7.42 (s, 1H), 3.74-3.66 (m, 4H), 1.71-1.49 (m, 6H). |
| **16** | 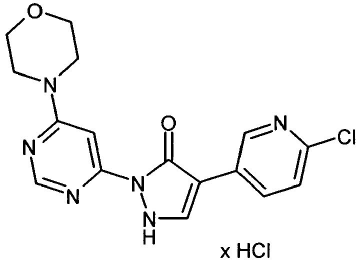 | 2A, g; 86% | m/z = 359; 2.09 min (3) | δ = 8.91 (s, 1H), 8.53-8.46 (m, 2H), 8.29 (d, 1H), 7.53-7.44 (m, 2H), 3.74-3.64 (m, 8H). |
| **17** | 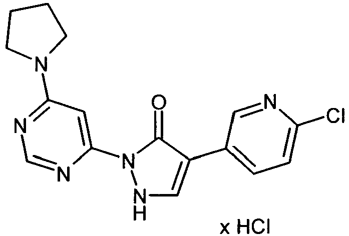 | 2A, g; 99% | m/z = 343; 2.19 min (3) | δ = 8.88 (s, 1H), 8.47 (s, 1H), 8.37 (s, 1H), 8.27 (d, 1H), 7.44 (d, 1H), 7.08 (s, 1H), 3.66-3.40 (m, 4H), 2.07-1.90 (m, 4H). |

g): 4-Hydrazino-6-piperidin-1-yl-pyrimidin, 4-Hydrazino-6-pyrrolidin-1-yl-pyrimidin und 4-Hydrazino-6-morpholin-4-yl-pyrimidin: Postovskii, I.Ya., Smirnova, N.B., Doklady Akademii NaukSSSR 1966, 166, 1136-1139; Chem. Abstr. 64:93457 (1966).

### Beispiel 18

### 2-(6-Morpholin-4-yl-pyrimidin-4-yl)-4-pyridin-3-yl-1,2-dihydro-3H-pyrazol-3-on-Hydrochlorid

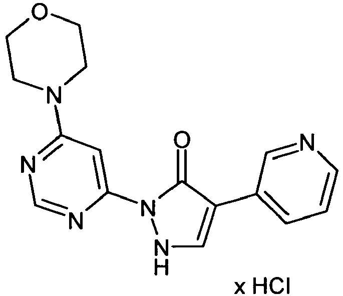

677 mg (3.07 mmol) der Verbindung aus Beispiel 3A, 500 mg (2.56 mmol) 4-Hydrazino-6-morpholin-4-yl-pyrimidin [Postovskii, I.Ya., Smirnova, N.B., Doklady Akademii Nauk SSSR 1966, 166, 1136-1139; Chem. Abstr. 64:93457 (1966)] und 60 mg (256 µmol) Campher-10-sulfonsäure werden in 20 ml wasserfreiem Ethanol gelöst und über Nacht unter Rückfluss erhitzt. Nach dem Abkühlen engt man ein, suspendiert den Rückstand in wenig Ethanol, saugt den Niederschlag ab, wäscht mit Ethanol und Diethylether, suspendiert erneut in Methanol, setzt einen Überschuss einer 4 N Lösung von Chlorwasserstoff in 1,4-Dioxan hinzu und engt wiederum ein. Man erhält so 423 mg (46% d. Th.) der Titelverbindung.
¹H-NMR (400 MHz, DMSO-d₆): δ = 9.29 (s, 1H), 8.87 (s, 1H), 8.57-8.55 (m, 2H), 8.50 (d, 1H), 7.96 (dd, 1H), 7.47 (s, 1H), 5.20-4.40 (m, 4H), 3.77-3.70 (m, 4H).
LC-MS (Methode 3): Rₜ = 0.94 min; MS (ESIpos): m/z = 325 [M+H]⁺.

### Beispiel 19

### 2-[6-(4-Methylpiperazin-1-yl)-pyrimidin-4-yl]-4-pyridin-3-yl-1,2-dihydro-3H-pyrazol-3-on-Dihydrochlorid

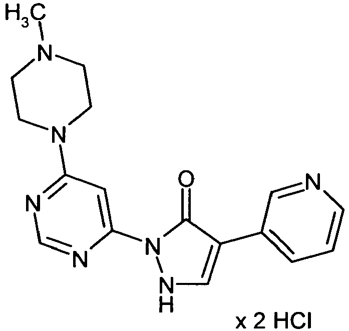

146 mg (663 µmol) der Verbindung aus Beispiel 3A, 115 mg (552 µmol) der Verbindung aus Beispiel 6A und 13 mg (55 µmol) Campher-10-sulfonsäure werden in 5 ml wasserfreiem Ethanol gelöst und über Nacht unter Rückfluss erhitzt. Nach dem Abkühlen engt man ein und reinigt den Rückstand mittels präparativer HPLC (RP18-Säule; Laufmittel: Acetonitril/Wasser-Gradient unter Zusatz von 0.1% konz. Salzsäure). Das erhaltene Gemisch aus Zielprodukt und Zwischenverbindung wird in 5 ml wasserfreiem Ethanol gelöst, mit 109 mg (607 µmol) einer 30%-igen methanolischen Natriummethanolat-Lösung versetzt und 1 h bei RT gerührt. Anschließend neutralisiert man mit 1 M Salzsäure, engt ein und reinigt den Rückstand mittels präparativer HPLC (RP18-Säule; Laufmittel: Acetonitril/Wasser-Gradient unter Zusatz von 0.1% konz. Salzsäure). Das wiederum erhaltene Gemisch aus Zielprodukt und Zwischenverbindung wird abermals in 5 ml wasserfreiem Ethanol gelöst, mit 99 mg (552 µmol) einer 30%-igen methanolischen Natriummethanolat-Lösung versetzt und 2 h bei RT gerührt. Anschließend neutralisiert man wieder mit 1 M Salzsäure, engt ein und reinigt den Rückstand mittels präparativer HPLC (RP18-Säule; Laufmittel: Acetonitril/Wasser-Gradient unter Zusatz von 0.1% konz. Salzsäure). Man erhält so 9 mg (4% d. Th.) der Titelverbindung.
¹H-NMR (400 MHz, DMSO-d₆): δ = 11.1 (s, 1H), 9.30 (s, 1H), 8.84 (d, 1H), 8.67 (s, 1H), 8.62 (s, 1H), 8.55 (d, 1H), 7.93 (dd, 1H), 7.64 (s, 1H), 4.62-4.50 (m, 2H), 3.57-3.44 (m, 4H), 3.18-3.05 (m, 2H), 2.81 (s, 3H).
LC-MS (Methode 1): Rₜ = 1.97 min; MS (ESIpos): m/z = 338 [M+H]⁺.

### Beispiel 20

### 2-(4-Hydroxychinazolin-2-yl)-4-pyridin-3-yl-1,2-dihydro-3H-pyrazol-3-on-Hydrochlorid

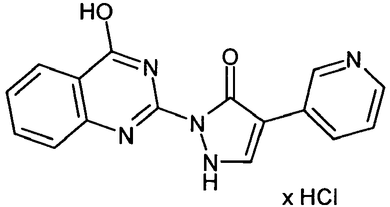

200 mg (908 µmol) der Verbindung aus Beispiel 3A, 133 mg (757 µmol) 2-Hydrazinochinazolin-4(3*H*)-on und 18 mg (76 µmol) Campher-10-sulfonsäure werden in 5 ml wasserfreiem Ethanol gelöst und über Nacht unter Rückfluss erhitzt. Nach dem Abkühlen saugt man den Niederschlag ab, wäscht ihn mit Diethylether, trocknet und reinigt den Niederschlag mittels präparativer HPLC vor (RP18-Säule; Laufmittel: Acetonitril/Wasser-Gradient unter Zusatz von 0.1% konz. Salzsäure). Die Produktfraktionen werden eingeengt, mit einem Überschuss einer 4 N Lösung von Chlorwasserstoff in 1,4-Dioxan versetzt und erneut eingeengt. Der Rückstand wird mit Diethylether gewaschen und getrocknet. Man erhält 75 mg (28% d. Th.) der Titelverbindung.
¹H-NMR (400 MHz, DMSO-d₆): δ = 9.10 (s, 1H), 8.39 (d, 1H), 8.21 (d, 1H), 8.08 (s, 1H), 8.06 (d, 1H), 7.74 (t, 1H), 7.59 (d, 1H), 7.48-7.42 (m, 1H), 7.35 (t, 1H).
LC-MS (Methode 1): Rₜ = 2.61 min; MS (ESIpos): m/z = 306 [M+H]⁺.

### Beispiel 21

### 2-[5-(4-Fluorphenyl)-1,3,4-thiadiazol-2-yl]-4-pyridin-3-yl-1,2-dihydro-3H-pyrazol-3-on-Hydro-chlorid

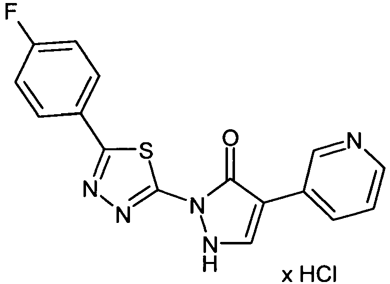

200 mg (908 µmol) der Verbindung aus Beispiel 3A, 159 mg (757 µmol) 2-(4-Fluorphenyl)-5-hydrazino-1,3,4-thiadiazol [zur Herstellung vgl. WO 2001/062208] und 18 mg (76 µmol) Campher-10-sulfonsäure werden in 5 ml wasserfreiem Ethanol gelöst und über Nacht unter Rückfluss erhitzt. Nach dem Abkühlen trennt man den Niederschlag ab, engt das Filtrat ein, wäscht den Filtrat-Rückstand mit Acetonitril und versetzt mit einem Überschuss einer 4 N Lösung von Chlorwasserstoff in 1,4-Dioxan. Nach erneutem Einengen wird der Rückstand mit Diethylether gewaschen und getrocknet. Man erhält 80 mg (31% d. Th.) der Titelverbindung.
¹H-NMR (400 MHz, DMSO-d₆): δ = 9.27 (s, 1H), 8.79 (d, 1H), 8.40 (s, 1H), 8.37 (d, 1H), 8.06-8.00 (m, 2H), 7.86 (dd, 1H), 7.43-7.36 (m, 2H).
LC-MS (Methode 1): Rₜ = 2.80 min; MS (ESIpos): m/z = 340 [M+H]⁺.

### Beispiel 22

### 2-(6-Phenylpyridazin-3-yl)-4-pyridin-3-yl-1,2-dihydro-3H-pyrazol-3-on-Hydrochlorid

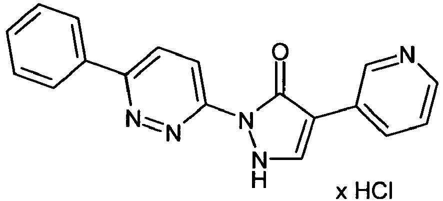

142 mg (644 µmol) der Verbindung aus Beispiel 3A, 100 mg (537 µmol) 3-Hydrazino-6-phenylpyridazin und 13 mg (54 µmol) Campher-10-sulfonsäure werden in 4 ml wasserfreiem Ethanol gelöst und über Nacht unter Rückfluss erhitzt. Nach dem Abkühlen engt man ein, versetzt den Rückstand mit einer Mischung aus Methanol und Acetonitril und stellt durch Zugabe von 1 M Salzsäure einen pH-Wert von 5 ein. Der Niederschlag wird abgesaugt und getrocknet. Man erhält 123 mg (65% d. Th.) der Titelverbindung.
¹H-NMR (400 MHz, DMSO-d₆): δ = 9.40 (s, 1H), 8.97-8.92 (m, 2H), 8.83 (d, 1H), 8.64 (d, 1H), 8.51 (d, 1H), 8.21-8.15 (m, 2H), 7.98 (dd, 1H), 7.64-7.54 (m, 3H).
LC-MS (Methode 4): Rₜ = 1.24 min; MS (ESIpos): m/z = 316 [M+H]⁺.

### Beispiel 23

### 2-(6-Chlorpyrimidin-4-yl)-4-pyridin-3-yl-1,2-dihydro-3H-pyrazol-3-on

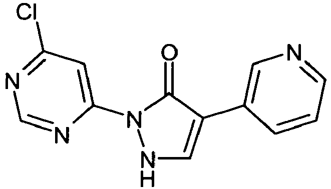

30.0 g (207.5 mmol) der Verbindung aus Beispiel 7A und 50.3 g (228.3 mmol) der Verbindung aus Beispiel 3A werden in 1000 ml Eisessig 16 h bei RT gerührt. Zur Aufarbeitung wird das Lösungsmittel am Rotationsverdampfer entfernt, der Rückstand mit Essigsäureethylester aufgenommen, mit gesättigter Natriumhydrogencarbonat-Lösung neutral gewaschen und die organische Phase im Vakuum eingeengt. Man löst den Rückstand in 1000 ml Ethanol, gibt 42.8 ml (41.1 g, 228.3 mmol) 30%-ige methanolische Natriummethylat-Lösung hinzu und rührt 2 h bei RT. Die Reaktionsmischung wird dann mit 1 N Salzsäure auf pH 5 gestellt und 16 h gerührt. Der Niederschlag wird abfiltriert, der Filterrückstand mit Wasser und Ethanol gewaschen und das Produkt im Vakuum getrocknet.
Ausbeute: 43.5 g (77% d. Th.)
LC-MS (Methode 1): Rₜ = 2.19 min; MS (ESIpos): m/z = 274 [M+H]⁺;
¹H-NMR (400 MHz, DMSO-d₆): δ = 9.18 (s, 1H), 8.78 (s, 1H), 8.70 (s, 1H), 8.20 (d, 1H), 8.08 (d, 1H), 8.02 (s, 1H), 7.22 (t, 1H).

### Beispiel 24

### 2-(6-Hydroxypyrimidin-4-yl)-4-pyridin-3-yl-1,2-dihydro-3H-pyrazol-3-on

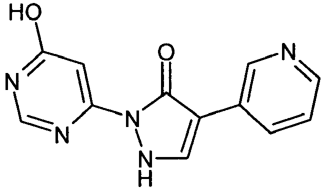

2.8 g (19.6 mmol) der Verbindung aus Beispiel 7A und 4.3 g (19.6 mmol) der Verbindung aus Beispiel 3A werden in 50 ml Eisessig 16 h in der Siedehitze (125°C Badtemperatur) gerührt. Zur Aufarbeitung wird der ausgefallene Niederschlag abfiltriert, der Filterrückstand mit Diethylether gewaschen und das Filtrat am Rotationsverdampfer eingeengt. Der Filtrat-Rückstand wird in 50 ml Ethanol gelöst, mit 18.5 ml (2.7 g, 39.2 mmol) 21%-iger ethanolischer Natriumethylat-Lösung versetzt und die Lösung 16 h bei RT gerührt. Man stellt die Reaktionsmischung danach mit 1 N Salzsäure auf pH 5 ein, rührt 2 h bei RT nach, filtriert den Niederschlag dann ab, wäscht den Filterrückstand mit Wasser und Ethanol nach und trocknet das Produkt im Vakuum.
Ausbeute: 2.0 g (40% d. Th.)
LC-MS (Methode 1): Rₜ = 1.84 min; MS (ESIpos): m/z = 256 [M+H]⁺;
¹H-NMR (400 MHz, DMSO-d₆): δ = 12.80 (br. s, 1 H), 9.18 (s, 1 H), 8.63 (s, 1 H), 8.48 (d, 1 H), 8.43 (d, 1H), 8.34 (s, 1H), 7.76 (dd, 1H), 7.22 (s, 1H).

### Beispiel 25

### 2-{6-[(2-Methoxyethyl)amino]pyrimidin-4-yl}-4-pyridin-3-yl-1,2-dihydro-3H-pyrazol-3-on-Hydrochlorid

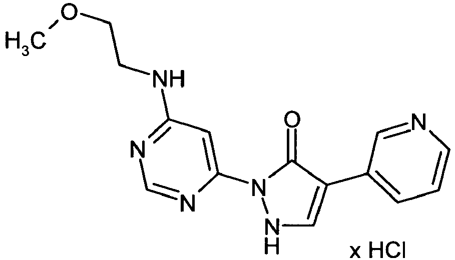

100 mg (0.4 mmol) der Verbindung aus Beispiel 23, 64 µl (55 mg, 0.7 mmol) 2-Methoxyethylamin und 127 µl *N,N-*Diisopropylethylamin (94 mg, 0.7 mmol) werden in 3 ml n-Butanol für 1.5 h unter Rückfluss gerührt. Das Lösungsmittel wird dann am Rotationsverdampfer vollständig entfernt. Der Rückstand wird mit Diethylether/Methanol verrührt, der Niederschlag abfiltriert und der Filterrückstand mit Diethylether gewaschen. Man verrührt den Feststoff in 1.5 ml 1 N Salzsäure, engt wieder ein und trocknet das Produkt im Vakuum.
Ausbeute: 87 mg (68% d. Th.)
LC-MS (Methode 1): Rₜ = 2.11 min; MS (ESIpos): m/z = 313 [M+H]⁺;
¹H-NMR (400 MHz, D₂O): δ = 8.98 (s, 1H), 8.62 (s, 1H), 8.47-8.32 (m, 1H), 8.26 (d, 1H), 8.11 (s, 1H), 7.81 (t, 1H), 6.91 (s, 1H), 3.73-3.43 (m, 4H), 3.31 (s, 3H).

### Beispiel 26

### 2-{6-[2-(Dimethylamino)ethoxy]pyrimidin-4-yl}-4-pyridin-3-yl-1,2-dihydro-3H-pyrazol-3-on-Hydrochlorid

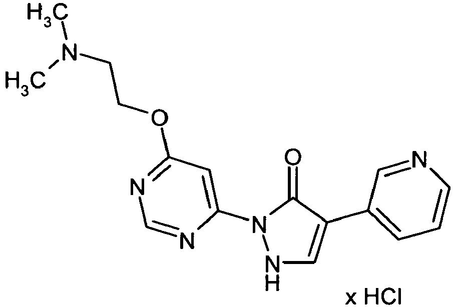

Eine Lösung von 35 µl (31 mg, 0.4 mmol) *N,N-*Dimethylethanolamin und 2 ml wasserfreiem THF wird mit 99 mg (0.4 mmol, 60%-ig in Mineralöl) Natriumhydrid versetzt und 10 min gerührt. Man gibt 100 mg (0.4 mmol) der Verbindung aus Beispiel 23, suspendiert in 3 ml wasserfreiem THF, sowie 6 mg (0.02 mmol) Tetra-n-butylammoniumiodid hinzu und rührt die Mischung 16 h bei RT. Der Ansatz wird dann mit 1 N Salzsäure und Wasser versetzt, am Rotationsverdampfer eingeengt und der Rückstand in Methanol verrührt. Der ausgefallene Feststoff wird abfiltriert und das Filtrat im Vakuum eingeengt. Der Filtrat-Rückstand wird in Diethylether verrührt, der Niederschlag abfiltriert und der Filterrückstand mittels präparativer HPLC weiter gereinigt (RP18-Säule; Laufinittel: Acetonitril/Wasser-Gradient unter Zusatz von 0.1% Ameisensäure). Das hierbei erhaltene Formiat-Salz der Zielverbindung wird durch Zugabe von 2 ml 1 M Salzsäure und erneutes Einengen in das Hydrochlorid überführt.
Ausbeute: 121 mg (96% d. Th.)
LC-MS (Methode 1): Rₜ = 1.83 min; MS (ESIpos): m/z = 327 [M+H]⁺;
¹H-NMR (400 MHz, D₂O): δ = 9.34 (s, 1H), 8.85 (d, 1H), 8.62 (s, 1H), 8.54 (d, 1H), 8.48 (s, 1H), 8.02 (dd, 1H), 7.73 (s, 1H), 4.92-4.40 (m, 2H), 3.69 (t, 2H), 3.01 (s, 6H).

### Beispiel 27

### 4-Pyridin-3-yl-2-[6-(4-pyrrolidin-1-ylpiperidin-1-yl)pyrimidin-4-yl]-1,2-dihydro-3H-pyrazol-3-on-Dihydrochlorid

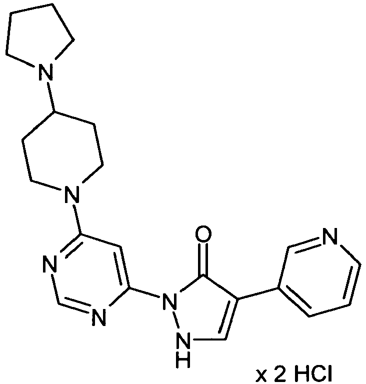

100 mg (0.4 mmol) der Verbindung aus Beispiel 23 und 113 mg (0.7 mmol) 4-Pyrrolidin-1-yl-piperidin werden in 3 ml THF vorgelegt. Die Reaktionsmischung wird in einer *single mode*-Mikrowelle (Emrys Optimizer) für 24 min bei 120°C umgesetzt. Man engt die abgekühlte Reaktionslösung dann am Rotationsverdampfer ein und chromatographiert den Rückstand mittels präparativer HPLC (RP18-Säule; Laufmittel: Acetonitril/Wasser-Gradient unter Zusatz von 0.1% Ameisensäure). Das hierbei erhaltene Formiat-Salz der Zielverbindung wird mit 1 ml einer 4 N Lösung von Chlorwasserstoff in Dioxan versetzt und 30 min bei RT gerührt. Die Suspension wird danach im Vakuum eingeengt und der Rückstand getrocknet.
Ausbeute: 166 mg (98% d. Th.)
LC-MS (Methode 1): Rₜ = 1.92 min; MS (ESIpos): m/z = 392 [M+H]⁺;
¹H-NMR (400 MHz, DMSO-d₆): δ = 11.45 (s, 1H), 9.48-9.28 (m, 1H), 8.88 (d, 1H), 8.67 (d, 1H), 8.49 (d, 1H), 8.47 (dd, 1H), 7.50 (s, 1H), 3.57-3.27 (m, 5H), 3.21-2.93 (m, 3H), 2.87-2.83 (m, 1H), 2.28-2.14 (m, 2H), 2.08-1.68 (m, 6H).

### Beispiel 28

### 2-{6-[4-(2-Methoxyethyl)piperazin-1-yl]pyrimidin-4-yl}-4-pyridin-3-yl-1,2-dihydro-3H-pyrazol-3-on-Dihydrochlorid

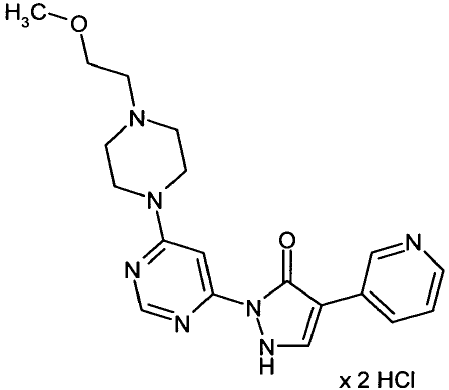

100 mg (0.4 mmol) der Verbindung aus Beispiel 23 und 113 mg (0.7 mmol) *N*-(Methoxyethyl)-piperazin werden in 3 ml THF vorgelegt. Die Reaktionsmischung wird in einer *single mode-*Mikrowelle (Emrys Optimizer) für 20 min bei 120°C umgesetzt. Man engt die abgekühlte Reaktionslösung dann am Rotationsverdampfer ein und chromatographiert den Rückstand mittels präparativer HPLC (RP18-Säule; Laufmittel: Acetonitril/Wasser-Gradient unter Zusatz von 0.1% Ameisensäure). Das hierbei erhaltene Formiat-Salz der Zielverbindung wird mit 1 ml einer 4 N Lösung von Chlorwasserstoff in Dioxan versetzt und 30 min bei RT gerührt. Die Suspension wird danach im Vakuum eingeengt und der Rückstand getrocknet.
Ausbeute: 124 mg (98% d. Th.)
LC-MS (Methode 8): R, = 0.82 min; MS (ESIpos): m/z = 382 [M+H]⁺;
¹H-NMR (400 MHz, DMSO-d₆): δ = 11.41 (br. s, 1H), 9.86 (br. s, 1H), 9.34 (s, 1H), 8.92 (d, 1H), 8.70 (s, 1H), 8.62 (s, 1H), 8.57 (d, 1H), 7.99 (dd, 1H), 7.62 (s, 1H), 3.82-3.71 (m, 4H), 3.68-3.28 (m, 9H), 3.26-3.10 (m, 2H).

### Beispiel 29

### 2-{6-[4-(Dimethylamino)piperidin-1-yl]pyrimidin-4-yl}-4-pyridin-3-yl-1,2-dihydro-3H-pyrazol-3-on-Dihydrochlorid

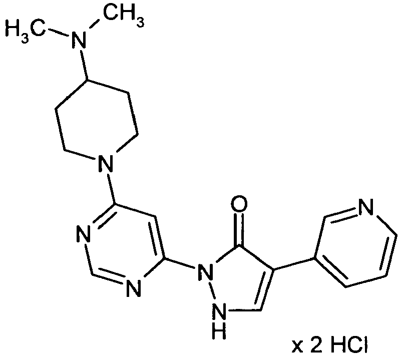

200 mg (0.7 mmol) der Verbindung aus Beispiel 23 und 187 mg (1.5 mmol) 4-(*N-*(Dimethyl-amino)piperidin werden in 3 ml THF vorgelegt. Die Reaktionsmischung wird in einer *single mode-*Mikrowelle (Emrys Optimizer) für 5 min bei 180°C umgesetzt. Man engt die abgekühlte Reaktionslösung dann am Rotationsverdampfer ein und chromatographiert den Rückstand mittels präparativer HPLC (RP18-Säule; Laufmittel: Acetonitril/Wasser-Gradient unter Zusatz von 0.1% Ameisensäure). Das hierbei erhaltene Formiat-Salz der Zielverbindung wird mit 1 ml einer 4 N Lösung von Chlorwasserstoff in Dioxan versetzt und 30 min bei RT gerührt. Die Suspension wird danach im Vakuum eingeengt und der Rückstand getrocknet.
Ausbeute: 257 mg (80% d. Th.)
LC-MS (Methode 9): Rₜ = 0.76 min; MS (ESIpos): m/z = 366 [M+H]⁺;
¹H-NMR (400 MHz, DMSO-d₆): δ = 11.32-11.05 (m, 1H), 9.34 (s, 1H), 9.03 (br. s, 1H), 8.87 (d, 1H), 8.58 (s, 2H), 8.49 (d, 1H), 7.95 (dd, 1H), 7.51 (s, 1H), 4.62-4.58 (m, 1H), 3.59-3.33 (m, 2H), 3.13-3.09 (m, 1H), 2.90-2.88 (m, 1H), 2.71 (s, 6H), 2.14-2.10 (m, 2H), 1.95-1.91 (m, 1H), 1.69-1.67 (m, 1H).

Die in der folgenden Tabelle 3 aufgeführten Verbindungen werden aus den entsprechenden Edukten nach folgenden Reaktionsbedingungen bzw. Aufarbeitungsmethoden erhalten:
1 Äquivalent der Beispielverbindung 23 wird in THF mit 2 Äquivalenten des entsprechenden Amins in einer *single mode*-Mikrowelle (Emrys Optimizer) für 10-30 min bei 120°C umgesetzt. Wird ein Ammoniumsalz der Amin-Komponente als Edukt verwendet, so setzt man 1 Äquivalent *N,N*-Diisopropylethylamin hinzu. Die Reinigung des jeweiligen Rohprodukts aus der aufkonzentrierten Reaktionsmischung erfolgt durch Ausrühren in Isopropanol. Man filtriert den erhaltenen Niederschlag ab und wäscht den Filterrückstand mit Isopropanol und/oder Diisopropylether nach und erhält so das Zielprodukt als freie Base (Methode A). In einer alternativen Aufarbeitung wird das eingeengte Filtrat oder die aufkonzentrierte Reaktionslösung mittels präparativer HPLC gereinigt (RP18-Säule; Laufmittel: Acetonitril/Wasser-Gradient unter Zusatz von 0.1% Ameisensäure). Das hierbei erhaltene Formiat-Salz wird anschließend mit einer 4 N Lösung von Chlorwasserstoff in Dioxan versetzt und 30 min bei RT gerührt. Die Suspension wird dann im Vakuum eingeengt und der Rückstand getrocknet (Methode B).

Alternativ werden 1 Äquivalent der Beispielverbindung 23 und 2 Äquivalente der Amin-Komponente in THF gelöst und für 5 min bei 180°C in einer *single mode*-Mikrowelle (Emrys Optimizer) umgesetzt. Man engt die Reaktionsmischung danach ein und reinigt das Rohprodukt mittels präparativer HPLC (RP18-Säule; Laufmittel: Acetonitril/Wasser-Gradient unter Zusatz von 0.1% Ameisensäure). Das hierbei erhaltene Formiat-Salz der Zielverbindung wird mit einer 4 N Lösung von Chlorwasserstoff in Dioxan versetzt und 30 min bei RT gerührt. Die Suspension wird dann im Vakuum eingeengt und der Rückstand getrocknet (Methode C).

**Tabelle 3**

| **Beispiel Nr.** | **Struktur** | **Ausbeute (% d. Th.) [Methode]** | **MS (ESI): [M+H]⁺; LC-MS: Rₜ (Methode)** | **¹H-NMR (400 MHz, DMSO-d₆)** |
|---|---|---|---|---|
| **30** | 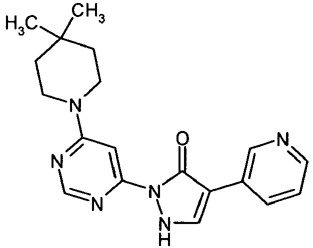 | 42% [A] | m/z = 351; 1.58 min (8) | δ=9.03 (s, 1H), 8.48 (s, 1H), 8.39 (s, 1H), 8.33 (d, 1H), 8.19 (d, 1H), 7.41 (br. s, 1H), 7.34 (dd, 1H), 3.70-3.68 (m, 4H), 1.41-1.37 (m, 4H), 0.99 (s, 6H). |
| **31** | 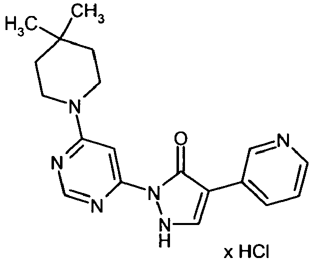 | 42% [B] | m/z = 351; 1.57 min (8) | δ = 9.28 (s, 1H), 8.84 (d, 1H), 8.53 (s, 1H), 8.49-8.43 (m, 2H), 7.93 (dd, 1H), 7.41 (s, 1H), 3.79-3.75 (m, 4H), 1.44-1.40 (m, 4H), 1.01 (s, 6H). |
| **32** | 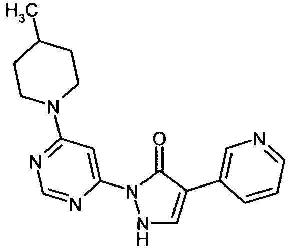 | 17% [A] | m/z = 337; 2.69 min (1) | δ = 9.01 (s, 1H), 8.37 (s, 1H), 8.18 (d, 1H), 8.12 (s, 1H), 8.01 (s, 1H), 7.71 (s, 1H), 7.20 (dd, 1H), 2.94-2.90 (m, 2H), 1.71-1.53 (m, 4H), 1.30-1.27 (m, 1H), 1.18-1.01 (m, 2H), 0.99-0.83 (m, 3H). |
| **33** | 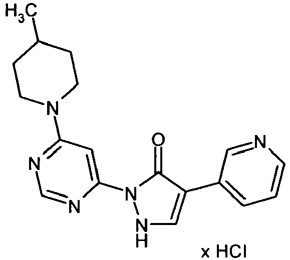 | 26% [B] | m/z = 337; 1.44 min (8) | δ = 9.28 (s, 1H), 8.84 (d, 1H), 8.53 (s, 1H), 8.49-8.42 (m, 2H), 7.93 (dd, 1H), 7.42 (s, 1H), 3.13-3.11 (m, 2H), 1.85-1.69 (m, 4H), 1.28-1.05 (m, 3H), 0.95 (d, 3H). |
| **34** | 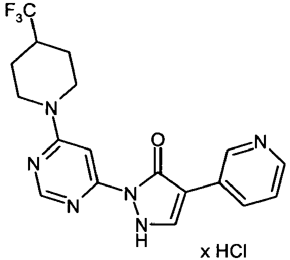 | 54% [B] | m/z = 391; 2.75 min (8) | δ = 9.30 (s, 1H), 8.86 (d, 1H), 8.54 (s, 2H), 8.49 (d, 1H), 7.94 (dd, 1H), 7.48 (s, 1H), 4.57-4.55 (m, 2H), 3.18-3.14 (m, 2H), 2.79-2.77 (m, 1H), 2.02-1.99 (m, 2H), 1.49-1.47 (m, 2H). |
| **35** | 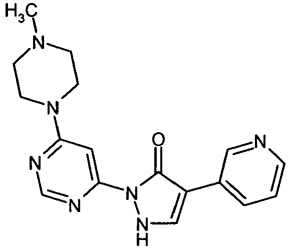 | 17% [A] | m/z = 338; 0.76 min (8) | δ = 9.05 (s, 1H), 8.47 (s, 1H), 8.32 (s, 1H), 8.28 (d, 1H), 8.21 (d, 1H), 7.61 (s, 1H), 7.32 (dd, 1H), 3.74-3.71 (m, 4H), 2.60-2.57 (m, 4H), 2.34 (s, 3H). |
| **36** | 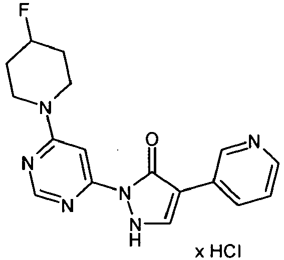 | 26% [B] | m/z = 341; 1.25 min (7) | δ = 9.28 (s, 1H), 8.86 (d, 1H), 8.55-8.53 (m, 2H), 8.48 (d, 1H), 7.97-7.90 (m, 1H), 7.50 (s, 1H), 5.09-4.89 (m, 1H), 3.91-3.72 (m, 4H), 2.07-1.90 (m, 2H), 1.89-1.76 (m, 2H). |
| **37** | 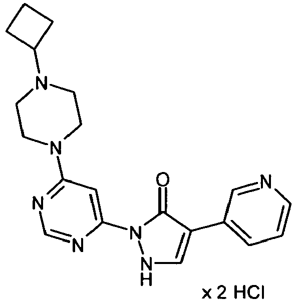 | 55% [B] | m/z = 378; 0.85 min (7) | δ = 9.30 (s, 1H), 8.86 (d, 1H), 8.65 (s, 1H), 8.60 (s, 1H), 8.54 (d, 1H), 7.98-7.89 (m, 1H), 7.60 (s, 1H), 4.64-4.46 (m, 1H), 3.13-2.82 (m, 4H), 2.42-2.27 (m, 3H), 2.21-2.08 (m, 3H), 1.82-1.60 (m, 3H), 1.30-1.22 (m, 1H). |
| **38** | 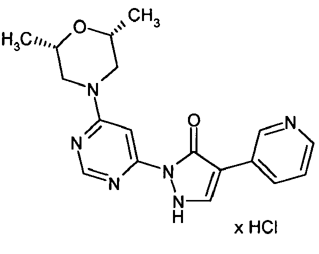 | 65% [B] | m/z = 353; 1.24 min (8) | (500 MHz, D₂O) δ = 9.05 (s, 1H), 8.67 (d, 1H), 8.47 (s, 1H), 8.34 (d, 1H), 8.17 (s, 1H), 7.94-7.85 (m, 1H), 7.07 (s, 1H), 4.02-3.94 (m, 1H), 3.84-3.73 (m, 1H), 3.36-3.29 (m, 1H), 2.98-2.75 (m, 3H), 1.29-1.21 (m, 6H). |
| **39** | 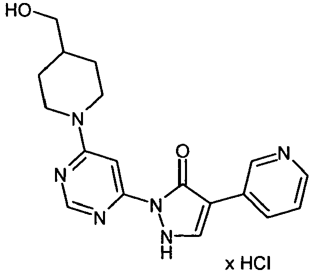 | 49% [C] | m/z = 353; 1.06 min (7) | δ = 9.26 (s, 1H), 8.84 (d, 1H), 8.52 (s, 1H), 8.48-8.41 (m, 2H), 7.95-7.89 (m, 1H), 7.42 (s, 1H), 4.64-4.39 (m, 1H), 3.32-3.23 (m, 3H), 3.16-3.07 (m, 1H), 1.88-1.72 (m, 3H), 1.20-1.10 (m, 1H). |
| **40** | 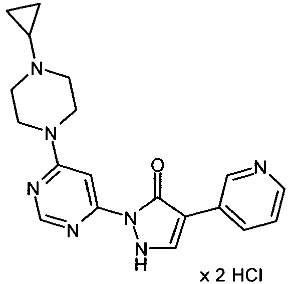 | 39% [B] | m/z = 364; 0.90 min (7) | δ = 9.33 (s, 1H), 8.91 (d, 1H), 8.68 (s, 1H), 8.62 (s, 1H), 8.57 (d, 1H), 8.02-7.96 (m, 1H), 7.64 (s, 1H), 3.66-3.49 (m, 4H), 3.40-3.22 (m, 2H), 2.90-2.79 (m, 1 H), 1.31-1.24 (m, 2H), 1.22-1.15 (m, 2H), 0.87-0.80 (m, 2H). |
| **41** | 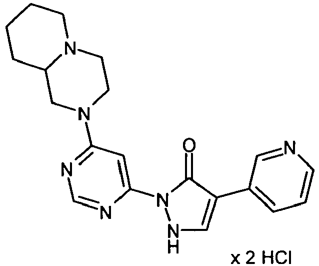 | 22% [C] | m/z = 378; 0.77 min (10) | δ=9.32 (s, 1H), 8.91 (d, 1H), 8.68 (s, 1H), 8.62 (s, 1H), 8.57 (d, 1H), 8.02-7.95 (m, 1H), 7.61 (s, 1H), 4.87-4.44 (m, 1H), 3.70-2.83 (m, 8H), 2.02-1.61 (m, 4H), 1.54-1.39 (m, 2H). |

### Beispiel 42

### 4-(5-Brompyridin-3-yl)-2-(6-piperidin-1-ylpyrimidin-4-yl)-1,2-dihydro-3H-pyrazol-3-on-Hydrochlorid

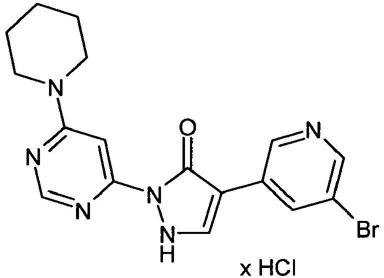

500 mg (1.7 mmol) der Verbindung aus Beispiel 11A, 323 mg (1.7 mmol) der Verbindung aus Beispiel 8A und 58 mg (0.3 mmol) p-Toluolsulfonsäure werden in 2 ml Ethanol 16 h bei 100°C gerührt. Nach Abkühlen auf RT werden 0.5 ml einer 4 N Lösung von Chlorwasserstoff in Dioxan hinzugefügt und 30 min bei RT gerührt. Der Niederschlag wird abfiltriert, zunächst mit Ethanol, dann mit Diethylether gewaschen und im Vakuum getrocknet.
Ausbeute: 260 mg (36% d. Th.)
LC-MS (Methode 7): Rₜ = 2.22 min; MS (ESIpos): m/z = 401 [M+H]⁺;
¹H-NMR (400 MHz, DMSO-d₆): δ = 9.12 (s, 1H), 8.75 (s, 1H), 8.53-8.46 (m, 3H), 7.42 (s, 1H), 3.83-3.63 (m, 4H), 1.73-1.54 (m, 6H).

### Beispiel 43

### 4-(5-Brompyridin-3-yl)-2-(6-morpholin-4-ylpyrimidin-4-yl)-1,2-dihydro-3H-pyrazol-3-on-Hydrochlorid

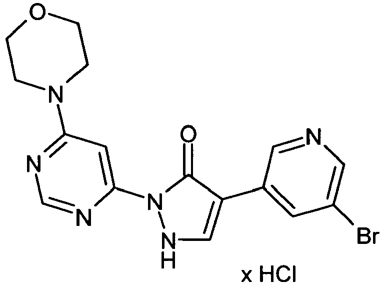

500 mg (1.7 mmol) der Verbindung aus Beispiel 11A, 326 mg (1.7 mmol) der Verbindung aus Beispiel 9A und 58 mg (0.3 mmol) p-Toluolsulfonsäure werden in 4 ml Ethanol 16 h bei 100°C gerührt. Nach Abkühlen auf RT werden 0.5 ml einer 4 N Lösung von Chlorwasserstoff in Dioxan hinzugefügt und 30 min bei RT gerührt. Der Niederschlag wird abfiltriert, zunächst mit Ethanol, dann mit Diethylether gewaschen und im Vakuum getrocknet.
Ausbeute: 235 mg (32% d. Th.)
LC-MS (Methode 7): Rₜ = 1.85 min; MS (ESIpos): m/z = 403 [M+H]⁺;
¹H-NMR (400 MHz, DMSO-d₆): δ = 9.12 (s, 1H), 8.73 (s, 1H), 8.56-8.50 (m, 3H), 7.49 (s, 1H), 3.75-3.67 (m, 8H).

### Beispiel 44

### 5-[3-Oxo-2-(6-piperidin-1-ylpyrimidin-4-yl)-2,3-dihydro-1H-pyrazol-4-yl]pyridin-3-carbonitril-Hydrochlorid

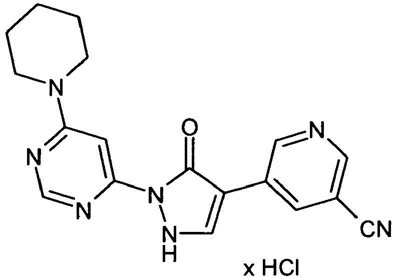

100 mg (0.2 mmol) der Verbindung aus Beispiel 42, 20 mg (0.2 mmol) Zinkcyanid und 8 mg (0.007 mmol) Tetrakis(triphenylphosphin)palladium in 2 ml DMF werden für insgesamt 75 min bei 220°C in einer *single mode*-Mikrowelle (Emrys Optimizer) umgesetzt. Nach Abkühlen auf RT wird die Reaktionsmischung im Vakuum eingeengt und der Rückstand in Ameisensäure aufgenommen und mittels präparativer HPLC gereinigt (RP18-Säule; Laufmittel: Acetonitril/Wasser-Gradient unter Zusatz von 0.1% Ameisensäure im Wasser). Das hierbei anfallende Formiat-Salz wird durch Zugabe von 0.5 ml einer 4 N Lösung von Chlorwasserstoff in Dioxan in das Hydrochlorid überführt. Das Produkt wird zunächst mit Essigsäureethylester, dann mit Diethylether gewaschen und im Vakuum getrocknet.
Ausbeute: 22 mg (25% d. Th.)
LC-MS (Methode 7): Rₜ = 1.97 min; MS (ESIpos): m/z = 348 [M+H]⁺;
¹H-NMR (400 MHz, DMSO-d₆): δ = 9.34 (s, 1H), 8.73-8.59 (m, 2H), 8.54-8.37 (m, 2H), 7.41 (s, 1H), 3.77-3.58 (m, 4H), 1.75-1.49 (m, 6H).

### Beispiel 45

### 5-[2-(6-Morpholin-4-ylpyrimidin-4-yl)-3-oxo-2,3-dihydro-1H-pyrazol-4-yl]pyridine-3-carbonitril-Hydrochlorid

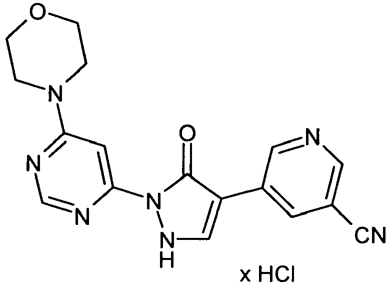

100 mg (0.2 mmol) der Verbindung aus Beispiel 43, 20 mg (0.2 mmol) Zinkcyanid und 8 mg (0.007 mmol) Tetrakis(triphenylphosphin)palladium in 2 ml DMF werden für insgesamt 105 min bei 220°C in einer *single mode*-Mikrowelle (Emrys Optimizer) umgesetzt. Nach Abkühlen auf RT wird die Reaktionsmischung im Vakuum eingeengt und der Rückstand in Ameisensäure aufgenommen und mittels präparativer HPLC gereinigt (RP18-Säule; Laufmittel: Acetonitril/Wasser-Gradient unter Zusatz von 0.1% Ameisensäure im Wasser). Das hierbei anfallende Formiat-Salz wird durch Zugabe von 0.5 ml einer 4 N Lösung von Chlorwasserstoff in Dioxan in das Hydrochlorid überführt.
Ausbeute: 11 mg (13% d. Th.)
LC-MS (Methode 7): Rₜ = 1.64 min; MS (ESIpos): m/z = 350 [M+H]⁺;
¹H-NMR (400 MHz, DMSO-d₆): δ = 9.36 (s, 1H), 8.75-8.64 (m, 2H), 8.59-8.49 (m, 2H), 7.49 (s, 1H), 3.76-3.65 (m, 8H).

### Beispiel 46

### 2-Pyrazin-2-yl-4-pyridin-3-yl-1,2-dihydro-3H-pyrazol-3-on-Hydrochlorid

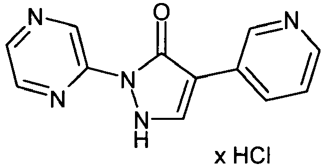

441 mg (2.0 mmol) der Verbindung aus Beispiel 12A und 220 mg (2.0 mmol) der Verbindung aus Beispiel 3A werden in 10 ml Ethanol vorgelegt. Man gibt 93 mg (0.4 mmol) Campher-10-sulfonsäure hinzu und rührt 5 h unter Rückfluss. Man lässt dann auf RT abkühlen, filtriert den gebildeten Feststoff ab und wäscht einmal mit wenig Ethanol. Anschließend versetzt man mit 10 ml einer 4 N Lösung von Chlorwasserstoff in Dioxan und rührt 30 min bei RT. Man engt danach am Rotationsverdampfer ein und trocknet den Rückstand im Hochvakuum.
Ausbeute: 260 mg (47% d. Th.)
LC-MS (Methode 1): Rₜ = 1.93 min; MS (ESIpos): m/z = 240 [M+H]⁺;
¹H-NMR (400 MHz, DMSO-d₆): δ = 9.70 (s, 1 H), 9.43 (s, 1 H), 9.08 (d, 1 H), 9.01 (s, 1 H), 8.70 (d, 1H), 8.61 (s, 2H), 8.08 (dd, 1H).

### Beispiel 47

### 4-Pyridin-3-yl-2-chinoxalin-2-yl-1,2-dihydro-3H-pyrazol-3-on-Hydrochlorid

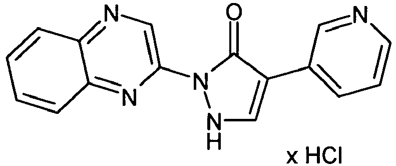

1.5 g (6.8 mmol) der Verbindung aus Beispiel 3A und 1.0 g (6.8 mmol) der Verbindung aus Beispiel 13A werden in 35 ml Ethanol vorgelegt. Man gibt 316 mg (1.4 mmol) Campher-10-sulfonsäure hinzu und rührt 6 h unter Rückfluss. Man kühlt dann auf 0°C, filtriert den entstandenen Feststoff ab und wäscht mit Ethanol nach. Anschließend versetzt man mit 10 ml einer 4 N Lösung von Chlorwasserstoff in Dioxan und rührt 30 min bei RT. Man engt danach am Rotationsverdampfer ein und trocknet den Rückstand im Hochvakuum.
Ausbeute: 470 mg (21 % d. Th.)
LC-MS (Methode 11): Rₜ = 1.22 min; MS (ESIpos): m/z = 290 [M+H]⁺;
¹H-NMR (400 MHz, DMSO-d₆): δ = 10.03 (s, 1H), 9.45 (s, 1H), 9.10-9.05 (m, 2H), 8.70 (d, 1H), 8.14 (d, 1H), 8.11-8.02 (m, 2H), 7.92 (dd, 1H), 7.85 (dd, 1H).

### Beispiel 48

### 4-Pyridin-3-yl-2-chinolin-2-yl-1,2-dihydro-3H-pyrazol-3-on-Hydrochlorid

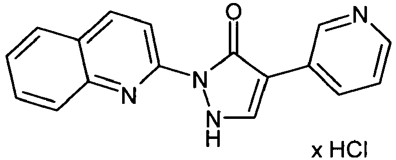

750 mg (3.4 mmol) der Verbindung aus Beispiel 14A und 542 mg (3.4 mmol) der Verbindung aus Beispiel 3A werden in 17.5 ml Ethanol vorgelegt. Man gibt 130 mg (0.7 mmol) p-Toluolsulfonsäure hinzu und rührt 16 h unter Rückfluss. Man lässt danach abkühlen und engt am Rotationsverdampfer ein. Man verrührt den Rückstand 30 min in einem Gemisch aus 6 ml DMSO und 10 ml Wasser, filtriert den Feststoff ab und trocknet ihn im Hochvakuum. Anschließend versetzt man mit 10 ml einer 4 N Lösung von Chlorwasserstoff in Dioxan und rührt 30 min bei RT. Man engt danach am Rotationsverdampfer ein und trocknet den Rückstand im Hochvakuum.
Ausbeute: 750 mg (65% d. Th.)
LC-MS (Methode 12): Rₜ = 1.14 min; MS (ESIpos): m/z = 289 [M+H]⁺;
¹H-NMR (400 MHz, DMSO-d₆): δ = 9.40 (s, 1H), 9.03 (d, 1H), 8.85 (s, 1H), 8.71-8.57 (m, 3H), 8.12-8.02 (m, 3H), 7.88 (dd, 1H), 7.64 (dd, 1H).

### Beispiel 49

### 2-(6-Azetidin-1-ylpyrimidin-4-yl)-4-pyridin-3-yl-1,2-dihydro-3H-pyrazol-3-on

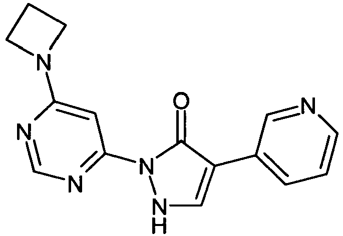

150 mg (0.5 mmol) der Verbindung aus Beispiel 23 und 63 mg (1.1 mmol) Azetidin werden in 4 ml Ethanol suspendiert und 40 min bei 120°C in einer *single mode*-Mikrowelle (CEM Explorer) umgesetzt. Der Feststoff wird abfiltriert, zweimal mit je 0.5 ml Ethanol gewaschen und verworfen. Die Mutterlauge wird mit den Waschlösungen vereinigt und das Lösungsmittel wird am Rotationsverdampfer entfernt. Der Rückstand wird mittels präparativer HPLC gereinigt (RP18-Säule; Laufmittel: Acetonitril/Wasser-Gradient unter Zusatz von 0.1% TFA). Man vereinigt die produkthaltigen Fraktionen und engt am Rotationsverdampfer ein. Der Rückstand wird 20 min in Ethanol unter Rückfluss verrührt und anschließend warm abfiltriert. Der erhaltene Feststoff wird im Hochvakuum getrocknet.
Ausbeute: 45 mg (28% d. Th.)
LC-MS (Methode 9): Rₜ = 0.38 min; MS (ESIpos): m/z = 295 [M+H]⁺;
¹H-NMR (400 MHz, DMSO-d₆): δ = 9.19 (s, 1H), 8.64 (d, 1H), 8.45 (s, 1H), 8.41 (d, 2H), 7.75 (dd, 1H), 6.88 (s, 1H), 4.20 (dd, 4H), 2.46-2.38 (m, 2H).

### Beispiel 50

### 2-[6-(3-Hydroxyazetidin-1-yl)pyrimidin-4-yl]-4-pyridin-3-yl-1,2-dihydro-3H-pyrazol-3-on-Hydrochlorid

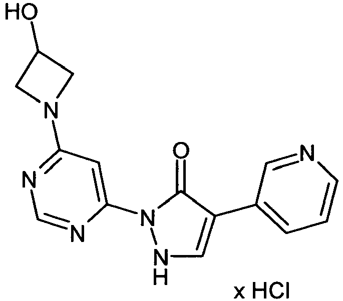

100 mg (0.4 mmol) der Verbindung aus Beispiel 23, 94 mg (0.7 mmol) *N,N-*Diisopropylethylamin und 80 mg (0.7 mmol) Azetidin-3-ol-Hydrochlorid werden in 3 ml THF suspendiert und 20 min bei 120°C in einer *single mode*-Mikrowelle (CEM Explorer) umgesetzt. Man gibt dann 2 ml Ethanol hinzu und setzt erneut für 20 min in einer *single mode*-Mikrowelle (CEM Explorer) um. Anschließend wird zunächst weitere 60 min bei 120°C und dann 60 min bei 175°C in einer *single mode-*Mikrowelle (CEM Explorer) umgesetzt. Das Gemisch wird danach direkt mittels präparativer HPLC aufgetrennt (RP18-Säule; Laufmittel: Acetonitril/Wasser-Gradient unter Zusatz von 0.1% TFA). Man vereinigt die produkthaltigen Fraktionen, engt am Rotationsverdampfer ein und trocknet den Rückstand im Hochvakuum. Der Rückstand wird dann 30 min in 5 ml einer 4 N Lösung von Chlorwasserstoff in Dioxan verrührt. Man filtriert den Feststoff ab, wäscht mit *tert.*-Butylmethylether und trocknet im Hochvakuum.
Ausbeute: 26 mg (20% d. Th.)
LC-MS (Methode 7): Rₜ = 0.92 min; MS (ESIpos): m/z = 311 [M+H]⁺;
¹H-NMR (400 MHz, DMSO-d₆): δ = 9.25 (s, 1H), 8.84 (d, 1H), 8.50-8.42 (m, 3H), 7.94 (dd, 1H), 6.93 (s, 1H), 4.70-4.62 (m, 1H), 4.43 (dd, 2H), 3.95 (dd, 2H).

### Beispiel 51

### 2-[6-(3,3-Difluorazetidin-1-yl)pyrimidin-4-yl]-4-pyridin-3-yl-1,2-dihydro-3H-pyrazol-3-on-Hydrochlorid

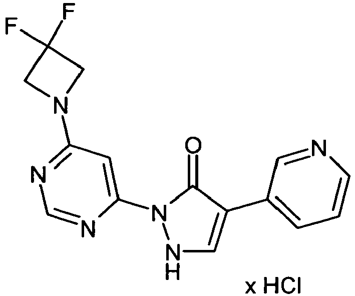

100 mg (0.4 mmol) der Verbindung aus Beispiel 23, 94 mg (0.7 mmol) *N,N-*Diisopropylethylamin und 95 mg (0.731 mmol) 3,3-Difluorazetidin-Hydrochlorid werden in 3 ml Ethanol suspendiert und 40 min bei 120°C in einer *single mode*-Mikrowelle (CEM Explorer) umgesetzt. Das Lösungsmittel wird danach entfernt und der Rückstand in 6 ml DMSO aufgenommen. Ungelöste Bestandteile werden durch Filtration entfernt und die erhaltene Lösung mittels präparativer HPLC aufgereinigt (RP18-Säule; Laufmittel: Acetonitril/Wasser-Gradient unter Zusatz von 0.1% TFA). Man vereinigt die produkthaltigen Fraktionen, engt am Rotationsverdampfer ein und trocknet den Rückstand im Hochvakuum. Der Rückstand wird dann 30 min in 5 ml einer 4 N Lösung von Chlorwasserstoff in Dioxan verrührt. Man filtriert den Feststoff ab, wäscht mit *tert*.-Butylmethylether und trocknet im Hochvakuum.
Ausbeute: 64 mg (44% d. Th.)
LC-MS (Methode 7): Rₜ = 1.13 min; MS (ESIpos): m/z = 331 [M+H]⁺;
¹H-NMR (400 MHz, DMSO-d₆): δ = 9.32 (s, 1H), 8.91 (d, 1H), 8.68 (s, 1H), 8.60-8.53 (m, 2H), 7.97 (dd, 1H), 7.26 (s, 1H), 4.66 (dd, 4H).

### Beispiel 52

### tert.-Butyl-{1-[6-(5-oxo-4-pyridin-3-yl-2,5-dihydro-1H-pyrazol-1-yl)pyrimidin-4-yl]azetidin-3-yl}-carbamat

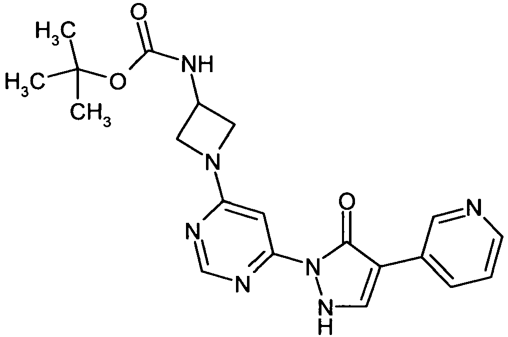

200 mg (0.7 mmol) der Verbindung aus Beispiel 23 und 252 mg (1.5 mmol) *tert*.-Butyl-azetidin-3-ylcarbamat werden in 6 ml Ethanol suspendiert und 40 min bei 120°C in einer *single mode*-Mikrowelle (CEM Explorer) umgesetzt. Der entstandene Feststoff wird abfiltriert, zweimal mit je 0.5 ml Ethanol gewaschen und im Hochvakuum getrocknet.
Ausbeute: 227 mg (76% d. Th.)
LC-MS (Methode 9): Rₜ = 0.72 min; MS (ESIpos): m/z = 410 [M+H]⁺;
¹H-NMR (400 MHz, DMSO-d₆): δ = 9.05 (s, 1H), 9.45 (s, 1H), 9.40 (s, 1H), 9.32 (d, 1H), 8.20 (d, 1H), 7.67 (d, 1H), 7.36 (dd, 1H), 4.55-4.45 (m, 1H), 4.35 (t, 2H), 3.98-3.91 (m, 2H), 1.40 (s, 9H).

### Beispiel 53

### 2-[6-(3-Aminoazetidin-1-yl)pyrimidin-4-yl]-4-pyridin-3-yl-1,2-dihydro-3H-pyrazol-3-on-Bistrifluoracetat

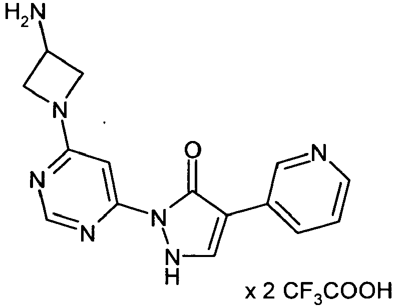

200 mg (0.5 mmol) der Verbindung aus Beispiel 52 werden in 5 ml Dichlormethan gelöst, mit 111 mg (1.0 mmol) TFA versetzt und 18 h bei RT gerührt. Man gibt weitere 1.10 g (9.8 mmol) TFA hinzu und rührt erneut 5 h bei RT. Man engt dann am Rotationsverdampfer ein und verrührt den Rückstand zweimal nacheinander in Dichlormethan, indem man jeweils 5 ml Dichlormethan hinzugibt und anschließend wieder am Rotationsverdampfer einengt. Man verrührt danach in gleicher Weise zweimal in *tert*.-Butylmethylether und einmal in Methanol und trocknet den Rückstand abschließend im Hochvakuum.
Ausbeute: 249 mg (95% d. Th.)
LC-MS (Methode 7): Rₜ = 0.72 min; MS (ESIpos): m/z = 310 [M+H]⁺;
¹H-NMR (400 MHz, DMSO-d₆): δ = 9.24 (s, 1H), 8.71 (d, 1H), 8.60-8.51 (m, 3H), 8.47 (d, 1H), 7.77 (dd, 1H), 7.10 (s, 1H), 4.46 (dd, 2H), 4.25-4.15 (m, 3H).

### Beispiel 54

### 2-[6-(3-Aminoazetidin-1-yl)pyrimidin-4-yl]-4-pyridin-3-yl-1,2-dihydro-3H-pyrazol-3-on-Dihydrochlorid

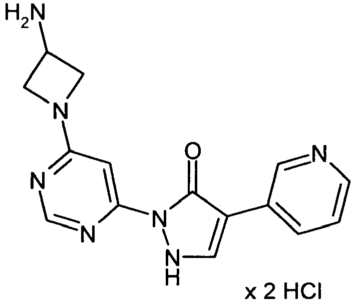

278 mg (0.5 mmol) der Verbindung aus Beispiel 53 werden 30 min in 10 ml einer 4 N Lösung von Chlorwasserstoff in Dioxan verrührt. Man filtriert den Feststoff danach ab, wäscht zweimal mit je 0.5 ml *tert*.-Butylmethylether und trocknet im Hochvakuum.
Ausbeute: 188 mg (95% d. Th.)
LC-MS (Methode 7): Rₜ = 0.74 min; MS (ESIpos): m/z = 310 [M+H]⁺;
¹H-NMR (400 MHz, DMSO-d₆): δ = 9.29 (s, 1H), 8.89-8.75 (m, 4H), 8.59 (s, 1H), 8.55 (s, 1H), 8.51 (d, 1H), 7.95 (dd, 1H), 7.10 (s, 1H), 4.50-4.42 (m, 2H), 4.28-4.20 (m, 3H).

### Beispiel 55

### 2-(6-{[6-(Diethylamino)hexyl]amino}pyrimidin-4-yl)-4-pyridin-3-yl-1,2-dihydro-3H-pyrazol-3-on

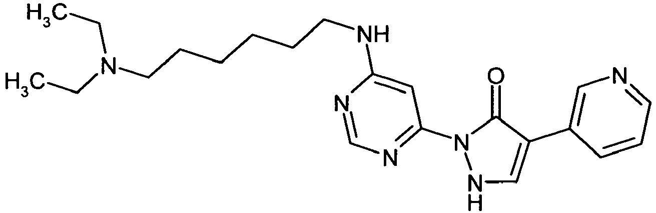

2.7 g (10.0 mmol) der Beispielverbindung 23 werden in 60 ml n-Butanol gelöst und als Stammlösung bereitgestellt.

17 mg (0.1 mmol) *N,N-*Diethylhexan-1,6-diamin werden vorgelegt und nacheinander mit 600 µl (0.1 mmol) der obigen Stammlösung sowie 35 µl (26 mg, 0.2 mmol) *N,N-*Diisopropylethylamin (Hünig-Base) versetzt. Man rührt die Reaktionsmischung 16 h bei 120°C. Zur Aufarbeitung lässt man das n-Butanol abdampfen. Der Rückstand wird in DMSO aufgenommen und filtriert. Das Filtrat wird mittels präparativer LC-MS (Methode 13) aufgereinigt. Die Produktfraktionen werden im Vakuum aufkonzentriert und der Rückstand getrocknet.
Ausbeute: 3 mg (7% d. Th.)
LC-MS (Methode 13): Rₜ = 1.24 min; MS (ESIpos): m/z = 410 [M+H]⁺.

Analog zur Arbeitsvorschrift von Beispiel 55 werden die in Tabelle 4 aufgeführten Verbindungen aus 0.1 mmol der Beispielverbindung 23 und 0.1 mmol des entsprechenden Amins hergestellt:

**Tabelle 4**

| **Beispiel Nr.** | **Struktur** | **Ausbeute (% d. Th.)** | **MS (ESI): [M+H]⁺; LC-MS: Rₜ (Methode 13)** |
|---|---|---|---|
| **56** | 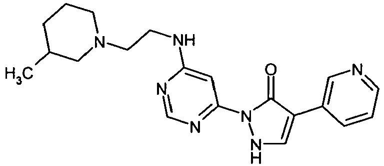 | 15% | m/z = 380; 1.19 min |
| **57** | 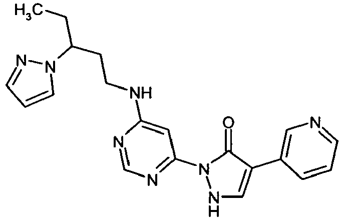 | 28% | m/z = 391; 1.52 min |
| **58** | 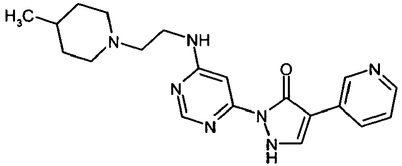 | 6% | m/z = 380; 0.30 min |
| **59** | 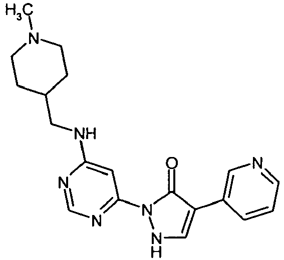 | 7% | m/z = 366; 0.30 min |
| **60** | 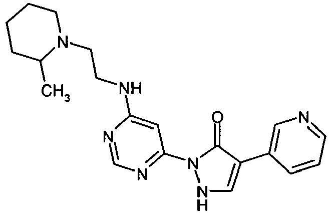 | 20% | m/z = 380; 1.18 min |
| **61** | 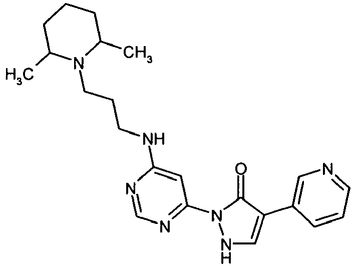 | 26% | m/z = 408; 1.20 min |
| **62** | 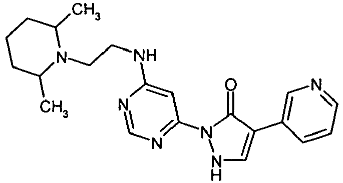 | 12% | m/z = 394; 1.21 min |
| **63** | 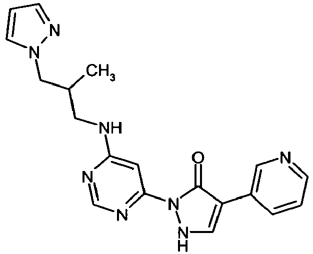 | 34% | m/z = 377; 1.41 min |
| **64** | 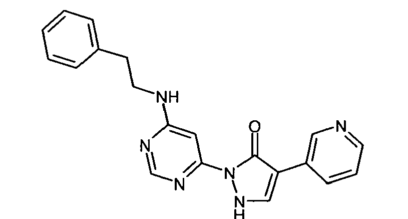 | 15% | m/z = 359; 1.57 min |
| **65** | 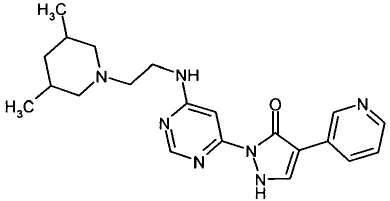 | 22% | m/z = 394; 1.21 min |
| **66** | 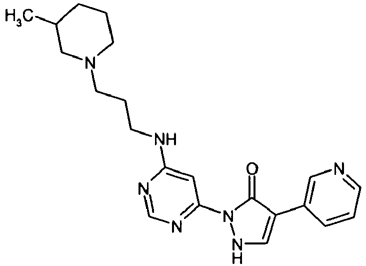 | 62% | m/z = 394; 1.20 min |
| **67** | 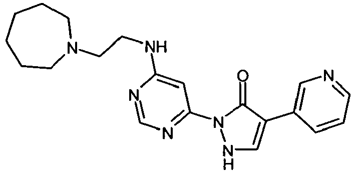 | 18% | m/z = 379; 1.18 min |
| **68** | 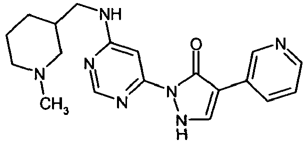 | 24% | m/z = 366; 1.16 min |
| **69** | 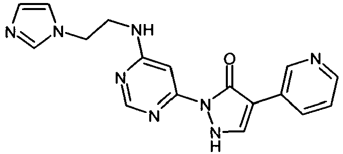 | 5% | m/z = 349; 0.30 min |
| **70** | 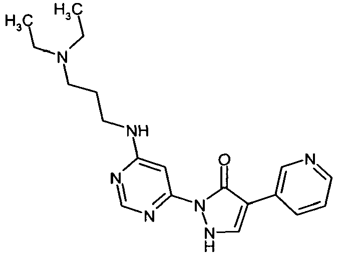 | 4% | m/z = 368; 0.30 min |
| **71** | 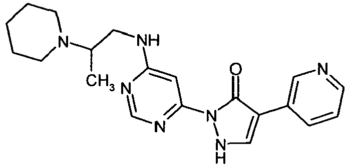 | 25% | m/z = 380; 1.18 min |
| **72** | 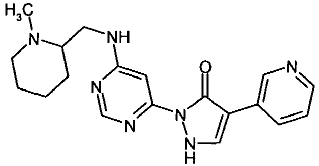 | 14% | m/z = 366; 0.30 min |
| **73** | 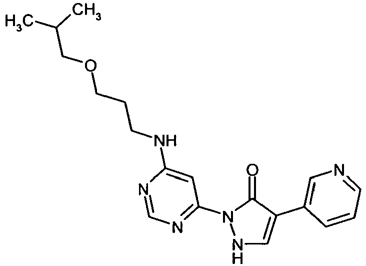 | 2% | m/z = 369; 1.57 min |
| **74** | 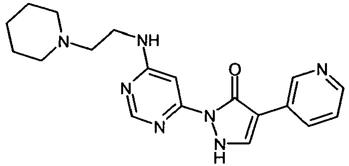 | 27% | m/z = 366; 1.13 min |
| **75** | 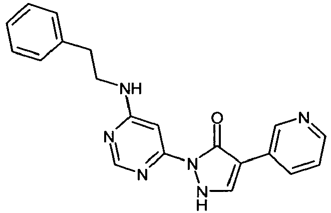 | 5% | m/z = 359; 1.56 min |
| **76** | 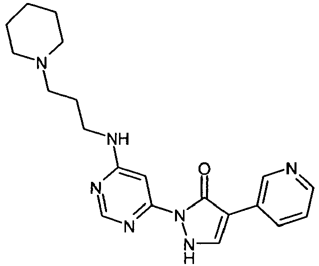 | 25% | m/z = 380; 1.14 min |
| **77** | 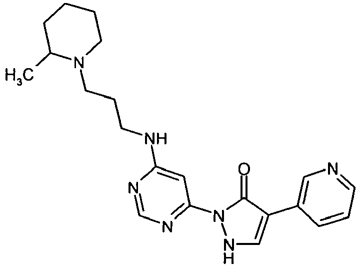 | 18% | m/z = 394; 1.17 min |
| **78** | 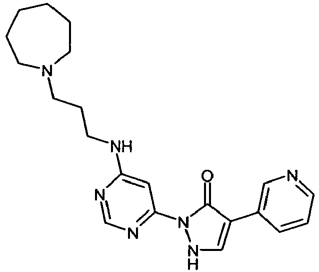 | 30% | m/z = 394; 1.18 min |
| **79** | 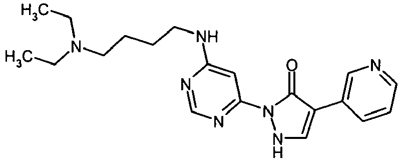 | 6% | m/z = 382; 0.30 min |
| **80** | 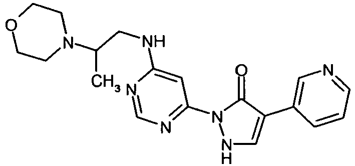 | 11% | m/z = 382; 0.30 min |
| **81** | 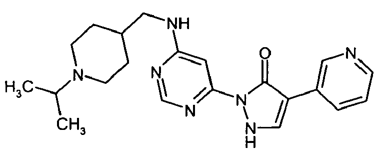 | 24% | m/z = 393; 1.18 min |
| **82** | 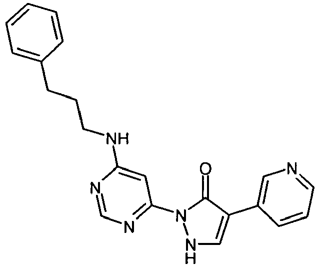 | 12% | m/z = 373; 1.64 min |
| **83** | 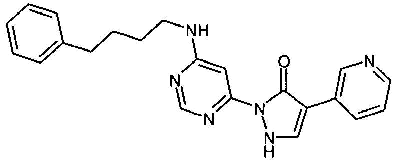 | 11% | m/z = 387; 1.71 min |
| **84** | 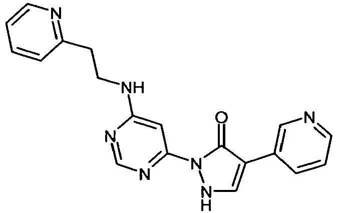 | 20% | m/z = 360; 1.18 min |
| **85** | 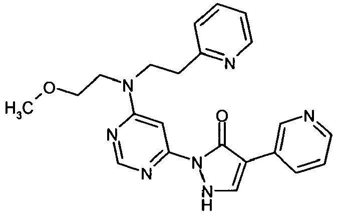 | 9% | m/z = 418; 1.28 min |
| **86** | 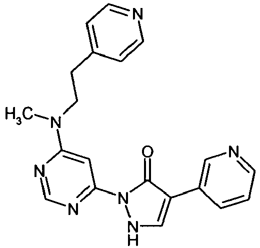 | 16% | m/z = 374; 1.19 min |
| **87** | 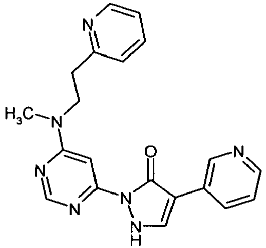 | 12% | m/z = 374; 1.21 min |

### Beispiel 88

### 2-[6-(3-Phenylpropoxy)pyrimidin-4-yl]-4-pyridin-3-yl-1,2-dihydro-3H-pyrazol-3-on

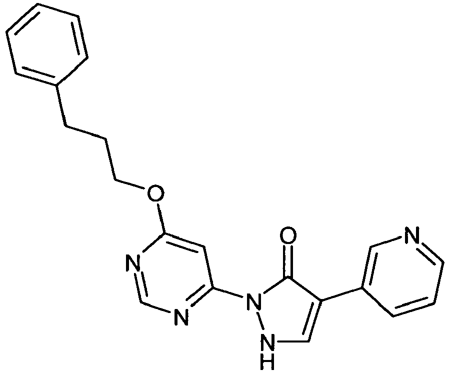

2.3 g (10.0 mmol) der Beispielverbindung 23 werden in 30 ml THF gelöst und als Stammlösung bereitgestellt.

Eine Lösung von 14 mg (0.1 mmol) 3-Phenylpropan-1-ol in 300 µl THF wird mit 5 mg (0.1 mmol) Natriumhydrid (60%-ig in Mineralöl) versetzt und 10 min bei RT geschüttelt. Nach Zugabe von 300 µl (0.1 mmol) der obigen Stammlösung und 2 mg (0.1 mmol) Tetra-n-butylammoniumiodid wird die Reaktionsmischung 16 h bei RT gerührt. Zur Aufarbeitung lässt man das Lösungsmittel abdampfen. Der Rückstand wird in DMSO aufgenommen und filtriert. Das Filtrat wird mittels präparativer LC-MS (Methode 13) aufgereinigt. Die Produktfraktionen werden im Vakuum aufkonzentriert und der Rückstand getrocknet.
Ausbeute: 4 mg (10% d. Th.)
LC-MS (Methode 13): Rₜ = 1.85 min; MS (ESIpos): m/z = 374 [M+H]⁺.

Analog zur Arbeitsvorschrift von Beispiel 88 werden die in Tabelle 5 aufgeführten Verbindungen aus 0.1 mmol der Beispielverbindung 23 und 0.1 mmol des entsprechenden Alkohols hergestellt:

**Tabelle 5**

| **Beispiel Nr.** | **Struktur** | **Ausbeute (% d. Th.)** | **MS (ESI): [M+H]⁺; LC-MS: Rₜ (Methode 13)** |
|---|---|---|---|
| **89** | 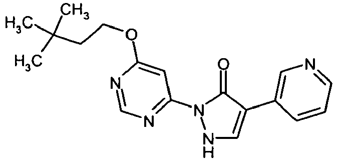 | 15% | m/z = 340; 1.91 min |
| **90** | 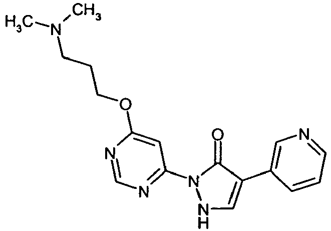 | 2% | m/z = 341; 0.30 min |
| **91** | 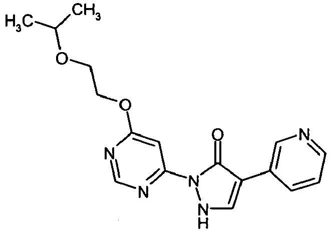 | 27% | m/z = 342; 1.47 min |
| **92** | 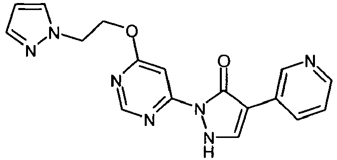 | 3% | m/z = 350; 1.37 min |
| **93** | 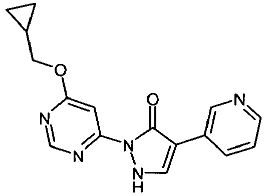 | 7% | m/z = 310; 1.52 min |
| **94** | 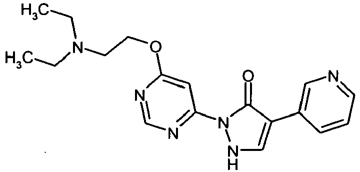 | 4% | m/z = 355; 1.09 min |
| **95** | 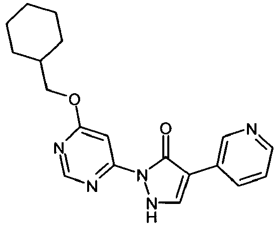 | 2% | m/z = 352; 1.92 min |
| **96** | 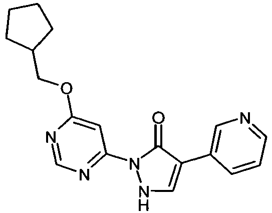 | 2% | m/z = 338; 1.87 min |
| **97** | 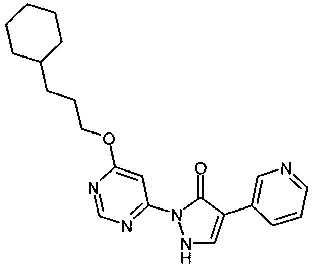 | 1% | m/z = 380; 2.26 min |
| **98** | 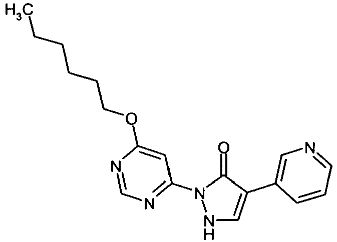 | 2% | m/z = 340; 2.01 min |
| **99** | 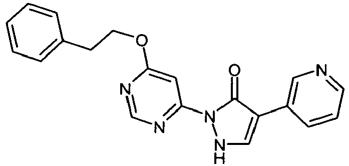 | 5% | m/z = 360; 1.73 min |

### Beispiel 100

### 2-{6-[(Azetidin-3-ylmethyl)amino]pyrimidin-4-yl}-4-pyridin-3-yl-1,2-dihydro-3H-pyrazol-3-on-Dihydrochlorid

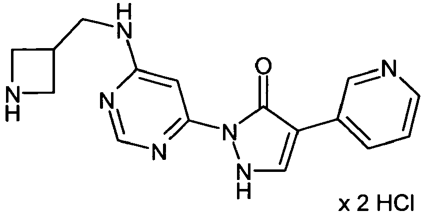

### Stufe a): tert.-Butyl-3-({[6-(5-oxo-4-pyridin-3-yl-2,5-dihydro-1H-pyrazol-1-yl)pyrimidin-4-yl]amino}methyl)azetidin-1-carboxylat

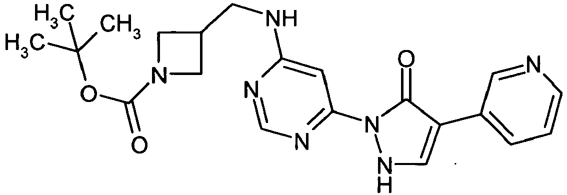

300 mg (1.1 mmol) der Verbindung aus Beispiel 23 werden in 6 ml Ethanol vorgelegt. Man gibt 408 mg (2.2 mmol) *tert*.-Butyl-3-(aminomethyl)azetidin-1-carboxylat hinzu und setzt zunächst 40 min bei 120°C, dann nochmals 40 min bei 150°C in einer *single mode*-Mikrowelle (CEM Explorer) um. Anschließend filtriert man den Feststoff ab, wäscht zweimal mit Methanol nach, verwirft den Feststoff und vereinigt die Waschlösungen mit der Mutterlauge. Man engt diese am Rotationsverdampfer ein und reinigt den Rückstand mittels präparativer HPLC (RP18-Säule; Laufmittel: Acetonitril/Wasser-Gradient). Man vereinigt die produkthaltigen Fraktionen, engt am Rotationsverdampfer ein und erhält 354 mg eines Rückstandes, welcher laut LC-MS und ¹H-NMR der Titelverbindung in einer Reinheit von ca. 50% entspricht und als solches weiter umgesetzt wird.

### Stufe b): 2-{6-[(Azetidin-3-ylmethyl)amino]pyrimidin-4-yl}-4-pyridin-3-yl-1,2-dihydro-3H-pyrazol-3-on-Dihydrochlorid

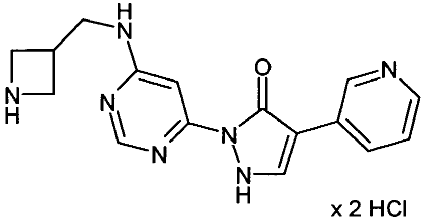

Man legt 301 mg der oben erhaltenen Zwischenstufe in 3 ml Dichlormethan vor, versetzt bei RT unter Rühren mit 1.1 ml (14.2 mmol) TFA und rührt 30 min bei RT. Anschließend verdünnt man das Reaktionsgemisch mit Methanol und engt am Rotationsverdampfer ein. Man gibt erneut Methanol hinzu, engt wieder ein und wiederholt diese Prozedur noch zweimal. Anschließend verrührt man den Rückstand 30 min bei RT in *tert*.-Butylmethylether, filtriert den Feststoff ab und trocknet ihn im Hochvakuum. Man reinigt dann mittels präparativer HPLC (RP18-Säule; Laufmittel: Acetonitril/Wasser-Gradient unter Zusatz von 0.1% TFA im Wasser). Die produkthaltigen Fraktionen werden vereinigt und am Rotationsverdampfer eingeengt. Der Rückstand wird 30 min in 4 ml einer 4 N Lösung von Chlorwasserstoff in Dioxan verrührt. Man filtriert den Feststoff ab und trocknet ihn im Hochvakuum.
Ausbeute: 29 mg (18% d. Th.)
LC-MS (Methode 7): Rₜ = 0.21 min; MS (ESIpos): m/z = 324 [M+H]⁺;
¹H-NMR (400 MHz, DMSO-d₆): δ = 10.36 (s, 1H), 9.33 (s, 1H), 8.89 (d, 1H), 8.81 (s, 1H), 8.72 (s, 1H), 8.56 (d, 1H), 8.50 (br. s, 3H), 8.03 (s, 1H), 7.94 (dd, 1H), 4.40 (dd, 1H), 4.30 (dd, 1H), 3.70-3.60 (m, 2H), 3.02-2.92 (m, 2H), 2.75-2.65 (m, 1H).

### B. Bewertung der pharmakologischen Wirksamkeit

Die pharmakologischen Eigenschaften der erfindungsgemäßen Verbindungen können in folgenden Assays gezeigt werden:

### Abkürzungen:

- DMEM: Dulbecco's Modified Eagle Medium
- FCS: Fetal Calf Serum
- TMB: 3,3',5,5'-Tetramethylbenzidin
- Tris: Tris(hydroxymethyl)-aminomethan

### 1. In vitro-Tests zur Bestimmung der Aktivität und Selektivität von HIF-Prolyl-4-Hydroxylase-Inhibitoren

### 1.a) Hemmung der Aktivität von HIF-Prolylhydroxylase:

Hydroxylierter HIF bindet spezifisch an den von Hippel-Lindau Protein-Elongin B-Elongin C-Komplex (VBC-Komplex). Diese Interaktion tritt nur auf, wenn HIF an einem konservierten Prolylrest hydroxyliert ist. Sie ist die Grundlage für die biochemische Bestimmung der HIF-Prolylhydroxylase-Aktivität. Der Test wird wie beschrieben durchgeführt [Oehme F., Jonghaus W., Narouz-Ott L., Huetter J., Flamme I., Anal. Biochem. 330 (1), 74-80 (2004)]:

Eine klare, mit NeutrAvidin HBC beschichtete 96-Loch-Mikrotiterplatte (Fa. Pierce) wird für 30 Minuten mit Blocker-Casein inkubiert. Anschließend wird die Platte dreimal mit je 200 µl Waschpuffer (50 mM Tris, pH 7.5, 100 mM NaCl, 10% (v/v) Blocker-Casein, 0.05% (v/v) Tween 20) pro Loch gewaschen. Das Peptid Biotin-DLDLEMLAPYIPMDDDFQL (Fa. Eurogentec, 4102 Seraing, Belgien) wird in einer Konzentration von 400 nM in 100 µl Waschpuffer zugegeben. Dieses Peptid dient als Substrat für die Prolylhydroxylierung und wird an die Mikrotiterplatte gebunden. Nach 60 Minuten Inkubation wird die Platte dreimal mit Waschpuffer gewaschen, 30 Minuten mit 1 mM Biotin in Blocker-Casein inkubiert und dann erneut dreimal mit Waschpuffer gewaschen.

Zur Durchführung der Prolylhydroxylase-Reaktion wird das an die Platte gebundene Peptidsubstrat 1 bis 60 Minuten mit einem Prolylhydroxylase-haltigen Zelllysat inkubiert. Die Reaktion findet in 100 µl Reaktionspuffer (20 mM Tris, pH 7.5, 5 mM KCl, 1.5 mM MgCl₂, 1 µM-1 mM 2-Oxoglutarat, 10 µM FeSO₄, 2 mM Ascorbat) bei Raumtemperatur statt. Die Reaktionsmischung enthält außerdem den zu testenden Hemmstoff der Prolylhydroxylase in verschiedenen Konzentrationen.

Die Testsubstanz wird bevorzugt, aber nicht ausschließlich bei Konzentrationen zwischen 1 nM und 100 µM eingesetzt. Durch dreimaliges Waschen der Platte mit Waschpuffer wird die Reaktion gestoppt.

Zur quantitativen Bestimmung der Prolylhydroxylierung wird ein Fusionsprotein, das sowohl Thioredoxin aus E. coli als auch den VBC-Komplex enthält, in 80 µl Bindungspuffer (50 mM Tris, pH 7.5, 120 mM NaCl) zugegeben. Nach 15 Minuten werden 10 µl einer Lösung von polyklonalem Anti-Thioredoxin-Antikörper aus Kaninchen in Bindungspuffer zugefügt. Nach weiteren 30 Minuten setzt man 10 µl einer Lösung von mit Meerrettich-Peroxidase gekoppeltem Anti-Kaninchen-Immunglobulin in Bindungspuffer hinzu. Nach 30 Minuten Inkubation bei Raumtemperatur wird dreimal mit Waschpuffer gewaschen, um ungebundenen VBC-Komplex und Antikörper zu entfernen. Um die Menge an gebundenem VBC-Komplex zu bestimmen, wird 15 Minuten mit TMB inkubiert. Die Farbreaktion wird durch Zugabe von 100 µl 1 M Schwefelsäure beendet. Durch Messung der optischen Dichte bei 450 nm wird die Menge an gebundenem VBC-Komplex bestimmt. Sie ist proportional zur Menge an hydroxyliertem Prolin im Peptidsubstrat.

Zur Detektion der Prolylhydroxylierung kann alternativ ein mit Europium (Fa. Perkin Elmer) gekoppelter VBC-Komplex verwendet werden. In diesem Fall wird die Menge an gebundenem VBC-Komplex durch zeitaufgelöste Fluoreszenz bestimmt. Außerdem ist die Verwendung von mit [³⁵S]-Methionin markiertem VBC-Komplex möglich. Hierfür kann der radioaktiv markierte VBC-Komplex durch *in vitro*-Transkription-Translation in Retikulozytenlysat hergestellt werden.

Die Ausführungsbeispiele hemmen die Aktivität der HIF-Prolylhydroxylase in diesem Test mit einem IC₅₀-Wert von ≤ 30 µM. In der nachstehenden Tabelle 1 sind repräsentative IC₅₀-Werte zu den Ausführungsbeispielen wiedergegeben:

**Tabelle 1**

| **Beispiel Nr.** | **IC₅₀ [µM]** |
|---|---|
| 3 | 0.7 |
| 6 | 2.8 |
| 7 | 0.88 |
| 16 | 0.48 |
| 18 | 0.18 |
| 22 | 2.7 |
| 26 | 4.0 |
| 28 | 0.89 |
| 44 | 1.12 |
| 46 | 0.93 |
| 55 | 1.7 |

### 1.b) Zellulärer, funktioneller in vitro-Test:

Die Quantifizierung der Wirksamkeit der erfindungsgemäßen Verbindungen erfolgt mit Hilfe einer rekombinanten Zelllinie. Die Zelle leitet sich ursprünglich von einer humanen Lungencarcinom-Zelllinie ab (A549, ATCC: American Type Culture Collection, Manassas, VA 20108, USA). Die Testzelllinie wird stabil mit einem Vektor transfiziert, der das Reportergen der *Photinus pyralis-*Luciferase (im folgenden Luciferase genannt) unter der Kontrolle eines artifiziellen Minimalpromotors enthält. Der Minimalpromotor besteht aus zwei Hypoxie-responsiblen Elementen stromaufwärts einer TATA-Box [Oehme F., Ellinghaus P., Kolkhof P., Smith T.J., Ramakrishnan S., Hütter J., Schramm M., Flamme I., Biochem. Biophys. Res. Commun. 296 (2), 343-9 (2002)]. Unter Einwirkung von Hypoxie (z.B. Kultivierung in Gegenwart von 1 % Sauerstoff für 24 Stunden) oder unter Einwirkung unselektiver Dioxygenase-Inhibitoren (z.B. Desferroxamin in einer Konzentration von 100 µM, Cobaltchlorid in einer Konzentration von 100 µM oder *N*-Oxalylglycindiethyl-ester in einer Konzentration von 1 mM) produziert die Testzelllinie Luciferase, die mit Hilfe geeigneter Biolumineszenz-Reagenzien (z.B. Steady-Glo^{®} Luciferase Assay System, Promega Corporation, Madison, WI 53711, USA) und eines geeigneten Luminometers detektiert und quantifiziert werden kann.

Testablauf:Die Zellen werden am Tag vor dem Test in einer exakt bemessenen Menge Kulturmedium (DMEM, 10% FCS, 2 mM Glutamin) in 384- oder 1536-Loch-Mikrotiterplatten ausplattiert und in einem Zellinkubator (96% Luftfeuchtigkeit, 5% v/v CO₂, 37°C) gehalten. Am Testtag werden dem Kulturmedium die Testsubstanzen in abgestuften Konzentrationen zugesetzt. In als Negativkontrolle dienenden Ansätzen wird den Zellen keine Testsubstanz zugesetzt. Als Positivkontrolle zur Bestimmung der Empfindlichkeit der Zelle für Inhibitoren wird z.B. Desferroxamin in einer Endkonzentration von 100 µM zugesetzt. Sechs bis 24 Stunden nach Übertragung der Testsubstanzen in die Löcher der Mikrotiterplatten wird das resultierende Lichtsignal im Luminometer gemessen. Anhand der Meßwerte wird eine Dosiswirkungsbeziehung aufgestellt, die als Grundlage für die Ermittlung der halbmaximalen Wirkkonzentration (als EC₅₀-Wert bezeichnet) dient.

### 1.c) Zellulärer, funktioneller in vitro-Test zur Veränderung der Genexpression:

Um die Veränderung der Expression spezifischer mRNAs in menschlichen Zelllinien nach Behandlung mit Testsubstanzen zu untersuchen, werden folgende Zelllinien auf 6- oder 24-Loch-platten kultiviert: humane Hepatomzellen (HUH, JCRB Cell Bank, Japan), humane embryonale Nierenfibroblasten (HEK/293, ATCC, Manassas, VA 20108, USA), humane Cervixcarcinomzellen (HeLa, ATCC, Manassas, VA 20108, USA), humane Nabelschnurvenenendothelzellen (HUVEC, Cambrex, East Rutherford, New Jersey 07073, USA). 24 Stunden nach Zugabe der Testsubstanzen werden die Zellen mit Phosphat-gepufferter Saline gewaschen und aus ihnen die Gesamt-RNA unter Verwendung einer geeigneten Methode gewonnen (z.B. Trizol^{®}-Reagenz, Invitrogen GmbH, 76131 Karlsruhe, Deutschland).

Für ein typisches Analyseexperiment wird je 1 µg der so gewonnenen Gesamt-RNA mit DNase I verdaut und unter Verwendung einer geeigneten Reversen-Transkriptase-Reaktion (ImProm-II Reverse Transcription System, Promega Corporation, Madison, WI 53711, USA) in eine komplementäre DNA (cDNA) übersetzt. 2.5% des so gewonnenen cDNA-Ansatzes werden jeweils für die Polymerase-Kettenreaktion verwendet. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht. Die hierbei benutzten Primer-Sonden-Kombinationen werden mittels der Primer Express 1.5 Software (Fa. Applied Biosystems, Inc.) generiert. Im einzelnen werden die mRNAs von Erythropoetin, Carboanhydrase IX, Lactatdehydrogenase A und vaskulärem Endothelzellwachstumsfaktor untersucht.

Substanzen gemäß der vorliegenden Erfindung führen zu einem signifikanten, dosisabhängigen Anstieg der mRNA Hypoxie-induzierter Gene in Zellen menschlichen Ursprungs.

### 2. In vivo-Tests zum Nachweis der Wirkung im Kardiovaskularsystem

### 2.a) In vivo-Test zur Veränderung der Genexpression:

Mäusen oder Ratten werden die in geeigneten Lösungsmitteln gelösten Prüfverbindungen entweder oral durch Schlundsonden-Applikation, intraperitoneal oder intravenös verabfolgt. Typische Dosierungen sind 0.1, 0.5, 1, 5, 10, 20, 50, 100 und 300 mg Substanz pro kg Körpergewicht und Verabfolgung. Kontrolltiere erhalten nur Lösungsmittel. 4, 8 oder 24 Stunden nach Gabe der Prüf-substanz werden die Tiere mit einer Überdosis Isofluran und anschließendem Genickbruch getötet und die zu untersuchenden Organe entnommen. Teile der Organe werden in flüssigem Stickstoff schockgefroren. Aus den Organteilen wird wie unter B.1.a) beschrieben Gesamt-RNA gewonnen und diese in eine cDNA übersetzt. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht.

Substanzen gemäß der vorliegenden Erfindung führen im Vergleich mit der Placebo-Kontrolle nach oraler oder parenteraler Verabreichung zu einem signifikanten, dosisabhängigen Anstieg der mRNA des Erythropoetins in der Niere.

### 2.b) Bestimmung des Erythropoetin-Spiegels im Serum:

Mäusen oder Ratten wird die Prüfsubstanz in einem geeigneten Lösungsmittel entweder intraperitoneal oder oral einmal oder zweimal täglich verabreicht. Typische Dosierungen sind 0.1, 0.5, 1, 5, 10, 20, 50, 100 und 300 mg Substanz pro kg Körpergewicht und Verabfolgung. Placebo-Kontrolltiere erhalten nur Lösungsmittel. Vor der Applikation und vier Stunden nach der letzten Substanzgabe wird den Tieren in Kurznarkose aus dem retroorbitalen Venenplexus oder der Schwanzvene 50 µl Blut entnommen. Das Blut wird durch Zusatz von Lithium-Heparin ungerinnbar gemacht. Durch Zentrifugieren wird das Blutplasma gewonnen. In dem Blutplasma wird mit Hilfe eines Erythropoetin-ELISA (Quantikine^{®} mouse Epo Immunoassay, R&D Systems, Inc., Minneapolis, USA) entsprechend der Anleitung des Herstellers der Gehalt an Erythropoetin bestimmt. Die Meßwerte werden anhand einer für Maus-Erythropoetin erhobenen Referenzmessung in pg/ml umgerechnet.

Substanzen gemäß der vorliegenden Erfindung führen nach oraler und parenteraler Verabreichung zu einem signifikanten, dosisabhängigen Anstieg des Plasma-Erythropoetins gegenüber dem Ausgangswert und der Placebo-Kontrolle.

### 2.c) Bestimmung der zellulären Zusammensetzung des peripheren Blutes:

Mäusen oder Ratten wird die Prüfsubstanz in einem geeigneten Lösungsmittel entweder intraperitoneal oder oral einmal oder zweimal täglich über mehrere Tage verabreicht. Typische Dosierungen sind z.B. 0.1, 0.5, 1, 5, 10, 20, 50, 100 und 300 mg Substanz pro kg Körpergewicht und Verabfolgung. Kontrolltiere erhalten nur Lösungsmittel. Am Versuchsende wird den Tieren in Kurznarkose aus dem Venenplexus des Augenwinkels oder der Schwanzvene Blut entnommen und durch Zusatz von Natriumcitrat ungerinnbar gemacht. In einem geeigneten elektronischen Messgerät werden in den Blutproben die Konzentrationen von Erythrozyten, Leukozyten und Thrombozyten bestimmt. Die Konzentration der Retikulozyten wird anhand von Blutausstrichen, die mit einer dafür geeigneten Farblösung (Fa. KABE Labortechnik, Nümbrecht) gefärbt werden, durch mikroskopische Durchmusterung von je 1000 Erythrozyten bestimmt. Für die Bestimmung des Hämatokrits wird Blut aus dem retroorbitalen Venenplexus mittels einer Hämatokritkapillare entnommen und der Hämatokritwert nach Zentrifugieren der Kapillare in einer dafür geeigneten Zentrifuge manuell abgelesen.

Substanzen gemäß der vorliegenden Erfindung führen nach oraler und parenteraler Verabreichung zu einem signifikanten, dosisabhängigen Anstieg des Hämatokrits, der Erythrozytenzahl und der Retikulozyten gegenüber dem Ausgangswert und der Placebo-Kontrolle.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 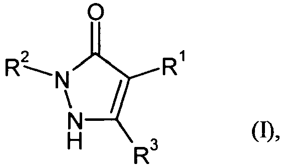 in welcher
R¹ für eine Heteroaryl-Gruppe der Formel 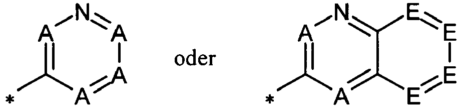 steht, worin
* die Verknüpfungsstelle mit dem Dihydropyrazolon-Ring bedeutet,
A bei jedem einzelnen Auftreten C-R⁴ oder N bedeutet, wobei maximal zwei Ringglieder A zugleich für N stehen,
und
E bei jedem einzelnen Auftreten C-R⁵ oder N bedeutet, wobei maximal zwei Ringglieder E zugleich für N stehen,
R² für eine Heteroaryl-Gruppe der Formel 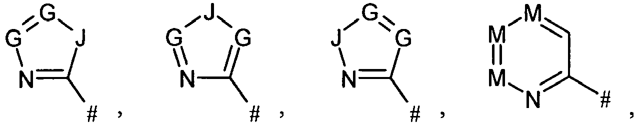 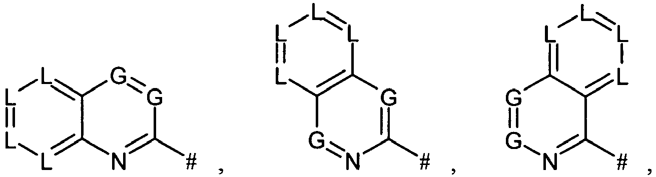 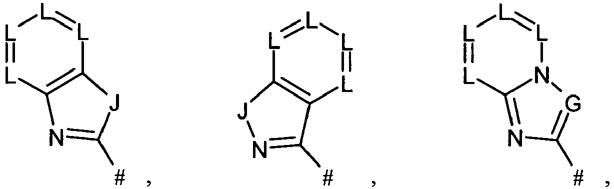 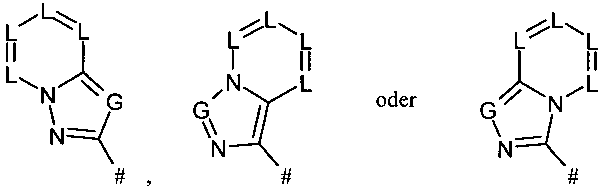 steht, worin
# die Verknüpfungsstelle mit dem Dihydropyrazolon-Ring bedeutet,
G bei jedem einzelnen Auftreten C-R⁶ oder N bedeutet,
J O, S oder N-R⁷ bedeutet,
L bei jedem einzelnen Auftreten C-R⁸ oder N bedeutet, wobei maximal zwei Ringglieder L zugleich für N stehen,
und
M bei jedem einzelnen Auftreten C-R⁹ oder N bedeutet, wobei insgesamt ein oder zwei Ringglieder M für N stehen, worin
R⁴, R⁶, R⁸ und R⁹ gleich oder verschieden sind und in jedem einzelnen Fall, unabhängig voneinander, für Wasserstoff oder einen Substituenten ausgewählt aus der Reihe Halogen, Cyano, Nitro, (C₁-C₆)-Alkyl, (C₃-C₇)-Cycloalkyl, 4- bis 10-gliedriges Heterocycloalkyl, Phenyl, 5- oder 6-gliedriges Heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² stehen, worin
(i) (C₁-C₆)-Alkyl seinerseits ein- bis dreifach, gleich oder verschieden, mit Resten ausgewählt aus der Reihe Halogen, Cyano, Oxo, (C₃-C₇)-Cycloalkyl, 4- bis 10-gliedriges Heterocycloalkyl, Phenyl, 5- oder 6-gliedriges Heteroaryl, -C(=O)-R¹⁰, -C(=O)-OR¹¹, -C(=O)-NR¹²R¹³, -O-C(=O)-R¹⁴ -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂R²⁵, -SO₂-R²⁶, -SO₂-NR²⁷R²⁸, -OR²⁹, -SR³⁰ und -NR³¹R³² substituiert sein kann,
wobei die zuletzt genannten Cycloalkyl-, Heterocycloalkyl-, Phenyl- und Heteroaryl-Reste ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*ii*) (C₃-C₇)-Cycloalkyl, 4- bis 10-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, (C₃-C₇)-Cycloalkyl, Halogen, Cyano, Oxo, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² substituiert sein können,
wobei der zuletzt genannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cycloalkyl, 4- bis 7-gliedrigem Heterocycloalkyl, Phenyl und/oder 5- oder 6-gliedrigem Heteroaryl substituiert sein kann,
(*iii*) R¹⁰, R¹¹, R¹², R¹⁴, R¹⁵, R¹⁸, R²⁰, R²², R²⁵, R²⁶, R²⁷, R²⁹, R³⁰ und R³¹ unabhängig voneinander bei jedem einzelnen Auftreten für einen Rest ausgewählt aus der Reihe Wasserstoff, (C₁-C₆)-Alkyl, (C₃-C₇)-Cycloalkyl, 4- bis 7-gliedriges Heterocycloalkyl, Phenyl und 5- oder 6-gliedriges Heteroaryl stehen, wobei
(C₃-C₇)-Cycloalkyl, 4- bis 7-gliedriges Heterocycloalkyl, Phenyl und 5- oder 6-gliedriges Heteroaryl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alk-oxycarbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cyclo-alkyl, 4- bis 7-gliedrigem Heterocycloalkyl, Phenyl und/oder 5- oder 6-gliedrigem Heteroaryl substituiert sein kann,
worin der zuletzt genannte Heterocycloalkyl-Rest seinerseits bis zu zweifach, gleich oder verschieden, mit (C₁-C₄)-Alkyl substituiert sein kann,
(*iv*) R¹³, R¹⁶, R¹⁷, R¹⁹, R²¹, R²³, R²⁴, R²⁸ und R³² unabhängig von-einander bei jedem einzelnen Auftreten für einen Rest ausgewählt aus der Reihe Wasserstoff und (C₁-C₆)-Alkyl stehen, wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und/oder worin
(v) R¹² und R¹³, R¹⁵ und R¹⁶, R¹⁷ und R¹⁸, R¹⁹ und R²⁰, R²¹ und R²², R²² und R²³, R²⁴ und R²⁵, R²⁷ und E²⁸ sowie R³¹ und R³² jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
R⁵ in jedem einzelnen Fall, unabhängig voneinander, für Wasserstoff oder einen Substituenten ausgewählt aus der Reihe Halogen, Cyano, Nitro, (C₁-C₆)-Alkyl, Trifluormethyl, Hydroxy, (C₁-C₆)-Alkoxy, Trifluormethoxy, Amino, Mono-(C₁-C₆)-alkylamino, Di-(C₁-C₆)-alkylamino, Hydroxycarbonyl und (C₁-C₆)-Alkoxycarbo-nyl steht
und
R⁷ für Wasserstoff oder einen Substituenten ausgewählt aus der Reihe (C₁-C₆)-Alkyl, (C₃-C₇)-Cycloalkyl, 4- bis 7-gliedriges Heterocyclo-alkyl, Phenyl und 5- oder 6-gliedriges Heteroaryl steht, worin
(i) (C₁-C₆)-Alkyl seinerseits ein- bis dreifach, gleich oder verschieden, mit Resten ausgewählt aus der Reihe Halogen, Cyano, Oxo, (C₃-C₇)-Cycloalkyl, 4- bis 7-gliedriges Heterocycloalkyl, Phenyl, 5- oder 6-gliedriges Heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² substituiert sein kann,
wobei die zuletzt genannten Cycloalkyl-, Heterocycloalkyl-, Phenyl- und Heteroaryl-Reste ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
und
(*ii*) (C₃-C₇)-Cycloalkyl, 4- bis 7-gliedriges Heterocycloalkyl, Phenyl und 5- oder 6-gliedriges Heteroaryl ihrerseits jeweils ein- bis dreifach, gleich oder verschieden, mit Resten ausgewählt aus der Reihe (C₁-C₆)-Alkyl, Halogen, Cyano, Oxo, -C(=O)-R¹⁰, -C(-O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ und -NR³¹R³² substituiert sein können,
wobei der zuletzt genannte Alkyl-Rest seinerseits bis zu dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cycloalkyl, 4- bis 7-gliedrigem Heterocycloalkyl, Phenyl und/oder 5- oder 6-gliedrigem Heteroaryl substituiert sein kann,
worin
(*a*) R¹⁰, R¹¹, R¹², R¹⁴, R¹⁵, R¹⁸, R²⁰, R²², R²⁵, R²⁶, R²⁷, R²⁹, R³⁰ und R³¹ unabhängig voneinander bei jedem einzelnen Auftreten für einen Rest ausgewählt aus der Reihe Wasserstoff, (C₁-C₆)-Alkyl, (C₃-C₇)-Cycloalkyl, 4- bis 7-gliedriges Heterocyclo-alkyl, Phenyl und 5- oder 6-gliedriges Heteroaryl stehen, wobei
(C₃-C₇)-Cycloalkyl, 4- bis 7-gliedriges Heterocycloalkyl, Phenyl und 5- oder 6-gliedriges Heteroaryl ihrerseits jeweils bis zu dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können
und
(C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₇)-Cyclo-alkyl, 4- bis 7-gliedrigem Heterocycloalkyl, Phenyl und/oder 5- oder 6-gliedrigem Heteroaryl substituiert sein kann,
(*b*) R¹³, R¹⁶, R¹⁷, R¹⁹, R²¹, R²³, R²⁴, R²⁸ und R³² unabhängig voneinander bei jedem einzelnen Auftreten für einen Rest ausgewählt aus der Reihe Wasserstoff und (C₁-C₆)-Alkyl stehen,
wobei (C₁-C₆)-Alkyl ein- bis dreifach, gleich oder verschieden, mit Halogen, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und/oder
(c) R¹² und R¹³, R¹⁵ und R¹⁶, R¹⁷ und R¹⁸, R¹⁹ und R²⁰, R²¹ und R²², R²² und R²³, R²⁴ und R²⁵, R²⁷ und R²⁸ sowie R³¹ und R³² jeweils paarweise zusammen mit den Atomen, an die sie gebunden sind, einen 5- oder 6-gliedrigen Heterocycloalkyl-Ring bilden können, welcher ein- bis dreifach, gleich oder verschieden, mit Halogen, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein kann, und
R³ für Wasserstoff, (C₁-C₆)-Alkyl oder (C₃-C₇)-Cycloalkyl steht,
sowie ihre Salze, Solvate und Solvate der Salze.

2. Verbindung der Formel (I) nach Anspruch 1, in welcher
R¹ für eine Heteroaryl-Gruppe der Formel 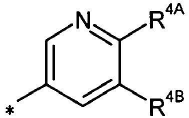 steht, worin
* die Verknüpfungsstelle mit dem Dihydropyrazolon-Ring bedeutet
und
R^{4A} und R^{4B} gleich oder verschieden sind und unabhängig voneinander Wasserstoff oder einen Substituenten ausgewählt aus der Reihe Fluor, Chlor, Brom, Cyano, (C₁-C₆)-Alkyl, Hydroxy, (C₁-C₆)-Alkoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl bedeuten,
wobei der genannte (C₁-C₆)-Alkyl-Rest seinerseits bis zu dreifach, gleich oder verschieden, mit Fluor, Chlor, Brom, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
R² für eine Heteroaryl-Gruppe der Formel 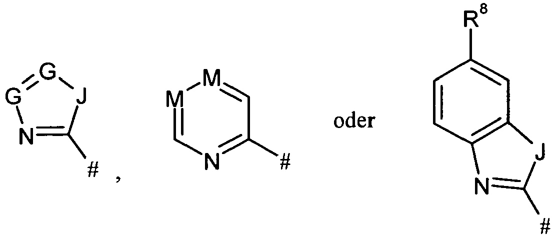 steht, worin
# die Verknüpfungsstelle mit dem Dihydropyrazolon-Ring bedeutet,
G jeweils C-R⁶ oder N bedeutet, wobei nicht mehr als eines der beiden Ring-glieder G für N steht,
J O oder S bedeutet,
M jeweils C-R⁹ oder N bedeutet, wobei eines der beiden Ringglieder M für N und das andere für C-R⁹ steht,
worin
R⁶ und R⁹ in jedem einzelnen Fall, unabhängig voneinander, für Wasserstoff oder einen Substituenten ausgewählt aus der Reihe Fluor, Chlor, Brom, Cyano, (C₁-C₆)-Alkyl, (C₃-C₆)-Cycloalkyl, 4- bis 6-gliedriges Heterocycloalkyl, Phenyl, 5- oder 6-gliedriges Heteroaryl, -C(-O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -OR²⁹ und -NR³¹R³² stehen, worin
(*i*) (C₁-C₆)-Alkyl seinerseits ein- bis dreifach, gleich oder verschieden, mit Resten ausgewählt aus der Reihe Fluor, Chlor, Brom, Cyano, (C₃-C₆)-Cycloalkyl, 4- bis 6-gliedriges Hetero-cycloalkyl, Phenyl, 5- oder 6-gliedriges Heteroaryl, -C(=O)-OR", -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -OR²⁹ und -NR³¹R³² substituiert sein kann,
wobei die zuletzt genannten Cycloalkyl-, Heterocycloalkyl-, Phenyl- und Heteroaryl-Reste ihrerseits jeweils bis zu zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl substituiert sein können,
(*ii*) (C₃-C₆)-Cycloalkyl, 4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/ oder (C₁-C₄)-Alkoxycarbonyl substituiert sein können,
(*iii*) R¹¹, R¹², R¹⁴, R¹⁵, R¹⁸, R²⁰, R²², R²⁵, R²⁹ und R³¹ unabhängig voneinander bei jedem einzelnen Auftreten für einen Rest ausgewählt aus der Reihe Wasserstoff, (C₁-C₆)-Alkyl, (C₃-C₆)-Cycloalkyl, 4- bis 6-gliedriges Heterocycloalkyl, Phenyl und 5- oder 6-gliedriges Heteroaryl stehen, wobei
(C₃-C₆)-Cycloalkyl, 4- bis 6-gliedriges Heterocycloalkyl, Phenyl und 5- oder 6-gliedriges Heteroaryl ihrerseits jeweils bis zu dreifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/ oder (C₁-C₄)-Alkoxycarbonyl substituiert sein können
und
(C₁-C₆)-Alkyl ein- bis dreifach, gleich oder verschieden, mit Fluor, Chlor, Brom, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl, (C₁-C₄)-Alkoxycarbonyl, (C₃-C₆)-Cycloalkyl, 4- bis 6-gliedrigem Heterocycloalkyl, Phenyl und/oder 5- oder 6-gliedrigem Heteroaryl substituiert sein kann,
(*iv*) R¹³, R¹⁶, R¹⁷, R¹⁹, R²¹, R²³, R²⁴ und R³² unabhängig voneinander bei jedem einzelnen Auftreten für einen Rest ausgewählt aus der Reihe Wasserstoff und (C₁-C₆)-Alkyl stehen,
wobei (C₁-C₆)-Alkyl ein- oder zweifach, gleich oder verschieden, mit Fluor, Chlor, Brom, Cyano, Hydroxy, Trifluormethoxy, (C₁-C₄)-Alkoxy, Amino, Mono-(C₁-C₄)-alkylamino, Di-(C₁-C₄)-alkylamino, Hydroxycarbonyl und/oder (C₁-C₄)-Alkoxycarbonyl substituiert sein kann,
und/oder worin
(v) R¹² und R¹³, R¹⁵ und R¹⁶, R¹⁷ und R¹⁸, R¹⁹ und R²⁰, R²¹ und R²², R²² und R²³, R²⁴ und R²⁵ sowie R³¹ und R³² jeweils paarweise zusammen mit den Atomen, an die sie gebunden sind, einen 5- oder 6-gliedrigen Heterocycloalkyl-Ring bilden können, welcher ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-atkylamino, Di-(C₁-C₄)-alkylamino, Hydroxycarbonyl und/oder (C₁-C₄)-Alkoxycarbonyl substituiert sein kann,
und
R⁸ Wasserstoff oder einen Substituenten ausgewählt aus der Reihe Fluor, Chlor, Brom, Cyano, (C₁-C₆)-Alkyl, Hydroxy, (C₁-C₆)-Alkoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl bedeutet,
und
R³ für Wasserstoff oder Methyl steht,
sowie ihre Salze, Solvate und Solvate der Salze.

3. Verbindung der Formel (I) nach Anspruch 1 oder 2, in welcher
R¹ für eine Heteroaryl-Gruppe der Formel 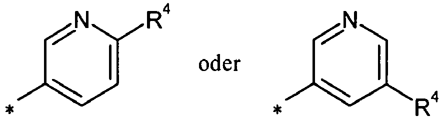 steht, worin
* die Verknüpfungsstelle mit dem Dihydropyrazolon-Ring bedeutet
und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarbonyl bedeutet,
R² für eine Heteroaryl-Gruppe der Formel 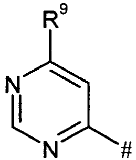 steht, worin
# die Verknüpfungsstelle mit dem Dihydropyrazolon-Ring bedeutet
und
R⁹ Wasserstoff, Fluor, Chlor, Brom, Cyano, (C₁-C₄)-Alkyl, Trifluormethyl, Hydroxy, (C₁-C₄)-Alkoxy, Trifluormethoxy, Amino, Mono-(C₁-C₄)-alkylamino, Di-(C₁-C₄)-alkylamino, Hydroxycarbonyl, (C₁-C₄)-Alkoxycarbonyl, 4- bis 6-gliedriges Heterocycloalkyl, Phenyl oder 5- oder 6-gliedriges Heteroaryl bedeutet, wobei
(C₁-C₄)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁-C₄)-alkylamino, Di-(C₁-C₄)-alkylamino, Hydroxycarbonyl und/oder (C₁-C₄)-Alkoxycarbonyl
substituiert sein können,
und
R³ für Wasserstoff steht,
sowie ihre Salze, Solvate und Solvate der Salze.

4. Verbindung der Formel (I) nach Anspruch 1 oder 2, in welcher
R¹ für eine Heteroaryl-Gruppe der Formel 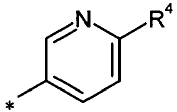 steht, worin
* die Verknüpfungsstelle mit dem Dihydropyrazolon-Ring bedeutet und
R⁴ Wasserstoff, Fluor, Chlor, Brom, Cyano, (C₁-C₄)-Alkyl, Trifluormethyl, Hydroxymethyl, (C₁-C₄)-Alkoxy, Trifluormethoxy, Hydroxycarbonyl oder (C₁-C₄)-Alkoxycarbonyl bedeutet,
R² für eine Heteroaryl-Gruppe der Formel 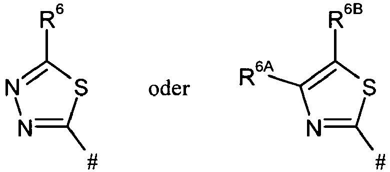 steht, worin
# die Verknüpfungsstelle mit dem Dihydropyrazolon-Ring bedeutet
und
R⁶, R^{6A} und R^{6B} gleich oder verschieden sind und unabhängig voneinander Wasserstoff oder einen Substituenten ausgewählt aus der Reihe Fluor, Chlor, Brom, Cyano, (C₁-C₄)-Alkyl, Trifluormethyl, Hydroxy, (C₁-C₄)-Alkoxy, Trifluormethoxy, Amino, Mono-(C₁-C₄)-alkylamino, Di-(C₁-C₄)-alkyl-amino, Hydroxycarbonyl, (C₁-C₄)-Alkoxycarbonyl, 4- bis 6-gliedriges Heterocycloalkyl, Phenyl und 5- oder 6-gliedriges Heteroaryl bedeuten, wobei
(C₁-C₄)-Alkyl seinerseits mit Hydroxy, (C₁-C₄)-Alkoxy oder Amino
und
4- bis 6-gliedriges Heterocycloalkyl, Phenyl und 5- oder 6-gliedriges Heteroaryl ihrerseits jeweils ein- oder zweifach, gleich oder verschieden, mit Fluor, Chlor, Brom, Cyano, (C₁-C₄)-Alkyl, Trifluormethyl, Hydroxy, (C₁-C₄)-Alkoxy, Trifluormethoxy, Oxo, Amino, Mono-(C₁C₄)-alkylamino, Di-(C₁-C₄)-alkylamino, Hydroxycarbonyl und/oder (C₁-C₄)-Alkoxycarbonyl
substituiert sein können,
und
R³ für Wasserstoff steht,
sowie ihre Salze, Solvate und Solvate der Salze.

5. Verfahren zur Herstellung einer Verbindung der Formel (I), wie in den Ansprüchen 1 bis 4 definiert, **dadurch gekennzeichnet, dass** man eine Verbindung der Formel (II) 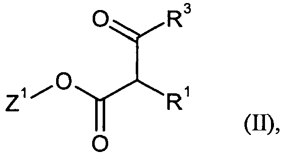 in welcher R¹ und R³ die in den Ansprüchen 1 bis 4 angegebenen Bedeutungen aufweisen und
Z¹ für Methyl oder Ethyl steht,
in einem inerten Lösungsmittel gegebenenfalls in Gegenwart einer Säure mit einer Verbindung der Formel (III) 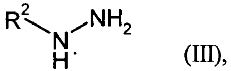 in welcher R² die in den Ansprüchen 1 bis 4 angegebene Bedeutung aufweist, zu Verbindungen der Formel (IV) 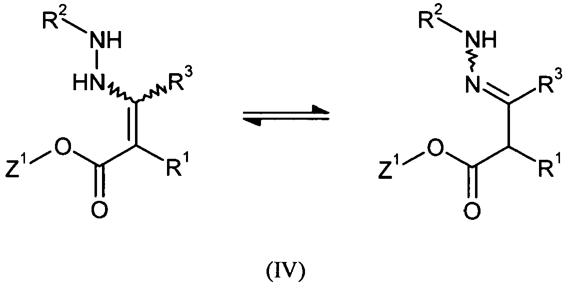 in welcher Z¹, R¹, R² und R³ die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I) cyclisieren,
und die Verbindungen der Formel (I) gegebenenfalls mit den entsprechenden (i) Lösungsmitteln und/oder (ii) Basen oder Säuren in ihre Solvate, Salze und/oder Solvate der Salze überführt.

6. Verfahren zur Herstellung einer Verbindung der Formel (I), wie in den Ansprüchen 1 bis 4 definiert, in welcher R³ Wasserstoff bedeutet, **dadurch gekennzeichnet, dass** man zunächst eine Verbindung der Formel (V) 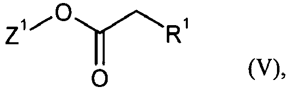 in welcher R¹ die in den Ansprüchen 1 bis 4 angegebene Bedeutung aufweist und
Z¹ für Methyl oder Ethyl steht,
mit einer Verbindung der Formel (VI) 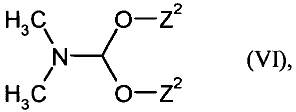 in welcher
Z² für Methyl oder Ethyl steht,
zu Verbindungen der Formel (VII) 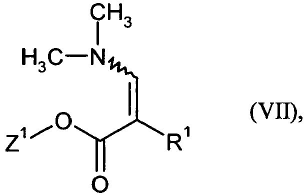 in welcher Z¹ und R¹ die oben angegebenen Bedeutungen aufweisen,
kondensiert und anschließend in Gegenwart einer Säure mit einer Verbindung der Formel (III) 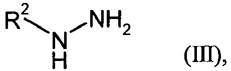 in welcher R² die in den Ansprüchen 1 bis 4 angegebene Bedeutung aufweist, zu Verbindungen der Formel (IV-A) 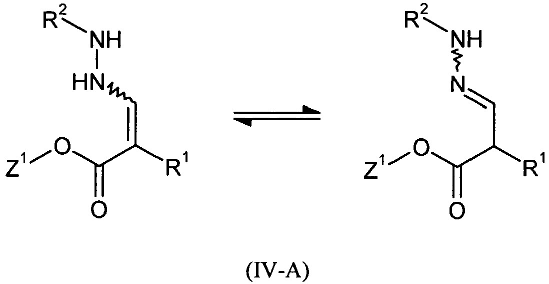 in welcher Z¹, R¹ und R² die oben angegebenen Bedeutungen aufweisen,
umsetzt, welche bereits unter diesen Reaktionsbedingungen oder in einem nachfolgenden Reaktionsschritt unter dem Einfluss einer Base zu den Verbindungen der Formel (I), worin R³ für Wasserstoff steht, cyclisieren.

7. Verbindung, wie in einem der Ansprüche 1 bis 4 definiert, zur Behandlung und/oder Prophylaxe von Krankheiten.

8. Verwendung einer Verbindung, wie in einem der Ansprüche 1 bis 4 definiert, zur Herstellung eines Arzneimittels zur Behandlung und/oder Prophylaxe von Herz-Kreislauf-Erkrankungen, Herzinsuffizienz, Anämie, chronischen Nierenerkrankungen und Niereninsuffizienz.

9. Arzneimittel enthaltend eine Verbindung, wie in einem der Ansprüche 1 bis 4 definiert, in Kombination mit einem inerten, nicht-toxischen, pharmazeutisch geeigneten Hilfsstoff.

10. Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika und Antibiotika.

11. Arzneimittel nach Anspruch 9 oder 10 zur Behandlung und/oder Prophylaxe von Herz-Kreislauf-Erkranlcungen, Herzinsuffizienz, Anämie, chronischen Nierenerkrankungen und Niereninsuffizienz.

## Claims

1. Compound of the formula (I) 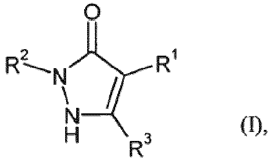 in which
R¹ represents a heteroaryl group of the formula 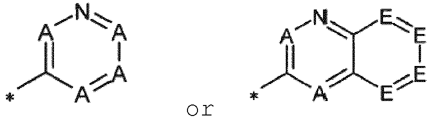 where
* represents the point of attachment to the dihydropyrazolone ring,
A in each individual occurrence represents C-R⁴ or N, where at most two ring members A represent N at the same time, and
E in each individual occurrence represents C-R⁵ or N, where at most two ring members E represent N at the same time,
R² represents a heteroaryl group of the formula 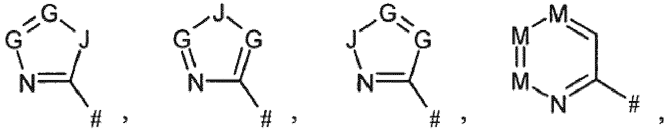 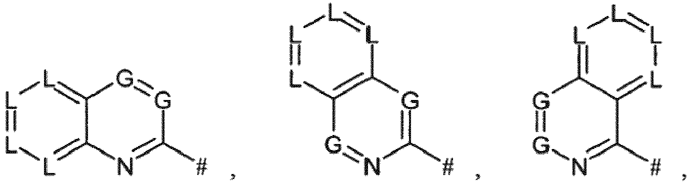 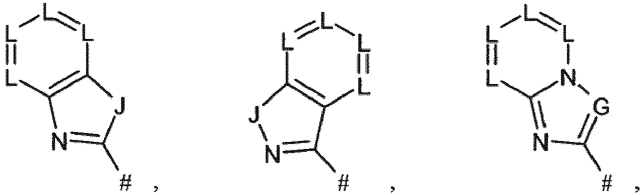 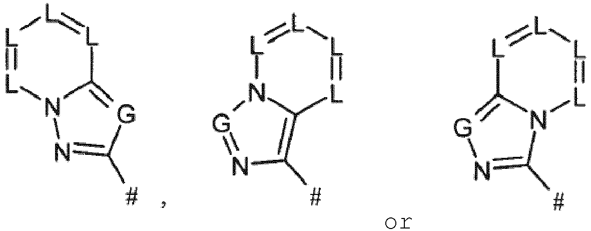 where
# represents the point of attachment to the dihydropyrazolone ring,
G in each individual occurrence represents C-R⁶ or N,
J represents O, S or N-R⁷,
L in each individual occurrence represents C-R⁸ or N, where at most two ring members L represent N at the same time,
and
M in each individual occurrence represents C-R⁹ or N, where in total one or two ring members M represent N,
where
R⁴, R⁶, R⁸ and R⁹ are identical or different and in each individual case independently of one another represent hydrogen or a substituent selected from the group consisting of halogen, cyano, nitro, (C₁-C₆) -alkyl, (C₃-C₇) -cycloalkyl, 4- to 10-membered heterocycloalkyl, phenyl, 5- or 6-membered heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(-O)-NR²²R²³, -NR²⁴-SO²-R²⁵, -SO₂-R²⁶, -SO₂-NR²⁷R²⁸, -OR²⁹, -SR³⁰ and -NR³¹R³², where
(i) (C₁-C₆) -alkyl for its part may be mono- to trisubstituted by identical or different radicals selected from the group consisting of halogen, cyano, oxo, (C₃-C₇)-cycloalkyl, 4- to 10-membered heterocycloalkyl, phenyl, 5- or 6-membered heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C (=O)-R¹⁸, -NR¹⁹-C(=O)-OR²⁰, -NR²¹-C(=O)-NR²²R²³, -NR²⁴-SO₂-R²⁵, ⁻SO₂-R²⁶, -SO₂-NR²⁷R²⁸, -OR²⁹, -SR³⁰ and -NR³¹R³²,
where the last-mentioned cycloalkyl, heterocycloalkyl, phenyl and heteroaryl radicals for their part may in each case be substituted up to three times by identical or different substituents from the group consisting of halogen, cyano, (C₁-C₄) -alkyl, trifluoromethyl, hydroxyl, (C₁-C₄) -alkoxy, trifluoromethoxy, oxo, amino, mono-(C₁-C₄) -alkylamino, di-(C₁-C₄)-alkylamino, hydroxycarbonyl and (C₁-C₄) -alkoxycarbonyl,
(ii) (C₃-C₇) -cycloalkyl, 4- to 10-membered heterocycloalkyl, phenyl and 5- or 6-membered heteroaryl for their part may in each case be mono- to trisubstituted by identical or different radicals selected from the group consisting of (C₁-C₆) -alkyl, (C₃-C₇)-cycloalkyl, halogen, cyano, oxo, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ and -NR³¹R³²,
where the last-mentioned alkyl radical for its part may be substituted up to three times by identical or different substituents from the group consisting of halogen, cyano, hydroxyl, trifluoromethoxy, (C₁-C₄) -alkoxy, amino, morio-(C₁-C₄)-alkylamino, di-(C₁-C₄)-alkylamino, hydroxycarbonyl, (C₁-C₄)-alkoxycarbonyl, (C₃-C₇)-cycloalkyl, 4- to 7-membered heterocycloalkyl, phenyl and 5- or 6-membered heteroaryl,
(*iii*) R¹⁰, R¹¹, R¹², R¹⁴, R¹⁵, R¹⁸, R²⁰ R²², R²⁵, R²⁶, R²⁷, R²⁹, R³⁰ and R³¹ independently of one another in each individual occurrence represent a radical selected from the group consisting of hydrogen, (C₁-C₆)-alkyl, (C₃-C₇)-Cycloalkyl, 4- to 7-membered heterocycloalkyl, phenyl and 5- or 6-membered heteroaryl, where
(C₃-C₇)-cycloalkyl, 4- to 7-membered heterocycloalkyl, phenyl and 5- or 6-membered heteroaryl for their part may in each case be substituted up to three times by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₇)-cycloalkyl, 4- to 7-membered heterocycloalkyl, phenyl and 5- or 6-membered heteroaryl,
where the last-mentioned heterocycloalkyl radical for its part may be substituted up to two times by identical or different substituents from the group consisting of (C₁-C₄)-alkyl,
(*iv*) R¹³, R¹⁶, R¹⁷, R¹⁹, R²¹, R²³, R²⁴, R²⁸ and R³² independently of one another in each individual occurrence represent a radical selected from the group consisting of hydrogen and (C₁-C₆)-alkyl,
where (C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄) -alkylamino, di-(C₁-C₄)-alkylamino, hydroxycarbonyl and (C₁-C₄)-alkoxycarbonyl,
and/or where
(*v*) R¹² and R¹³, R¹⁵ and R¹⁶, R¹⁷ and R¹⁸, R¹⁹ and R²⁰, R²¹ and R²², R²² and R²³, R²⁴ and R²⁵, R²⁷ and R²⁸ and also R³¹ and R³² in each case as a pair together with the atoms to which they are attached may form a 5- or 6-membered heterocycloalkyl ring which may be mono- to trisubstituted by identical or different substituents from the group consisting of halogen, cyano, (C₁-C₄) -alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
R⁵ in each individual case, independently of one another, represents hydrogen or a substituent selected from the group consisting of halogen, cyano, nitro, (C₁-C₆)-alkyl, trifluoromethyl, hydroxyl, (C₁-C₆)-alkoxy, trifluoromethoxy, amino, mono-(C₁-C₆)-alkylamino, di-(C₁-C₆)-alkylamino, hydroxycarbonyl and (C₁-C₆)-alkoxycarbonyl
and
R⁷ represents hydrogen or a substituent selected from the group consisting of (C₁-C₆) -alkyl, (C₃-C₇) -cycloalkyl, 4- to 7-membered heterocycloalkyl, phenyl and 5- or 6-membered heteroaryl, where
(*i*) (C₁-C₆) -alkyl for its part may be mono- to trisubstituted by identical or different radicals selected from the group consisting of halogen, cyano, oxo, (C₃-C₇)-cycloalkyl, 4- to 7-membered heterocycloalkyl, phenyl, 5- or 6-membered heteroaryl, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴- SO₂-R²⁵, -SO₂-R²⁶, -SO₂-NR²⁷R²⁸, -OR²⁹, -SR³⁰ and -NR³¹R³²,
where the last-mentioned cycloalkyl, heterocycloalkyl, phenyl and heteroaryl radicals for their part may in each case be substituted up to three times by identical or different substituents from the group consisting of halogen, cyano, (C₁-C₄) -alkyl, trifluoromethyl, hydroxyl, (C₁-C₄) -alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
and
(*ii*) (C₃-C₇) -cycloalkyl, 4- to 7-membered heterocycloalkyl, phenyl and 5- or 6-membered heteroaryl for their part may in each case be mono- to trisubstituted by identical or different radicals selected from the group consisting of (C₁-C₆)-alkyl, halogen, cyano, oxo, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ and -NR³¹R³²,
where the last-mentioned alkyl radical for its part may be substituted up to three times by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄) -alkoxycarbonyl, (C₃-C₇)-cycloalkyl, 4- to 7-membered heterocycloalkyl, phenyl and 5- or 6-membered heteroaryl,
where
(*a*) R¹⁰, R¹¹, R¹², R¹⁴, R¹⁵, R¹⁸, R²⁰, R²², R²⁵, R²⁶, R²⁷, R²⁹, R³⁰ and R³¹ independently of one another in each individual occurrence represent a radical selected from the group consisting of hydrogen, (C₁-C₆)-alkyl, (C₃-C₇)-cycloalkyl, 4- to 7-membered heterocycloalkyl, phenyl and 5- or 6-membered heteroaryl, where
(C₃-C₇)-cycloalkyl, 4- to 7-membered heterocycloalkyl, phenyl and 5- or 6-membered heteroaryl for their part may in each case be substituted up to three times by identical or different substituents from the group consisting of halogen, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₇)-cycloalkyl, 4- to 7-membered heterocycloalkyl, phenyl and 5- or 6-membered heteroaryl,
(*b*) R¹³, R¹⁶, R¹⁷, R¹⁹, R²¹, R²³, R²⁴, R²⁸ and R³² independently of one another in each individual occurrence represent a radical selected from the group consisting of hydrogen and (C₁-C₆)-alkyl,
where (C₁-C₆)-alkyl may be mono- to trisubstituted by identical or different substituents from the group consisting of halogen, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
and/or
(*c*) R¹² and R¹³, R¹⁵ and R¹⁶, R¹⁷ and R¹⁸, R¹⁹ and R²⁰, R²¹ and R²², R²² and R²³, R²⁴ and R²⁵, R²⁷ and R²⁸ and also R³¹ and R³² in each case as a pair together with the atoms to which they are attached may form a 5- or 6-membered heterocycloalkyl ring which may be mono- to trisubstituted by identical or different substituents from the group consisting of halogen, cyano, (C₁-C₄)-alkyl, trifluoro-methyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl, and
R³ represents hydrogen, (C₁-C₆)-alkyl or (C₃-C₇)-cycloalkyl,
and salts, solvates and solvates of the salts thereof.

2. Compound of the formula (I) according to Claim 1 in which
R¹ represents a heteroaryl group of the formula 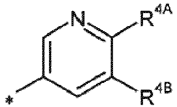 where
* represents the point of attachment to the
dihydropyrazolone ring
and
R^{4A} and R^{4B} are identical or different and independently of one another represent hydrogen or a substituent selected from the group consisting of fluorine, chlorine, bromine, cyano, (C₁-C₆)-alkyl, hydroxyl, (C₁-C₆)-alkoxy, trifluoromethoxy, amino, mono-(C₁-C₆)-alkylamino, di-(C₁-C₆)-alkylamino, hydroxycarbonyl and (C₁-C₆)-alkoxycarbonyl,
where the (C₁-C₆)-alkyl radical mentioned for its part may be substituted up to three times by identical or different substituents from the group consisting of fluorine, chlorine, bromine, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
R² represents a heteroaryl group of the formula 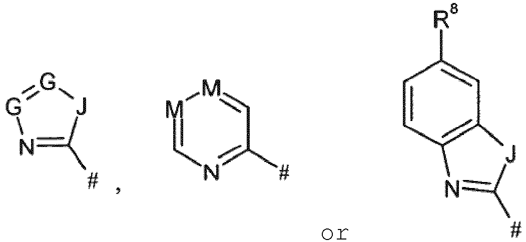 where
# represents the point of attachment to the dihydropyrazolone ring,
G represents in each case C-R⁶ or N, where not more than one of the two ring members G represents N,
J represents O or S,
M represents in each case C-R⁹ or N, where one of the two ring members M represents N and the other represents C-R⁹,
where
R⁶ and R⁹ in each individual case independently of one another represent hydrogen or a substituent selected from the group consisting of fluorine, chlorine, bromine, cyano, (C₁-C₆)-alkyl, (C₃-C₆)-cycloalkyl, 4- to 6-membered heterocycloalkyl, phenyl, 5- or 6-membered heteroaryl, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO²-R²⁵, -OR²⁹ and -NR³¹R³², where
(*i*) (C₁-C₆)-alkyl for its part may be mono- to trisubstituted by identical or different radicals selected from the group consisting of fluorine, chlorine, bromine, cyano, (C₃-C₆)-cycloalkyl, 4- to 6-membered heterocycloalkyl, phenyl, 5- or 6-membered heteroaryl, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -OR²⁹ and -NR³¹R³²,
where the last-mentioned cycloalkyl, heterocycloalkyl, phenyl and heteroaryl radicals for their part may in each case be substituted up to two times by identical or different substituents from the group consisting of fluorine, chlorine, bromine, cyano, (C₁-C₄) -alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
(*ii*) (C₃-C₆)-cycloalkyl, 4- to 6-membered heterocycloalkyl, phenyl and 5- or 6-membered heteroaryl for their part may in each case be mono- or disubstituted by identical or different substituents from the group consisting of fluorine, chlorine, bromine, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
(*iii*) R¹¹ R¹², R¹⁴, R¹⁵, R¹⁸, R²⁰, R²², R²⁵, R²⁹ and R³¹ independently of one another in each individual occurrence represent a radical selected from the group consisting of hydrogen, (C₁-C₆)-alkyl, (C₃-C₆)-cycloalkyl, 4- to 6-membered heterocycloalkyl, phenyl and 5- or 6-membered heteroaryl, where
(C₃-C₆)-cycloalkyl, 4- to 6-membered heterocycloalkyl, phenyl and 5- or 6-membered heteroaryl for their part may in each case be substituted up to three times by identical or different substituents from the group consisting of fluorine, chlorine, bromine, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl
and
(C₁-C₆)-alkyl may be mono- to trisubstituted by identical or different substituents from the group consisting of fluorine, chlorine, bromine, cyano, hydroxyl, trifluoromethoxy, (C₁-C₄)-alkoxy, amino, mono-(C₁-C₄)-alkylamino, di-(C₁-C₄)-alkylamino, hydroxycarbonyl, (C₁-C₄)-alkoxycarbonyl, (C₃-C₆)-cycloalkyl, 4- to 6-membered heterocycloalkyl, phenyl and 5- or 6-membered heteroaryl,
(*iv*) R¹³, R¹⁶, R¹⁷, R¹⁹, R²¹, R²³, R²⁴ and R³² independently of one another in each individual occurrence represent a radical selected from the group consisting of hydrogen and (C₁-C₆)-alkyl,
where (C₁-C₆)-alkyl may be mono- or disubstituted by identical or different substituents from the group consisting of fluorine, chlorine, bromine, cyano, hydroxyl, trifluoromethoxy, (C₁-C₄)-alkoxy, amino, mono-(C₁-C₄)-alkylamino, di-(C₁-C₄)-alkylamino, hydroxycarbonyl and (C₁-C₄)-alkoxycarbonyl,
and/or where
(*v*) R¹² and R¹³, R¹⁵ and R¹⁶, R¹⁷ and R¹⁸, R¹⁹ and R²⁰, R²¹ and R²², R²² and R²³, R²⁴ and R²⁵ and also R³¹ and R³² in each case as a pair together with the atoms to which they are attached may form a 5- or 6-membered heterocycloalkyl ring which may be mono- or disubstituted by identical or different substituents from the group consisting of fluorine, chlorine, bromine, cyano, (C₁-C₄)-alkyl, trifluoromethyl, hydroxyl, (C₁-C₄)-alkoxy, trifluoromethoxy, oxo, amino, mono-(C₁-C₄)-alkyl-amino, di-(C₁-C₄)-alkylamino, hydroxycarbonyl and (C₁-C₄)-alkoxy-carbonyl, and
R⁸ represents hydrogen or a substituent selected from the group consisting of fluorine, chlorine, bromine, cyano, (C₁-C₆)-alkyl, hydroxyl, (C₁-C₆)-alkoxy, trifluoromethoxy, amino, mono-(C₁-C₆)-alkylamino, di-(C₁-C₆)-alkylamino, hydroxycarbonyl and (C₁-C₆)-alkoxy-carbonyl, and
R³ represents hydrogen or methyl,
and salts, solvates and solvates of the salts thereof.

3. Compound of the formula (I) according to Claim 1 or 2 in which
R¹ represents a heteroaryl group of the formula 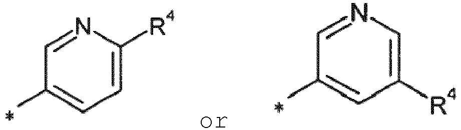 where
* represents the point of attachment to the dihydropyrazolone ring
and
R⁴ represents hydrogen, fluorine, chlorine, bromine, cyano, (C₁-C₄)-alkyl, trifluoromethyl, hydroxymethyl, (C₁-C₄)-alkoxy, trifluoromethoxy, hydroxycarbonyl or (C₁-C₄)-alkoxycarbonyl,
R² represents a heteroaryl group of the formula 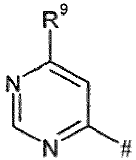 where
# represents the point of attachment to the dihydropyrazolone ring and
R⁹ represents hydrogen, fluorine, chlorine, bromine, cyano, (C₁-C₄)-alkyl, trifluoromethyl, hydroxyl, (C₁-C₄)-alkoxy, trifluoromethoxy, amino, mono-(C₁-C₄)-alkylamino, di-(C₁-C₄)-alkylamino, hydroxycarbonyl, (C₁-C₄)-alkoxycarbonyl, 4- to 6-membered heterocycloalkyl, phenyl or 5- or 6-membered heteroaryl, where
(C₁-C₄)-alkyl for its part may be substituted by hydroxyl, (C₁-C₄)-alkoxy or amino
and
4- to 6-membered heterocycloalkyl, phenyl and 5- or 6-membered heteroaryl for their part may in each case be mono-or disubstituted by identical or different substituents from the group consisting of fluorine, chlorine, bromine, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl, and
R³ represents hydrogen,
and salts, solvates and solvates of the salts thereof.

4. Compound of the formula (I) according to Claim 1 or 2 in which
R¹ represents a heteroaryl group of the formula 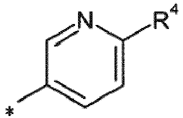 where
* represents the point of attachment to the dihydropyrazolone ring
and
R⁴ represents hydrogen, fluorine, chlorine, bromine, cyano, (C₁-C₄)-alkyl, trifluoromethyl, hydroxymethyl, (C₁-C₄)-alkoxy, trifluoromethoxy, hydroxycarbonyl or (C₁-C₄)-alkoxycarbonyl,
R² represents a heteroaryl group of the formula 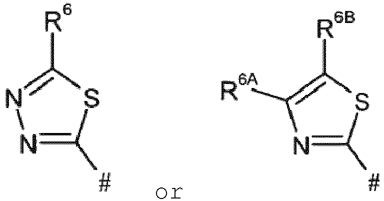 where
# represents the point of attachment to the dihydropyrazolone ring
and
R⁶, R^{6A} and R^{6B} are identical or different and independently of one another represent hydrogen or a substituent selected from the group consisting of fluorine, chlorine, bromine, cyano, (C₁-C₄)-alkyl, trifluoromethyl, hydroxyl, (C₁-C₄)-alkoxy, trifluoromethoxy, amino, mono-(C₁-C₄)-alkylamino, di-(C₁-C₄)-alkylamino, hydroxycarbonyl, (C₁-C₄)-alkoxycarbonyl, 4- to 6-membered heterocycloalkyl, phenyl and 5- or 6-membered heteroaryl, where
(C₁-C₄)-alkyl for its part may be substituted by hydroxyl, (C₁-C₄)-alkoxy or amino
and
4- to 6-membered heterocycloalkyl, phenyl and 5- or 6-membered heteroaryl for their part may in each case be mono-or disubstituted by identical or different substituents from the group consisting of fluorine, chlorine, bromine, cyano, (C₁-C₄)-alkyl, trifluoromethyl, hydroxyl, (C₁-C₄)-alkoxy, trifluoromethoxy, oxo, amino, mono-(C₁-C₄)-alkylamino, di-(C₁-C₄)-alkylamino, hydroxycarbonyl and (C₁-C₄)-alkoxycarbonyl,
and
R³ represents hydrogen,
and salts, solvates and solvates of the salts thereof.

5. Process for the preparation of a compound of the formula (I) as defined in Claims 1 to 4, **characterized in that** a compound of the formula (II) 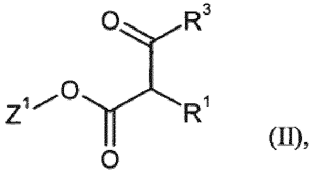 in which R¹ and R³ have the meanings given in Claims 1 to 4 and
Z¹ represents methyl or ethyl,
is reacted in an inert solvent, if appropriate in the presence of an acid, with a compound of the formula (III) 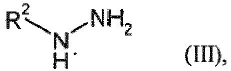 in which R² has the meaning given in Claims 1 to 4,
to give compounds of the formula (IV) 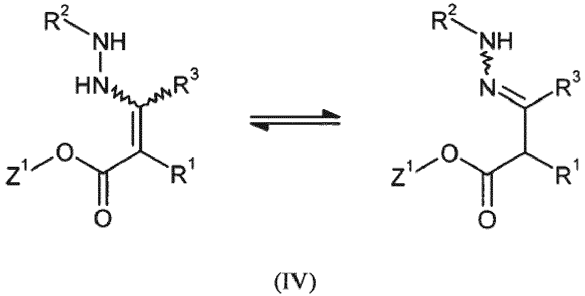 in which Z¹, R¹, R² and R³ have the meanings given above,
which, already under these reaction conditions or in a subsequent reaction step under the action of a base, cyclize to the compounds of the formula (I),
and the compounds of the formula (I) are, if appropriate with the appropriate (i) solvents and/or (ii) bases or acids, converted into their solvates, salts and/or solvates of the salts.

6. Process for the preparation of a compound of the formula (I) as defined in Claims 1 to 4 in which R³ represents hydrogen, **characterized in that** initially a compound of the formula (V) 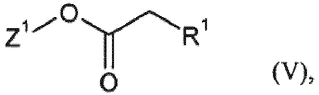 in which R¹ has the meaning given in Claims 1 to 4 and
Z¹ represents methyl or ethyl,
is condensed with a compound of the formula (VI) 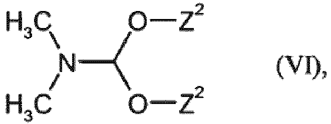 in which
Z² represents methyl or ethyl,
to give compounds of the formula (VII) 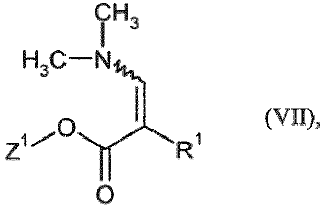 in which Z¹ and R¹ have the meanings given above,
and then reacted in the presence of an acid with a compound of the formula (III) 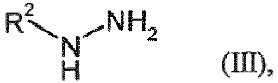 in which R² has the meaning given in Claims 1 to 4,
to give compounds of the formula (IV-A) 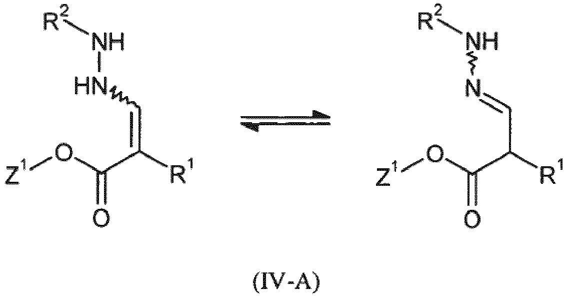 in which Z¹, R¹ and R² have the meanings given above,
which, already under these reaction conditions or in a subsequent reaction step under the action of a base, cyclize to the compounds of the formula (I) in which R³ represents hydrogen.

7. Compound as defined in any of Claims 1 to 4 for treatment and/or prophylaxis of diseases.

8. Use of a compound as defined in any of Claims 1 to 4 for the preparation of a medicament for treatment and/or prophylaxis of cardiovascular diseases, cardiac insufficiency, anemia, chronic kidney diseases and renal insufficiency.

9. Medicament comprising a compound as defined in any of Claims 1 to 4 in combination with an inert, non-toxic, pharmaceutically suitable auxiliary.

10. Medicament comprising a compound as defined in any of Claims 1 to 4 in combination with one or more further active compounds chosen from the group consisting of ACE inhibitors, angiotensin II receptor antagonists, beta receptor blockers, calcium antagonists, PDE inhibitors, mineralocorticoid receptor antagonists, diuretics, aspirin, iron supplements, vitamin B12 and folic acid supplements, statins, digitalis (digoxin) derivatives, tumor chemotherapeutics and antibiotics.

11. Medicament according to Claim 9 or 10 for treatment and/or prophylaxis of cardiovascular diseases, cardiac insufficiency, anemia, chronic kidney diseases and renal insufficiency.

## Revendications

1. Composé de formule (I) 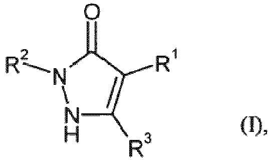 dans lequel
R¹ représente un groupe hétéroaryle de formule 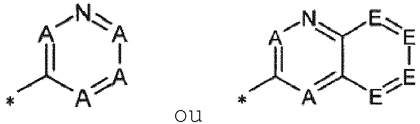 dans lesquelles
* signifie l'emplacement de liaison avec le cycle dihydropyrazolone,
A signifie à chaque occurrence individuelle C-R⁴ ou N, au plus deux éléments de cycle A représentant simultanément N,
et
E signifie à chaque occurrence individuelle C-R⁵ ou N, au plus deux éléments de cycle E représentant simultanément N,
R² représente un groupe hétéroaryle de formule 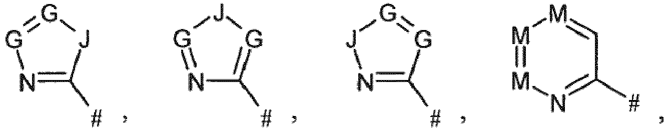 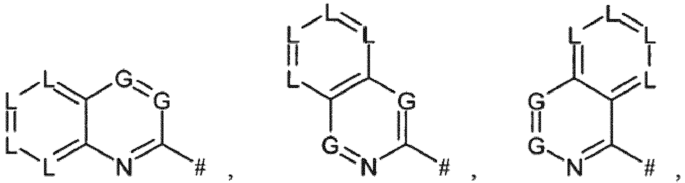 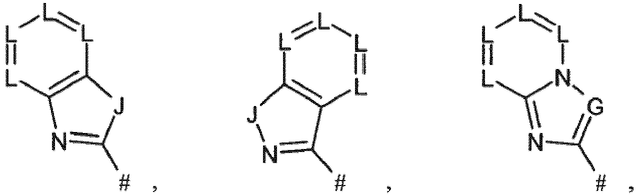 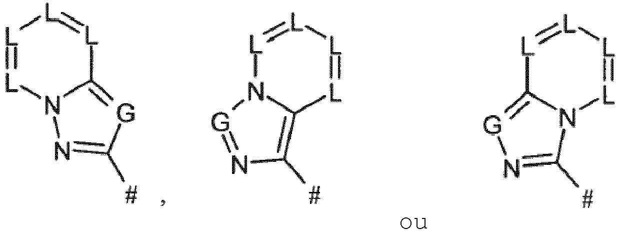 dans lesquelles
# signifie l'emplacement de liaison avec le cycle dihydropyrazolone,
G signifie à chaque occurrence individuelle C-R⁶ ou N,
J signifie O, S ou N-R⁷,
L signifie à chaque occurrence individuelle C-R⁸ ou
N, au plus deux éléments de cycle L représentant simultanément N,
et
M signifie à chaque occurrence individuelle C-R⁹ ou N, au total un ou deux éléments de cycle M représentant N,
R⁴, R⁶, R⁸ et R⁹ étant identiques ou différents, et représentant dans chaque cas individuel, indépendamment les uns des autres, l'hydrogène ou un substituant choisi dans la série constituée par halogène, cyano, nitro, alkyle en (C₁-C₆), cycloalkyle en (C₃-C₇), hétérocycloalkyle de 4 à 10 éléments, phényle, hétéroaryle à 5 ou 6 éléments, -C(=O)-R¹⁰, -C(=O)-OR¹¹, - C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)₋OR²⁰, -NR²¹-C(=O)-NR²²R²³, -NR²⁴-SO₂-R²⁵, - SO₂-R²⁶, -SO₂-NR²⁷R²⁸, -OR²⁹, -SR³⁰ et -NR³¹R³²,
(*i*) l'alkyle en (C₁-C₆) pouvant de son côté être substitué une à trois fois, de manière identique ou différente, avec des radicaux choisis dans la série constituée par halogène, cyano, oxo, cycloalkyle en (C₃-C₇), hétérocycloalkyle de 4 à 10 éléments, phényle, hétéroaryle à 5 ou 6 éléments, -C(=O)-R¹⁰, -C(=O)-OR¹¹, - C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, - SO₂-R²⁶, -SO₂-NR²⁷R²⁸, -OR²⁹, -SR³⁰ et -NR³¹R³²,
les radicaux cycloalkyle, hétérocycloalkyle, phényle et hétéroaryle mentionnés en dernier pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
(*ii*) le cycloalkyle en (C₃-C₇), l'hétérocycloalkyle de 4 à 10 éléments, le phényle et l'hétéroaryle à 5 ou 6 éléments pouvant chacun de leur côté être substitués une à trois fois, de manière identique ou différente, avec des radicaux choisis dans la série constituée par alkyle en (C₁-C₆), cycloalkyle en (C₃-C₇), halogène, cyano, oxo, -C(=O)-R¹⁰, -C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴-SO₂-R²⁵, -SO₂-R²⁶, -SO₂-NR²⁷R²⁸, -OR²⁹, -SR³⁰ et -NR³¹R³²,
le radical alkyle mentionné en dernier pouvant de son côté être substitué jusqu'à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₇), hétérocycloalkyle de 4 à 7 éléments, phényle et/ou hétéroaryle à 5 ou 6 éléments,
(*iii*) R¹⁰, R¹¹, R¹², R¹⁴, R¹⁵, R¹⁸, R²⁰, R²², R²⁵, R²⁶, R²⁷, R²⁹, R³⁰ et R³¹ représentant indépendamment les uns des autres à chaque occurrence individuelle un radical choisi dans la série constituée par hydrogène, alkyle en (C₁-C₆), cycloalkyle en (C₃-C₇), hétérocycloalkye de 4 à 7 éléments, phényle et hétéroaryle à 5 ou 6 éléments,
le cycloalkyle en (C₃-C₇), l'hétérocycloalkye de 4 à 7 éléments, le phényle et l'hétéroaryle à 5 ou 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁C₄), di-alkylamino en (C₁-C₄), hydroxycarbonyle, alcoxycarbonyle en (C₁-C₄), cycloalkyle en (C₃-C₇), hétérocycloalkyle de 4 à 7 éléments, phényle et/ou hétéroaryle à 5 ou 6 éléments,
le radical hétérocycloalkyle mentionné en dernier pouvant de son côté être substitué jusqu'à deux fois, de manière identique ou différente, avec alkyle en (C₁-C₄),
(*iv*) R¹³, R¹⁶, R¹⁷, R¹⁹, R²¹, R²³, R²⁴, R²⁸ et R³² représentant indépendamment les uns des autres à chaque occurrence individuelle un radical choisi dans la série constituée par hydrogène et alkyle en (C₁-C₆), l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
et/ou
*(v)* R¹² et R¹³, R¹⁵ et R¹⁶, R¹⁷ et R¹⁸, R¹⁹ et R²⁰, R²¹ et R²², R²² et R²³, R²⁴ et R²⁵, R²⁷ et R²⁸, ainsi que R³¹ et R³² pouvant chacun former en paires avec les atomes auxquels ils sont reliés un cycle hétérocycloalkyle à 5 ou 6 éléments, qui peut être substitué une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄), les R⁵ représentant dans chaque cas individuel, indépendamment les uns des autres, l'hydrogène ou un substituant choisi dans la série constituée par halogène, cyano, nitro, alkyle en (C₁-C₆), trifluorométhyle, hydroxy, alcoxy en (C₁-C₆), trifluorométhoxy, amino, mono-alkylamino en (C₁-C₆), di-alkylamino en (C₁-C₆), hydroxycarbonyle et alcoxycarbonyle en (C₁-C₆),
et
R⁷ représentant l'hydrogène ou un substituant choisi dans la série constituée par alkyle en (C₁-C₆), cycloalkyle en (C₃-C₇), hétérocycloalkyle de 4 à 7 éléments, phényle et hétéroaryle à 5 ou 6 éléments,
(*i*) l'alkyle en (C₁-C₆) pouvant de son côté être substitué une à trois fois, de manière identique ou différente, avec des radicaux choisis dans la série constituée par halogène, cyano, oxo, cycloalkyle en (C₃-C₇), hétérocycloalkyle de 4 à 7 éléments, phényle, hétéroaryle à 5 ou 6 éléments, -C(=O)-R¹⁰, -C(=O)-OR¹¹, - C(=O)-NR¹²R¹³, -O-C(=O)-R¹⁴, -O-C(=O)-NR¹⁵R¹⁶, -NR¹⁷C(=O)-R¹⁸, -NR¹⁹-C(=O)-OR²⁰, -NR²¹-C(=O)-NR²²R²³, -NR²⁴-SO₂-R²⁵, - SO₂-R²⁶, -SO₂-NR²⁷R²⁸, -OR²⁹, -SR³⁰ et -NR³¹R³²,
les radicaux cycloalkyle, hétérocycloalkyle, phényle et hétéroaryle mentionnés en dernier pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄), et
(*ii*) le cycloalkyle en (C₃-C₇), l'hétérocycloalkyle de 4 à 7 éléments, le phényle et l'hétéroaryle à 5 ou 6 éléments pouvant chacun de leur côté être substitués une à trois fois, de manière identique ou différente, avec des radicaux choisis dans la série constituée par alkyle en (C₁-C₆)), halogène, cyano, oxo, -C(=O)-R¹⁰, - C(=O)-OR¹¹, -C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, - NR²⁴-SO₂-R²⁵, -SO₂-R²⁶, -SO₂-NR²⁷R²⁸, -OR²⁹, -SR³⁰ et - NR³¹R³²,
le radical alkyle mentionné en dernier pouvant de son côté être substitué jusqu'à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₇), hétérocycloalkyle de 4 à 7 éléments, phényle et/ou hétéroaryle à 5 ou 6 éléments,
(*a*) R¹⁰, R¹¹ R¹², R¹⁴, R¹⁵, R¹⁸, R²⁰, R²², R²⁵, R²⁶, R²⁷, R²⁹, R³⁰ et R³¹ représentant indépendamment les uns des autres à chaque occurrence individuelle un radical choisi dans la série constituée par hydrogène, alkyle en (C₁-C₆), cycloalkyle en (C₃-C₇), hétérocycloalkyle de 4 à 7 éléments, phényle et hétéroaryle à 5 ou 6 éléments,
le cycloalkyle en (C₃-C₇), l'hétérocycloalkyle de 4 à 7 éléments, le phényle et l'hétéroaryle à 5 ou 6 éléments pouvant chacun de leur côté être substitués jusqu'à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₇), hétérocycloalkyle de 4 à 7 éléments, phényle et/ou hétéroaryle à 5 ou 6 éléments,
(*b*) R¹³, R¹⁶, R¹⁷, R¹⁹, R²¹, R²³, R²⁴, R²⁸ et R³² représentant indépendamment les uns des autres à chaque occurrence individuelle un radical choisi dans la série constituée par hydrogène et alkyle en (C₁-C₆),
l'alkyle en (C₁-C₆) pouvant être substitué une à trois fois, de manière identique ou différente, avec halogène, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
et/ou
(*c*) R¹² et R¹³, R¹⁵ et R¹⁶, R¹⁷ et R¹⁸, R¹⁹ et R²⁰, R²¹ et R²², R²² et R²³, R²⁴ et R²⁵, R²⁷ et R²⁸, ainsi que R³¹ et R³² formant chacun en paires avec les atomes auxquels ils sont reliés un cycle hétérocycloalkyle à 5 ou 6 éléments, qui peut être substitué une à trois fois, de manière identique ou différente, avec halogène, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
R³ représente hydrogène, alkyle en (C₁-C₆) ou cycloalkyle en (C₃-C₇),
ainsi que leurs sels, solvates et solvates des sels.

2. Composé de formule (I) selon la revendication 1, dans lequel
R¹ représente un groupe hétéroaryle de formule 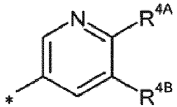 dans laquelle
* signifie l'emplacement de liaison avec le cycle dihydropyrazolone
et
R^{4A} et R^{4B} sont identiques ou différents, et signifient indépendamment l'un de l'autre l'hydrogène ou un substituant choisi dans la série constituée par fluor, chlore, brome, cyano, alkyle en (C₁-C₆), hydroxy, alcoxy en (C₁-C₆), trifluorométhoxy, amino, mono-alkylamino en (C₁-C₆), di-alkylamino en (C₁-C₆), hydroxycarbonyle et alcoxycarbonyle en (C₁-C₆),
le radical alkyle en (C₁-C₆) mentionné pouvant de son côté être substitué jusqu'à trois fois, de manière identique ou différente, avec fluor, chlore, brome, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄), R² représente un groupe hétéroaryle de formule 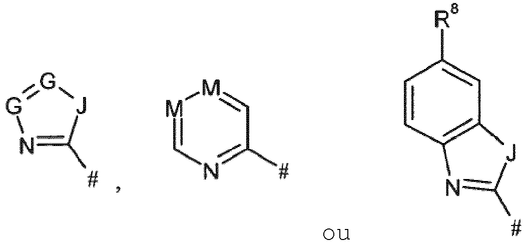
dans lesquelles
# signifie l'emplacement de liaison avec le cycle dihydropyrazolone,
G signifie à chaque fois C-R⁶ ou N, pas plus d'un des deux éléments de cycle G représentant N,
J signifie O ou S,
M signifie à chaque fois C-R⁹ ou N, un des deux éléments de cycle M représentant N et l'autre représentant C-R⁹,
R⁶ et R⁹ représentant dans chaque cas individuel, indépendamment l'un de l'autre, l'hydrogène ou un substituant choisi dans la série constituée par fluor, chlore, brome, cyano, alkyle en (C₁-C₆), cycloalkyle en (C₃-C₆), hétérocycloalkyle de 4 à 6 éléments, phényle, hétéroaryle à 5 ou 6 éléments, -C(=O)-OR¹¹, -C(=O)-NR¹²R¹³, -O-C(=O)-R¹⁴, -O-C(=O)-NR¹⁵R¹⁶, _NR¹⁷-C(=O)-R¹⁸, - NR¹⁹-C(=O)-OR²⁰, -NR21-C(=O)-NR²²R²³, -NR²⁴-SO₂-R²⁵, -OR²⁹ et -NR³¹R³²,
*(i)* l'alkyle en (C₁-C₆) pouvant de son côté être substitué une à trois fois, de manière identique ou différente, avec des radicaux choisis dans la série constituée par fluor, chlore, brome, cyano, cycloalkyle en (C₃-C₆), hétérocycloalkyle de 4 à 6 éléments, phényle, hétéroaryle à 5 ou 6 éléments, -C(=O)-OR¹¹, - C(=O)-NR¹²R¹³, -O-C(=O)-R¹⁴, -O-C(=O)-NR¹⁵R¹⁶, -NR¹⁷-C(=O)-R¹⁸, -NR¹⁹-C(=O)-OR²⁰, -NR²¹-C(=O)-NR²²R²³, -NR²⁴- SO₂-R²⁵,-OR²⁹ et -NR³¹R³²,
les radicaux cycloalkyle, hétérocycloalkyle, phényle et hétéroaryle mentionnés en dernier pouvant chacun de leur côté être substitués jusqu'à deux fois, de manière identique ou différente, avec fluor, chlore, brome, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
(*ii*) le cycloalkyle en (C₃-C₆), l'hétérocycloalkyle de 4 à 6 éléments, le phényle et l'hétéroaryle à 5 ou 6 éléments pouvant chacun de leur côté être substitués une ou deux fois, de manière identique ou différente, avec fluor, chlore, brome, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
(*iii*) R¹¹, R¹², R¹⁴, R¹⁵, R¹⁸, R²⁰, R²², R²⁵, R²⁹ et R³¹ représentant indépendamment les uns des autres à chaque occurrence individuelle un radical choisi dans la série constituée par hydrogène, alkyle en (C₁-C₆), cycloalkyle en (C₃-C₆), hétérocycloalkye de 4 à 6 éléments, phényle et hétéroaryle à 5 ou 6 éléments,
le cycloalkyle en (C₃-C₆), l'hétérocycloalkye de 4 à 6 éléments, le phényle et l'hétéroaryle à 5 ou 6 éléments pouvant chacun de leur côté être substitués jusqu'à trois fois, de manière identique ou différente, avec fluor, chlore, brome, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
l'alkyle en (C₁-C₆) pouvant être substitué une à trois fois, de manière identique ou différente, avec fluor, chlore, brome, cyano, hydroxy, trifluorométhoxy, alcoxy en (C₁-C₄), amino, mono-alkylamino en (C₁-C₄), di-alkylamino en (C₁-C₄), hydroxycarbonyle, alcoxycarbonyle en (C₁-C₄), cycloalkyle en (C₃-C₆), hétérocycloalkyle de 4 à 6 éléments, phényle et/ou hétéroaryle à 5 ou 6 éléments,
(*iv*) R¹³, R¹⁶, R¹⁷, R¹⁹, R²¹, R²³, R²⁴ et R³² représentant indépendamment les uns des autres à chaque occurrence individuelle un radical choisi dans la série constituée par hydrogène et alkyle en (C₁-C₆),
l'alkyle en (C₁-C₆) pouvant être substitué une ou deux fois, de manière identique ou différente, avec fluor, chlore, brome, cyano, hydroxy, trifluorométhoxy, alcoxy en (C₁-C₄), amino, mono-alkylamino en (C₁-C₄), di-alkylamino en (C₁-C₄), hydroxycarbonyle et/ou alcoxycarbonyle en (C₁-C₄),
et/ou
*(v)* R¹² et R¹³, R¹⁵ et R¹⁶, R¹⁷ et R¹⁸, R¹⁹ et R²⁰, R²¹ et R²², R²² et R²³, R²⁴ et R²⁵, ainsi que R³¹ et R³² pouvant chacun former en paires avec les atomes auxquels ils sont reliés un cycle hétérocycloalkyle à 5 ou 6 éléments, qui peut être substitué une ou deux fois, de manière identique ou différente, avec fluor, chlore, brome, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
R⁸ signifiant l'hydrogène ou un substituant choisi dans la série constituée par fluor, chlore, brome, cyano, alkyle en (C₁-C₆), hydroxy, alcoxy en (C₁-C₆), trifluorométhoxy, amino, mono-alkylamino en (C₁-C₆), di-alkylamino en (C₁-C₆), hydroxycarbonyle et alcoxycarbonyle en (C₁-C₆),
et
R³ représente hydrogène ou méthyle,
ainsi que leurs sels, solvates et solvates des sels.

3. Composé de formule (I) selon la revendication 1 ou 2, dans lequel
R¹ représente un groupe hétéroaryle de formule 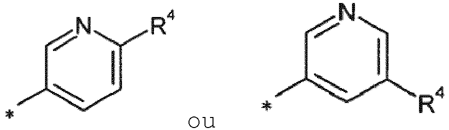
dans lesquelles
* signifie l'emplacement de liaison avec le cycle dihydropyrazolone
et
R⁴ signifie hydrogène, fluor, chlore, brome, cyano, alkyle en (C₁-C₄), trifluorométhyle, hydroxyméthyle, alcoxy en (C₁-C₄), trifluorométhoxy, hydroxycarbonyle ou alcoxycarbonyle en (C₁-C₄),
R² représente un groupe hétéroaryle de formule 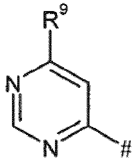
dans laquelle
# signifie l'emplacement de liaison avec le cycle dihydropyrazolone
et
R⁹ signifie hydrogène, fluor, chlore, brome, cyano, alkyle en (C₁-C₄), trifluorométhyle, hydroxy, alcoxy en (C₁-C₄), trifluorométhoxy, amino, mono-alkylamino en (C₁-C₄), di-alkylamino en (C₁-C₄), hydroxycarbonyle, alcoxycarbonyle en (C₁-C₄), hétérocycloalkyle de 4 à 6 éléments, phényle ou hétéroaryle à 5 ou 6 éléments,
l'alkyle en (C₁-C₄) pouvant de son côté être substitué avec hydroxy, alcoxy en (C₁-C₄) ou amino,
et
l'hétérocycloalkyle de 4 à 6 éléments, le phényle et l'hétéroaryle à 5 ou 6 éléments pouvant chacun de leur côté être substitués une ou deux fois, de manière identique ou différente, avec fluor, chlore, brome, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
R³ représente hydrogène,
ainsi que leurs sels, solvates et solvates des sels.

4. Composé de formule (I) selon la revendication 1 ou 2, dans lequel
R¹ représente un groupe hétéroaryle de formule 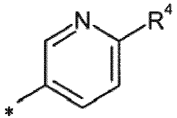
dans laquelle
* signifie l'emplacement de liaison avec le cycle dihydropyrazolone
et
R⁴ signifie hydrogène, fluor, chlore, brome, cyano, alkyle en (C₁-C₄), trifluorométhyle, hydroxyméthyle, alcoxy en (C₁-C₄), trifluorométhoxy, hydroxycarbonyle ou alcoxycarbonyle en (C₁-C₄),
R² représente un groupe hétéroaryle de formule 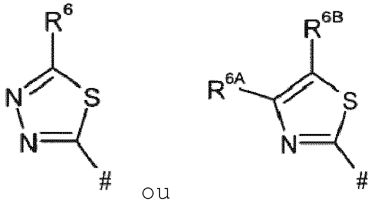
dans lesquelles
# signifie l'emplacement de liaison avec le cycle dihydropyrazolone
et
R⁶, R^{6A} et R^{6B} sont identiques ou différents, et signifient indépendamment les uns des autres l'hydrogène ou un substituant choisi dans la série constituée par fluor, chlore, brome, cyano, alkyle en (C₁-C₄), trifluorométhyle, hydroxy, alcoxy en (C₁-C₄), trifluorométhoxy, amino, mono-alkylamino en (C₁-C₄), di-alkylamino en (C₁-C₄), hydroxycarbonyle, alcoxycarbonyle en (C₁-C₄), hétérocycloalkyle de 4 à 6 éléments, phényle et hétéroaryle à 5 ou 6 éléments,
l'alkyle en (C₁-C₄) pouvant de son côté être substitué avec hydroxy, alcoxy en (C₁-C₄) ou amino,
et
l'hétérocycloalkyle de 4 à 6 éléments, le phényle et l'hétéroaryle à 5 ou 6 éléments pouvant chacun de leur côté être substitués une ou deux fois, de manière identique ou différente, avec fluor, chlore, brome, cyano, alkyle en (C₁-C₄), trifluorométhyle, hydroxy, alcoxy en (C₁-C₄), trifluorométhoxy, oxo, amino, mono-alkylamino en (C₁-C₄), di-alkylamino en (C₁-C₄), hydroxycarbonyle et/ou alcoxycarbonyle en (C₁-C₄),
et
R³ représente hydrogène,
ainsi que leurs sels, solvates et solvates des sels.

5. Procédé de fabrication d'un composé de formule (I), tel que défini dans les revendications 1 à 4, **caractérisé en ce qu'**un composé de formule (II) 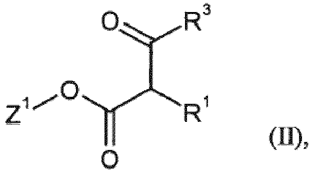 dans laquelle R¹ et R³ ont les significations indiquées dans les revendications 1 à 4, et
Z¹ représente méthyle ou éthyle,
est mis en réaction dans un solvant inerte, éventuellement en présence d'un acide, avec un composé de formule (III) 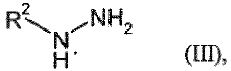 dans laquelle R² a la signification indiquée dans les revendications 1 à 4,
pour former des composés de formule (IV) 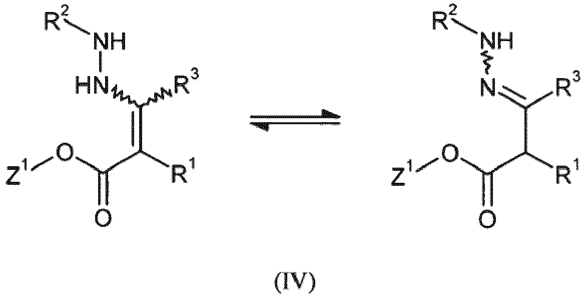 dans laquelle Z¹, R¹, R² et R³ ont les significations indiquées précédemment,
qui sont déjà cyclisés dans ces conditions de réaction ou lors d'une étape de réaction ultérieure sous l'effet d'une base pour former les composés de formule (I),
et les composé de formule (I) sont éventuellement transformés avec (i) les solvants et/ou (ii) les bases ou les acides appropriés en leurs solvates, sels et/ou solvates des sels.

6. Procédé de fabrication d'un composé de formule (I), tel que défini dans les revendications 1 à 4, dans lequel R³ signifie l'hydrogène, **caractérisé en ce qu'**un composé de formule (V) 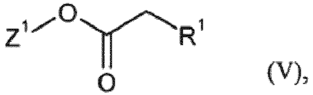 dans laquelle R¹ a la signification indiquée dans les revendications 1 à 4, et
Z¹ représente méthyle ou éthyle,
est tout d'abord condensé avec un composé de formule (VI) 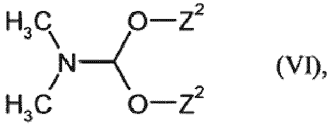 dans laquelle
Z² représente méthyle ou éthyle,
pour former des composés de formule (VII) 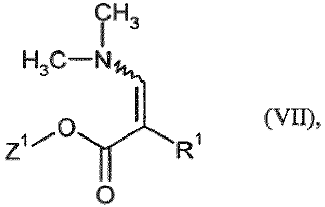 dans laquelle Z¹ et R¹ ont les significations indiquées précédemment,
puis mis en réaction en présence d'un acide avec un composé de formule (III) 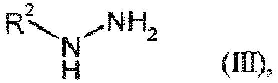 dans laquelle R² a la signification indiquée dans les revendications 1 à 4,
pour former des composés de formule (IV-A) 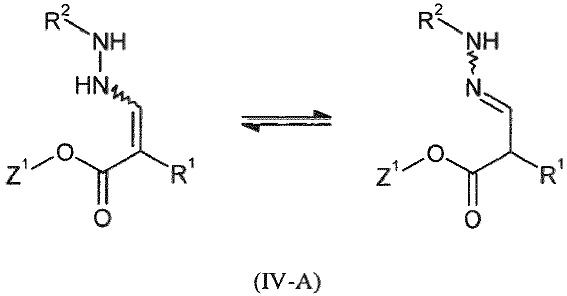 dans laquelle Z¹, R¹ et R² ont les significations indiquées précédemment,
qui sont déjà cyclisés dans ces conditions de réaction ou lors d'une étape de réaction ultérieure sous l'effet d'une base pour former les composés de formule (I), dans lesquels R³ représente l'hydrogène.

7. Composé, tel que défini dans l'une quelconque des revendications 1 à 4, pour le traitement et/ou la prophylaxie de maladies.

8. Utilisation d'un composé, tel que défini dans l'une quelconque des revendications 1 à 4, pour la fabrication d'un médicament pour le traitement et/ou la prophylaxie de maladies cardiovascuaires, de l'insuffisance cardiaque, de l'anémie, des maladies rénales chroniques et de l'insuffisance rénale.

9. Médicament contenant un composé, tel que défini dans l'une quelconque des revendications 1 à 4, en combinaison avec un adjuvant inerte, non toxique, pharmaceutiquement approprié.

10. Médicament contenant un composé, tel que défini dans l'une quelconque des revendications 1 à 4, en combinaison avec un ou plusieurs autres agents actifs choisis dans le groupe constitué par les inhibiteurs d'ACE, les antagonistes des récepteurs d'angiotensine II, les bloquants des récepteurs bêta, les antagonistes de calcium, les inhibiteurs de PDE, les antagonistes des récepteurs minéralocorticoïdes, les diurétiques, l'aspirine, les suppléments de fer, les suppléments de vitamine B12 et d'acide folique, les statines, les dérivés de digitalis (digoxine), les agents chimiothérapeutiques antitumoraux et les antibiotiques.

11. Médicament selon la revendication 9 ou 10, pour le traitement et/ou la prophylaxie de maladies cardiovascuaires, de l'insuffisance cardiaque, de l'anémie, des maladies rénales chroniques et de l'insuffisance rénale.
